(19) Europäisches Patentamt
European Patent Office
Office européen des brevets

(11) EP 3 290 412 A1

(12) EUROPEAN PATENT APPLICATION

(43) Date of publication:
**07.03.2018 Bulletin 2018/10**

(21) Application number: **16186511.8**

(22) Date of filing: **31.08.2016**

(51) Int Cl.:
***C07D 401/14*** (2006.01) ***C07D 401/04*** (2006.01)
***C07D 403/04*** (2006.01) ***A61K 31/506*** (2006.01)
***A61P 31/18*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Università degli Studi di Siena**
  **53100 Siena (IT)**
- **IRBM - Science Park S.p.A.**
  **00040 Pomezia (RM) (IT)**
- **Université de Strasbourg**
  **67000 Strasbourg (FR)**

(72) Inventors:
- **BOTTA, Maurizio**
  **53100 Siena (IT)**
- **MORI, Mattia**
  **53100 Siena (IT)**
- **SALADINI, Francesco**
  **53100 Siena (IT)**
- **ZAZZI, Maurizio**
  **53100 Siena (IT)**
- **FEZZARDI, Paola**
  **00071 Pomezia (IT)**
- **HARPER, Steven**
  **00071 Pomezia (IT)**
- **MALANCONA, Savina**
  **00071 Pomezia (IT)**
- **SANTORIELLO, Marisabella**
  **00071 Pomezia (IT)**
- **SUMMA, Vincenzo**
  **00071 Pomezia (IT)**
- **DE FORNI, Davide**
  **07041 Alghero (IT)**
- **KOVALENKO, Lesia**
  **67401 Illkirch (FR)**
- **MELY, Yves**
  **67401 Illkirch (FR)**
- **PIRES, Manuel**
  **67401 Illkirch (FR)**
- **REAL, Eleonore**
  **67401 Illkirch (FR)**
- **LYONNAIS, Sébastien**
  **08870 Sitges (ES)**
- **MIRAMBEAU, Gilles**
  **08012 Barcelona (ES)**

(74) Representative: **Trillat, Anne-Cecile et al**
**De Simone & Partners S.p.A.**
**Via Vincenzo Bellini, 20**
**00198 Roma (IT)**

# (54) HIV-1 NUCLEOCAPSID INHIBITORS

(57) The present invention discloses novel anti-HIV agents targeting the HIV Nucleocapsid protein (NC), which is one of the most conserved sequences within HIV strains and is highly required for HIV replication. The compounds of the invention are particularly useful to overcome antiretroviral drug-resistance in HIV-1 infected individuals.



Printed by Jouve, 75001 PARIS (FR)

## Description

### FIELD OF THE INVENTION

AIDS is one of the most serious pandemic diseases of the modem era. Although current therapies based on targeting key processes of the HIV replication cycle are potent and selective, several clinical failures are recorded due to the emergence of drug resistance. Hence, there is an urgent need for novel drugs and alternative therapeutic strategies. Compounds of the present invention are novel anti-HIV agents targeting the HIV Nucleocapsid protein (NC), which is one of the most conserved sequences within HIV strains and is highly required for HIV replication, being therefore a primary target to overcome antiretroviral drug-resistance.

### BACKGROUND OF THE INVENTION

The Joint United Nations Program on HIV/AIDS (UNAIDS) estimated that at the end of 2010 there were approximately 34 million people infected by HIV and about 30 million people have died of AIDS-related causes since the beginning of the epidemic. During the last 25 years the arsenal of drugs to fight HIV infection has increased continuously. However, most of these drugs experienced clinical failure due to Drug Resistance (DR), i.e. the ability of HIV to mutate and overcome the effects of a given drug to prevent its replication. DR has been the most critical limitation in antiretroviral therapy since its advent, as substantiated by the World Health Organization in establishing the "global HIV drug resistance network". Although strategies to fight DR may span from the development of an effective vaccine to target host essential proteins for HIV replication, the most commonly and realistic adopted strategy is still the design of potent and selective inhibitors of target proteins. However, novel compounds developed as inhibitors of well-known HIV target proteins often lack full activity because of drug resistance. Thus, future anti-HIV drug research efforts should focus on new HIV targets. In this context it was developed an innovative strategy targeting a highly conserved and multi-functional protein such as the HIV-1 nucleocapsid protein (NC), to prevent the emergence of DR and halting the virus life cycle at multiple steps. NC is a 55-aminoacid small basic protein with two zinc-fingers that assists the HIV-1 reverse transcriptase (RT) during reverse transcription by chaperoning the annealing of the cellular primer tRNA to the primer binding site (PBS) and the two obligatory DNA strand transfers necessary for the synthesis of a complete functional double-stranded vDNA with two long terminal repeats (see Mori M. et al, ACS Chemical Biology 2014, 9, 1950-1955). NC is also thought to protect the nascent vDNA agains nucleases (Krishnamoorthy G. et al, Nucleic Acids Res., 2003, 31, 5425-5432) and to assist the viral Integrase (IN) for the integration of the viral DNA into the host genome (Poljak L. et al, J. Mol. Biol., 2003, 329, 411-421). As a domain of the Gag structural polyprotein precursor, NC selects the genomic RNA and promotes its dimerization and packaging during virus assembly. Finally, NC binding to RNA is critical for Gag processing by the viral protease enzymeand for the subsequent formation of the condensed ribonucleoprotein architecture within the virion (Mirambeau G. et al., 2007, PLoS One 2, e669). NC can hardly mutate, so that NC mutants are generally nonfunctional and result in noninfectious viruses. Therefore, specific inhibition of NC is thought to generate a sustained antiretroviral activity. Inhibition of NC has been achieved by different strategies leading to antiretroviral effects in vitro and in vivo (Mori M et al, Curr. Pharm. Des., 2011, 17, 3713-3728), although generally through unspecific mechanisms and thus causing significant cytotoxicity. Indeed, there is a critical deficiency of small molecule NC inhibitors (NCIs) endowed with a clear and safe mechanism of action as well as with antiviral activity in vitro, which may be developed as effective and safe antiretroviral agents.

WO 2012076822 describes peptide ligands for the nucleocapsid protein NCp7 that inhibit the replication of human immunodeficiency virus. Said peptide inhibitors were screened for their ability to inhibit destabilization of the cTAR hairpin by NCp7.

EP1087941 pertains to the discovery of a novel family of thiolesters and uses thereof. Also provided for are viricidal compounds and pharmaceutical formulations comprising these novel thiolesters. The invention also provides thiolester-inactivated viruses and thiolester-complexed viral proteins.

WO03014062 refers to certain thiol and acylthiol compounds capable to inhibit retrovirus growth by attacking the highly conserved zinc finger regions of essential viral proteins. These compounds, compositions containing them, and methods of using them to treat retroviral infections such as HIV are described. These compounds are also useful for preparation of vaccines comprised of inactivated retroviruses such as HIV, prevention of the transmission of such retroviruses, and detection of retroviral proteins.

WO 2005/121376 relates to novel Human Immunodeficiency Virus I (HIV-1) variants that are resistant against protease inhibitors including the high genetic barrier protease inhibitor R0033-4649 and show mutations not in the protease itself but in the viral Gag protein (Matrix and Capsid) and the viral cleavage sites thus establishing a novel resistance mechanism. The invention further relates to diagnostic assays for determining the presence in a sample of the HTV variant.

WO2004032869 provides methods and pharmaceutical compositions for inhibiting viral replication, particularly

retroviral replication. The methods comprise administration of stibonic acid or diphenyl compounds that disrupt viral nucleocapsid binding to nucleic acids. WO2011/156674 discloses inhibitors of retroviral growth that are useful in treatment of retroviral infections such as HIV. The compounds are thioaryl-derivatives targeting the HIV-1 nucleocapsid protein (NCp7).

While several compounds have been proposed targeting NCp7, none have advanced through clinical trials due to toxicity or lack of specificity.

The problem solved by the invention is therefore the discovery of innovative antiretroviral compounds, in particular compounds that target the HIV-1 nucleocapsid protein. WO2003035076 discloses 4,5-dihydroxypyrimidine-6-carboxamides as HIV-1 Integrase inhibitors. HIV-1 integrase catalyzes the insertion of the viral DNA into the genome of the host cell, which is an essential step in the viral replication cycle. Structure-activity relationship studies on this series of compounds are extensively reported in the literature (see for example Pace, P. et al, J. Med. Chem. 2007, Vol. 50, No. 9, 2225-2239) and elucidates structural determinants affecting the potency at HIV-1 integrase enzyme. In particular, it is evident that an aromatic ring separated by at least one $sp^3$ atom at the amide in position 6 of the dihydroxypyrimidine core is essential for the activity. This pyrimidine series of compounds was further developed by Merck into the marketed drug Isentress™.

SUMMARY OF THE INVENTION

Within the present invention, it was surprisingly discovered that a specific series of compounds selected within the general formula claimed in WO2003035076 showed positive results in biological assays unveiling HIV-1 nucleocapsid inhibition properties, while more potent HIV integrase inhibitors, including Isentress™, in the same experimental settings were inactive. In particular, the combination of an aliphatic amide bearing a cyclo(hetero)alkyl substituent in position 6 and a nitrogen-containing heteroaromatic ring in position 2 of the dihydroxypyrimidine contributes to the inhibition of HIV-1 nucleocapsid while unfavourable for HIV-integrase activity.

Based on the present results, the compounds of the invention are HIV-1 nucleocapsid inhibitors particularly useful for overcoming HIV-1 resistance to known classes of antiretroviral drugs, included viral strains resistant to HIV integrase inhibitors.

The present invention is directed toward the use of dihydroxy-pyrimidine derivatives targeting the HIV-1 NCp7 nucleocapsid protein as antiretroviral compounds for the treatment of HIV-1 infection, in particular drug resistant HIV-1 infection.

Therefore the present invention provides a compound of general formula (I):

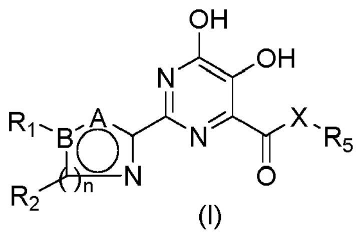

(I)

wherein:

A is selected from N, O, $CR_3$;
B is selected from N or C;
n is 1 or 2;
X is O, $NR_4$;
each of $R_1$ and $R_2$ is absent or independently selected from H, $C_{1-6}$alkyl, $C_{1-6}$alkoxy, halo$C_1$-$_6$alkyl, halo$C_{1-6}$alkoxy, halogen, cyano, nitro, hydroxyl, C(O)-$C_{1-6}$alkyl, C(O)O-$C_{1-6}$alkyl, C(O)-aryl, C(O)heteroaryl, $N(R^a)_2$, $C(O)N(R^a)_2$, $SO_2N(R^a)_2$, aryl, heteroaryl, aryl-$C_{1-3}$alkyl, aryl-$C_1$-$_3$alkoxy, each of said $C_{1-6}$alkyl, aryl and heteroaryl groups being optionally substituted by one or
more groups independently chosen from halogen, hydroxy, $N(R^a)_2$, $C_{1-6}$alkyl, halo$C_{1-6}$alkyl, $C_{1-6}$alkoxy, halo$C_{1-6}$alkoxy, $C_{1-6}$alkyl-$N(R^a)_2$, nitro, aryl, heteroaryl;
or $R_1$ and $R_2$ together form a fused 5 or 6 membered aromatic ring optionally containing an heteroatom selected from N, O or S, said fused aromatic ring being optionally substituted by one or more groups independently chosen from halogen, hydroxy, $N(R^a)_2$, $C_{1-6}$alkyl, halo$C_{1-6}$alkyl, $C_{1-6}$alkoxy, halo$C_{1-6}$alkoxy, $C_{1-6}$alkyl-$N(R^a)_2$, nitro, aryl, heteroaryl;
when n = 2, $R_2$ is the substituent of both carbon atoms and $R_2$ and $R_2$ together may form a fused 5 or 6 membered

aromatic ring optionally containing an heteroatom selected from N, O or S, said fused aromatic ring being optionally substituted by one or more groups independently chosen from halogen, hydroxy, $N(R^a)_2$, $C_{1-6}$alkyl, halo$C_{1-6}$alkyl, $C_{1-6}$alkoxy, halo$C_{1-6}$alkoxy, $C_{1-6}$alkyl-$N(R^a)_2$, nitro, aryl, heteroaryl;

$R_3$ is selected from H, $C_{1-6}$alkyl, halogen, halo$C_{1-6}$alkyl, nitro, cyano and $N(R^a)_2$;

$R_4$ is selected from H, $C_{1-6}$alkyl, halo$C_{1-6}$alkyl, aryl, heteroaryl, aryl-$C_{1-3}$alkyl;

$R_5$ is a group of general formula (II)

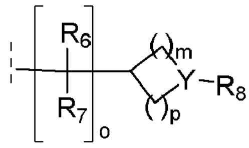

(II)

wherein

o is selected from 0 to 3;

m and p are selected from 0 to 3, with the proviso that at least one of m and n is not 0;

Y is selected from N and $CR_9$;

$R_6$ and $R_7$ are independently selected from H, $C_{1-6}$alkyl, halo$C_{1-6}$alkyl, $C_{3-6}$cycloalkyl, $C_{6-10}$aryl, $C_{6-10}$aryl-$C_{1-6}$alkyl, heteroaryl-$C_{1-3}$alkyl, $C_{3-15}$heterocyclyl, $C_{3-15}$heterocyclyl-$C_{1-3}$alkyl, $C_{3-6}$cycloalkyl-$C_{1-3}$alkyl, $C_{3-6}$cycloalkyl, aryl-$C_{1-3}$alkyl, heteroaryl-$C_{1-3}$alkyl, $C_{3-15}$heterocyclyl, $C_{3-15}$heterocyclyl-$C_{1-3}$alkyl, $C_{3-6}$cycloalkyl-$C_{1-3}$alkyl, each of them being optionally substituted by one or more groups independently chosen from $C_{1-6}$alkyl, $C_{1-6}$alkoxy, halogen, cyano, hydroxyl, C(O)-$C_{1-6}$alkyl, aryl, heteroaryl, aryl-$C_{1-3}$alkyl; or $R_6$ and $R_7$ are linked forming a cycloalkyl ring selected from cyclopropyl, cyclobutyl, cyclopentyl, cyclohexyl and cycloheptyl;

$R_8$ is selected from H, halogen, $C_{1-6}$alkyl, halo$C_{1-6}$alkyl, $C_{3-6}$cycloalkyl, $C_{6-10}$aryl, $C_{6-10}$aryl-$C_{1-6}$alkyl, heteroaryl-$C_{1-3}$alkyl, $C_{3-15}$heterocyclyl, $C_{3-15}$heterocyclyl-$C_{1-3}$alkyl, optionally substituted by one or more groups independently chosen from $C_{1-6}$alkyl, $C_{1-6}$alkoxy, halogen, hydroxyl, COOH, C(O)-$C_{1-6}$alkyl, $CONH_2$, CONH-$C_{1-6}$alkyl;

$R_9$ is selected from H, halogen, $C_{1-6}$alkyl;

or if X = N, $R_4$ and $R_5$ are alkyl chain linked together forming a ring selected from aziridine, azetidine, pyrrolidine, pyperidine, azepane, morpholine, piperazine, N-methylpiperazine wherein each of said ring is optionally substituted with $C_{1-6}$alkyl or $C_{6-10}$aryl;

each $R^a$ is independently selected from hydrogen, $C_{1-6}$alkyl, C(O)-$C_{1-6}$alkyl, C(O)-aryl, C(O)heteroaryl, C(O)O-$C_{1-6}$alkyl, $C_{6-10}$aryl-$C_{1-6}$alkyl, heteroaryl-$C_{1-3}$alkyl or $N(R^a)_2$ is a cyclic amine selected from pyrrolidine, pyperidine, and morpholine optionally substituted with one or more groups independently selected from $C_{1-6}$alkyl, halogen, hydroxy;

and pharmaceutically acceptable salts, tautomers, stereoisomers thereof, for use as HIV-1 nucleocapsid inhibitor in the treatment of an HIV-1 infection.

Preferably A is $CR_3$ or N and B is C.

Preferably the compound is of general formula (III):

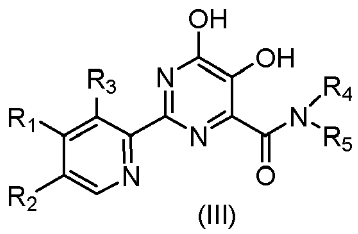

(III)

Wherein $R_1$, $R_2$, $R_3$, $R_4$ and $R_5$ are as defined above.

Preferably the compound is selected from:

- N-(cyclohexylmethyl)-5,6-dihydroxy-2-(pyridin-2-yl)pyrimidine-4-carboxamide;
- cyclohexylmethyl 5,6-dihydroxy-2-(pyridin-2-yl)pyrimidine-4-carboxylate;
- N-(cyclopropylmethyl)-5,6-dihydroxy-2-(pyridin-2-yl)pyrimidine-4-carboxamide;
- N-cyclohexyl-5,6-dihydroxy-2-(pyridin-2-yl)pyrimidine-4-carboxamide;

- (R)-N-(1-cyclohexylethyl)-5,6-dihydroxy-2-(pyridin-2-yl)pyrimidine-4-carboxamide;
- N-(2-cyclohexylethyl)-5,6-dihydroxy-2-(pyridin-2-yl)pyrimidine-4-carboxamide;
- 5,6-dihydroxy-N-((1-methylpiperidin-3-yl)methyl)-2-(pyridin-2-yl)pyrimidine-4-carboxamide;
- N-(3-cyclohexylpropyl)-5,6-dihydroxy-2-(pyridin-2-yl)pyrimidine-4-carboxamide;
- (5,6-dihydroxy-2-(pyridin-2-yl)pyrimidin-4-yl)(4-phenylpiperidin-1-yl)methanone;
- cyclohexylmethyl 5,6-dihydroxy-2-(3-methylpyridin-2-yl)pyrimidine-4-carboxylate;
- N-(cyclohexylmethyl)-5,6-dihydroxy-2-(3-methylpyridin-2-yl)pyrimidine-4-carboxamide;
- 2-cyclohexylethyl 5,6-dihydroxy-2-(pyridin-2-yl)pyrimidine-4-carboxylate;
- cyclohexyl 5,6-dihydroxy-2-(pyridin-2-yl)pyrimidine-4-carboxylate;
- 3-cyclohexylpropyl 5,6-dihydroxy-2-(pyridin-2-yl)pyrimidine-4-carboxylate;
- cyclohexylmethyl 2-(3-chloropyridin-2-yl)-5,6-dihydroxypyrimidine-4-carboxylate;
- 2-(3-chloropyridin-2-yl)-N-(cyclohexylmethyl)-5,6-dihydroxypyrimidine-4-carboxamide;
- 1-cyclohexylethyl 5,6-dihydroxy-2-(pyridin-2-yl)pyrimidine-4-carboxylate;
- (R)-N-(1-cyclohexylethyl)-5,6-dihydroxy-2-(6-methylpyridin-2-yl)pyrimidine-4-carboxamide;
- (R)-N-(1-cyclohexylethyl)-5,6-dihydroxy-2-(4-methylpyridin-2-yl)pyrimidine-4-carboxamide;
- (R)-2-(5-aminopyridin-2-yl)-N-(1-cyclohexylethyl)-5,6-dihydroxypyrimidine-4-carboxamide;
- benzyl (R)-6-(4-((1-cyclohexylethyl)carbamoyl)-5,6-dihydroxypyrimidin-2-yl)nicotinate;
- (R)-N-(1-cyclohexylethyl)-5,6-dihydroxy-2-(quinolin-2-yl)pyrimidine-4-carboxamide;
- (R)-N-(1-cyclohexylethyl)-5,6-dihydroxy-2-(3-methylpyridin-2-yl)pyrimidine-4-carboxamide;
- N-(1-cyclohexylcyclopropyl)-5,6-dihydroxy-2-(pyridin-2-yl)pyrimidine-4-carboxamide;
- (R)-N-(1-cyclohexylethyl)-5,6-dihydroxy-2-(isoquinolin-3-yl)pyrimidine-4-carboxamide;
- (R)-2-(4-chloropyridin-2-yl)-N-(1-cyclohexylethyl)-5,6-dihydroxypyrimidine-4-carboxamide;
- (R)-N-(1-cyclohexylethyl)-5,6-dihydroxy-2-(4-(trifluoromethyl)pyridin-2-yl)pyrimidine-4-carboxamide;
- (R)-N-(1-cyclohexylethyl)-5,6-dihydroxy-2-(4-methoxypyridin-2-yl)pyrimidine-4-carboxamide;
- (R)-2-(1H-benzo[d]imidazol-2-yl)-N-(1-cyclohexylethyl)-5,6-dihydroxypyrimidine-4-carboxamide;
- (R)-N-(1-cyclohexylethyl)-5,6-dihydroxy-2-(3-(trifluoromethyl)pyridin-2-yl)pyrimidine-4-carboxamide;
- (R)-N-(1-cyclohexylethyl)-5,6-dihydroxy-2-(isoquinolin-1-yl)pyrimidine-4-carboxamide;
- (R)-N-(1-cyclohexylethyl)-5,6-dihydroxy-2-(5-methoxypyridin-2-yl)pyrimidine-4-carboxamide;
- (R)-2-(3-chloropyridin-2-yl)-N-(1-cyclohexylethyl)-5,6-dihydroxypyrimidine-4-carboxamide;
- (R)-2-(6-chloropyridin-2-yl)-N-(1-cyclohexylethyl)-5,6-dihydroxypyrimidine-4-carboxamide;
- (R)-2-(4-(benzyloxy)pyridin-2-yl)-N-(1-cyclohexylethyl)-5,6-dihydroxypyrimidine-4-carboxamide;
- (R)-N-(1-cyclohexylethyl)-5,6-dihydroxy-2-(5-(trifluoromethyl)pyridin-2-yl)pyrimidine-4-carboxamide;
- (R)-N-(1-cyclohexylethyl)-5,6-dihydroxy-2-(6-methoxypyridin-2-yl)pyrimidine-4-carboxamide;
- (S)-N-(1-cyclohexylethyl)-5,6-dihydroxy-2-(pyridin-2-yl)pyrimidine-4-carboxamide;
- (S)-N-(1-cyclohexylethyl)-5,6-dihydroxy-2-(3-methylpyridin-2-yl)pyrimidine-4-carboxamide;
- (R)-N-(1-cyclohexylethyl)-5,6-dihydroxy-2-(4-nitropyridin-2-yl)pyrimidine-4-carboxamide;
- (R)-N-(1-cyclohexylethyl)-5,6-dihydroxy-2-(5-methylpyridin-2-yl)pyrimidine-4-carboxamide;
- tert-butyl (R)-(2-(4-((1-cyclohexylethyl)carbamoyl)-5,6-dihydroxypyrimidin-2-yl)pyridin-3-yl)carbamate;
- N-(2-cyclopentylethyl)-5,6-dihydroxy-2-(pyridin-2-yl)pyrimidine-4-carboxamide;
- (R)-N-(1-cyclohexylethyl)-5,6-dihydroxy-2-(6-(trifluoromethyl)pyridin-2-yl)pyrimidine-4-carboxamide;
- (R)-2-(6-aminopyridin-2-yl)-N-(1-cyclohexylethyl)-5,6-dihydroxypyrimidine-4-carboxamide;
- benzyl (R)-2-(4-((1-cyclohexylethyl)carbamoyl)-5,6-dihydroxypyrimidin-2-yl)isonicotinate;
- N-(cyclobutylmethyl)-5,6-dihydroxy-2-(isoquinolin-3-yl)pyrimidine-4-carboxamide;
- (R)-2-(4-aminopyridin-2-yl)-N-(1-cyclohexylethyl)-5,6-dihydroxypyrimidine-4-carboxamide;
- (R)-2-(5-chloropyridin-2-yl)-N-(1-cyclohexylethyl)-5,6-dihydroxypyrimidine-4-carboxamide;
- (R)-2-(5-(benzylcarbamoyl)pyridin-2-yl)-N-(1-cyclohexylethyl)-5,6-dihydroxypyrimidine-4-carboxamide;
- (R)-2-(5-carbamoylpyridin-2-yl)-N-(1-cyclohexylethyl)-5,6-dihydroxypyrimidine-4-carboxamide;
- (S)-N-(1-cyclohexylethyl)-5,6-dihydroxy-2-(4-(trifluoromethyl)pyridin-2-yl)pyrimidine-4-carboxamide;
- (R)-2-(5-((benzylamino)methyl)pyridin-2-yl)-N-(1-cyclohexylethyl)-5,6-dihydroxypyrimidine-4-carboxamide;
- benzyl (R)-6-(4-((1-cyclohexylethyl)carbamoyl)-5,6-dihydroxypyrimidin-2-yl)picolinate;
- (R)-N-(1-cyclohexylethyl)-5,6-dihydroxy-2-(5-(hydroxymethyl)pyridin-2-yl)pyrimidine-4-carboxamide;
- 5,6-dihydroxy-N-(4-methylcyclohexyl)-2-(pyridin-2-yl)pyrimidine-4-carboxamide;
- 5,6-dihydroxy-N-(3-methylcyclohexyl)-2-(pyridin-2-yl)pyrimidine-4-carboxamide;
- N-(4,4-dimethylcyclohexyl)-5,6-dihydroxy-2-(pyridin-2-yl)pyrimidine-4-carboxamide;
- 5,6-dihydroxy-N-(2-methylcyclohexyl)-2-(pyridin-2-yl)pyrimidine-4-carboxamide;
- (R)-N-(1-cyclohexylethyl)-2-(5-((ethylamino)methyl)pyridin-2-yl)-5,6-dihydroxypyrimidine-4-carboxamide;
- tert-butyl (R)-(6-(4-((1-cyclohexylethyl)carbamoyl)-5,6-dihydroxypyrimidin-2-yl)pyridin-3-yl)carbamate;
- (R)-2-(5-acetamidopyridin-2-yl)-N-(1-cyclohexylethyl)-5,6-dihydroxypyrimidine-4-carboxamide;

- N-(2-cyclopentyl-2-methylpropyl)-5,6-dihydroxy-2-(pyridin-2-yl)pyrimidine-4-carboxamide;
- (R)-N-(1-cyclohexylpropan-2-yl)-5,6-dihydroxy-2-(pyridin-2-yl)pyrimidine-4-carboxamide;
- N-(1-cyclopentylethyl)-5,6-dihydroxy-2-(pyridin-2-yl)pyrimidine-4-carboxamide;
- (R)-N-(1-cyclohexylethyl)-5,6-dihydroxy-2-(6-methoxyquinolin-2-yl)pyrimidine-4-carboxamide;
- (R)-N-(1-cyclohexylethyl)-2-(5,6-dimethylpyridin-2-yl)-5,6-dihydroxypyrimidine-4-carboxamide;
- N-[(1R)-1-cyclohexylethyl]-2-(4-methoxyquinolin-2-yl)-5,6-bis(oxidanyl)pyrimidine-4-carboxamide;
- N-[(1S)-1-cyclohexylethyl]-2-(4-methoxypyridin-2-yl)-5,6-bis(oxidanyl)pyrimidine-4-carboxamide;
- benzyl (S)-6-(4-((1-cyclohexylethyl)carbamoyl)-5,6-dihydroxypyrimidin-2-yl)nicotinate;
- benzyl (S)-2-(4-((1-cyclohexylethyl)carbamoyl)-5,6-dihydroxypyrimidin-2-yl)isonicotinate;
- (S)-N-(1-cyclohexylethyl)-5,6-dihydroxy-2-(isoquinolin-3-yl)pyrimidine-4-carboxamide;
- (R)-N-(1-cyclohexylethyl)-5,6-dihydroxy-2-(4-methylquinolin-2-yl)pyrimidine-4-carboxamide;
- (R)-2-(4-(benzylcarbamoyl)pyridin-2-yl)-N-(1-cyclohexylethyl)-5,6-dihydroxypyrimidine-4-carboxamide;
- (S)-N-(1-cyclohexylethyl)-5,6-dihydroxy-2-(quinolin-2-yl)pyrimidine-4-carboxamide;
- (R)-N-(1-cyclohexylethyl)-5,6-dihydroxy-2-(4-(4-methylpiperazin-1-yl)pyridin-2-yl)pyrimidine-4-carboxamide;
- N-(4,4-dimethylcyclohexyl)-5,6-dihydroxy-2-(4-methoxypyridin-2-yl)pyrimidine-4-carboxamide;
- (S)-2-(4-chloropyridin-2-yl)-N-(1-cyclohexylethyl)-5,6-dihydroxypyrimidine-4-carboxamide;
- (S)-N-(1-cyclohexylethyl)-5,6-dihydroxy-2-(4-methylpyridin-2-yl)pyrimidine-4-carboxamide;
- (S)-N-(1-cyclohexylethyl)-5,6-dihydroxy-2-(isoquinolin-1-yl)pyrimidine-4-carboxamide;
- (S)-2-(5-aminopyridin-2-yl)-N-(1-cyclohexylethyl)-5,6-dihydroxypyrimidine-4-carboxamide;
- (R)-N-(6-(4-((1-cyclohexylethyl)carbamoyl)-5,6-dihydroxypyrimidin-2-yl)pyridin-3-yl)oxazole-5-carboxamide;
- N-(1-cyclohexylcyclopropyl)-5,6-dihydroxy-2-(isoquinolin-3-yl)pyrimidine-4-carboxamide;
- N-(1-cyclohexylcyclopropyl)-5,6-dihydroxy-2-(4-methylpyridin-2-yl)pyrimidine-4-carboxamide;
- (R)-N-(1-cyclohexylethyl)-5,6-dihydroxy-2-(1H-pyrrolo[3,2-c]pyridin-4-yl)pyrimidine-4-carboxamide;
- (S)-N-(1-cyclohexylethyl)-5,6-dihydroxy-2-(1H-pyrrolo[3,2-c]pyridin-4-yl)pyrimidine-4-carboxamide;
- 2-(4-chloropyridin-2-yl)-N-(1-(4,4-difluorocyclohexyl)ethyl)-5,6-dihydroxypyrimidine-4-carboxamide;
- (R)-2-(4-(2-aminoethyl)pyridin-2-yl)-N-(1-cyclohexylethyl)-5,6-dihydroxypyrimidine-4-carboxamide;
- (R)-N-(1-cyclohexylethyl)-2-(5-(N-ethylsulfamoyl)pyridin-2-yl)-5,6-dihydroxypyrimidine-4-carboxamide;
- N-(1-(4,4-difluorocyclohexyl)ethyl)-5,6-dihydroxy-2-(isoquinolin-1-yl)pyrimidine-4-carboxamide;
- (R)-N-(1-cyclohexylethyl)-5,6-dihydroxy-2-(4-(2-(((1-methyl-1H-pyrrol-2-yl)methyl)amino)ethyl)pyridin-2-yl)pyrimidine-4-carboxamide;
- (R)-N-(1-cyclohexylethyl)-2-(4-(2-(dimethylamino)ethyl)pyridin-2-yl)-5,6-dihydroxypyrimidine-4-carboxamide;
- (R)-N-(1-cyclohexylethyl)-5,6-dihydroxy-2-(5-(pyrrolidin-1-ylmethyl)pyridin-2-yl)pyrimidine-4-carboxamide;
- (R)-N-(1-cyclohexylethyl)-5,6-dihydroxy-2-(5-(4-methylpiperazin-1-yl)pyridin-2-yl)pyrimidine-4-carboxamide;
- (R)-N-(1-cyclohexylethyl)-2-(4-((ethylamino)methyl)pyridin-2-yl)-5,6-dihydroxypyrimidine-4-carboxamide;
- (R)-2-(4-((benzylamino)methyl)pyridin-2-yl)-N-(1-cyclohexylethyl)-5,6-dihydroxypyrimidine-4-carboxamide;

and pharmaceutically acceptable salts, tautomers, stereoisomers thereof.

The present invention also provides a combination of a compound as defined above or pharmaceutically acceptable salts, tautomers, stereoisomers thereof with at least one further active compound for use in the treatment of an HIV-1 infection.

The present invention also provides a pharmaceutical composition comprising an effective amount of one or more compound as defined above, alone or in combination with at least one further active compound, and at least one pharmaceutically acceptable excipient for use in the treatment of HIV-1 infections.

Preferably the at least one further active compound is active against HIV.

Still preferably the at least one further active compound active against HIV is selected from the group consisting of a nucleoside reverse transcriptase inhibitor, a non-nucleoside reverse transcriptase inhibitor, a protease inhibitor, an integrase inhibitor, an entry inhibitor and a fusion inhibitor.

In a preferred embodiment the HIV-1 infection is a drug resistant HIV-1 infection.

Still preferably the drug resistant HIV-1 is resistant to at least one member selected from the group consisting of a nucleoside reverse transcriptase inhibitor, a non-nucleoside reverse transcriptase inhibitor, a protease inhibitor, an integrase inhibitor, an entry inhibitor and a fusion inhibitor.

In a preferred embodiment the technical effect of compounds of the invention is to exert inhibition of the HIV-1 nucleocapsid protein.

The present invention includes within its scope prodrugs of the compound of formula (I) or its derivatives as defined in the claims. In general, such prodrugs will be functional derivatives of the compound of formula (I) which are readily convertible *in vivo* into the required compound of formula (I). Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation *in vivo* may be, for example, as the result of some metabolic process, such as chemical or enzymatic hydrolysis of a carboxylic, phosphoric or sulphate ester, or reduction or oxidation of a susceptible functionality.

The present invention includes within its scope solvates of the compounds of formula (I) or (III) and salts thereof, for example, hydrates.

The compounds of the present invention may have asymmetric centers, chiral axes, and chiral planes (as described in: E.L. Eliel and S.H. Wilen, Stereochemistry of Carbon Compounds, John Wiley & Sons, New York, 1994, pages 1119-1190), and occur as racemates, racemic mixtures, and as individual diastereomers, with all possible isomers and mixtures thereof, including optical isomers, all such stereoisomers being included in the present invention. In addition, the compounds disclosed herein may exist as tautomers and both tautomeric forms are intended to be encompassed by the scope of the invention, even though only one tautomeric structure is depicted.

The compounds may exist in different isomeric forms, all of which are encompassed by the present invention.

When any variable occurs more than one time in any constituent, its definition on each occurrence is independent of every other occurrence. Also, combinations of substituents and variables are permissible only if such combinations result in stable compounds.

It is understood that substituents and substitution patterns on the compounds of the instant invention can be selected by one of ordinary skill in the art to provide compounds that are chemically stable and that can be readily synthesized from readily available starting materials by techniques known in the art, as well as those methods set forth below. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents. A substituent on a saturated, partially saturated or unsaturated heterocycle can be attached at any substitutable position.

As used herein, "alkyl" is intended to include both branched and straight-chain saturated aliphatic hydrocarbon groups having the specified number of carbon atoms. For example,"$C_1$-$C_6$ alkyl" is defined to include groups having 1, 2, 3, 4, 5 or 6 carbons in a linear or branched arrangement. For example, "$C_1$-$C_6$ alkyl" specifically includes methyl, ethyl, *n*-propyl, *i*-propyl, *n*-butyl, *t*-butyl, *i*-butyl, pentyl, hexyl, and so on. The term "cycloalkyl" means a monocyclic, bicyclic or polycyclic saturated aliphatic hydrocarbon group having the specified number of carbon atoms. For example, "$C_{3-10}$cycloalkyl" includes cyclopropyl, methyl-cyclopropyl, 2,2-dimethyl-cyclobutyl, 2-ethyl-cyclopentyl, cyclohexyl, and so on. In an embodiment of the invention the term "cycloalkyl" includes the groups described immediately above and further includes monocyclic unsaturated aliphatic hydrocarbon groups. For example, "cycloalkyl" as defined in this embodiment includes cyclopropyl, methyl-cyclopropyl, 2,2-dimethyl-cyclobutyl, 2-ethyl-cyclopentyl, cyclohexyl, cyclopentenyl, cyclobutenyl, 7,7-dimethylbicyclo[2.2.1]heptyl and so on. Preferred cycloalkyl groups are cyclopropyl, cyclobutyl, cyclopentyl and cyclohexyl. The term $C_{3-6}$cycloalkyl-$C_{1-3}$alkyl refers to a radical of the formula -$R_aR_c$ wherein $R_a$ is an alkylene chain having from 1 to 3 carbon atoms and $R_c$ is a cycloalkyl group having 3 to 6 carbon atoms.

"Alkoxy" represents an alkyl group of indicated number of carbon atoms attached through an oxygen bridge. "Alkoxy" therefore encompasses the definitions of alkyl above. In particular the term $C_{1-6}$alkoxy indicates an alkyl chain having the specified number of carbons and attached through an oxygen bridge. Examples of suitable $C_{1-6}$alkoxy groups include methoxy, ethoxy, n-propoxy, *i*-propoxy, *n*-butoxy, *s*-butoxy and *t*-butoxy. The preferred alkoxy group is methoxy. The terms "halo$C_{1-6}$alkyl" and "halo$C_{1-6}$alkoxy" mean a $C_{1-6}$alkyl or $C_{1-6}$alkoxy group in which one or more (in particular, 1 to 3) hydrogen atoms have been replaced by halogen atoms, especially fluorine or chlorine atoms. Preferred are fluoro$C_{1-6}$alkyl and fluoro$C_{1-6}$alkoxy groups, in particular fluoro$C_{1-3}$alkyl and fluoro$C_{1-3}$alkoxy groups, for example, $CF_3$, $CHF_2$, $CH_2F$, $CH_2CH_2F$, $CH_2CHF_2$, $CH_2CF_3$, $OCF_3$, $OCHF_2$, $OCH_2F$, $OCH_2CH_2F$, $OCH_2CHF_2$ or $OCH_2CF_3$, and most especially $CF_3$, $OCF_3$ and $OCHF_2$.

The term "hydroxy$C_{1-6}$alkyl" means a $C_{1-6}$alkyl group in which one or more (in particular, 1 to 3) hydrogen atoms have been replaced by hydroxy groups. Preferred are $CH_2OH$, $CH_2CHOH$ and $CHOHCH_3$.

As used herein, the term "$C_{2-6}$alkenyl" refers to a non-aromatic hydrocarbon radical, straight or branched, containing from 2 to 6 carbon atoms and at least one carbon to carbon double bond. Preferably one carbon to carbon double bond is present, and up to four non-aromatic carbon-carbon double bonds may be present. Alkenyl groups include ethenyl, propenyl, butenyl and 2-methylbutenyl. The straight or branched portion of the alkenyl group may contain double bonds and may be substituted if a substituted alkenyl group is indicated. Preferred alkenyl groups include ethenyl and propenyl.

The term "$C_{2-6}$alkynyl" refers to a hydrocarbon radical straight or branched, containing from 2 to 6 carbon atoms and at least one carbon to carbon triple bond. Up to three carbon-carbon triple bonds may be present. Alkynyl groups include ethynyl, propynyl, butynyl, 3-methylbutynyl and so on. The straight or branched portion of the alkynyl group may

contain triple bonds and may be substituted if a substituted alkynyl group is indicated. Preferred alkynyl groups include ethynyl and propynyl.

As used herein, "$C_{6-10}$aryl" is intended to mean any stable monocyclic or bicyclic carbon ring of 6 to 10 atoms, wherein at least one ring is aromatic. Examples of such aryl elements include phenyl, naphthyl, tetrahydronaphthyl, indanyl and tetrahydrobenzo[7]annulene. The preferred aryl group is phenyl or naphthyl, especially phenyl.

The term aryl-$C_{1-3}$alkyl refers to a radical of the formula $-R_aR_c$ wherein $R_a$ is an alkylene chain having from 1 to 3 carbon atoms and $R_c$ is an aryl group as defined above.

The term $C_{6-10}$aryl-$C_{1-6}$alkyl refers to a radical of the formula $-R_aR_c$ wherein $R_a$ is an alkylene chain having from 1 to 6 carbon atoms and $R_c$ is a $C_{6-10}$aryl aryl group as defined above.

As used herein, "heteroaryl" is intended to mean any stable monocyclic or bicyclic ring containing 1, 2, 3 or 4 heteroatoms independently selected from N, O and S, wherein at least one ring is aromatic. In particular the term heteroaryl includes 5 membered aromatic heterocycles containing 1, 2, 3 or 4 heteroatoms independently selected from N, O and S , but not more than one of which is O or S; 6 membered aromatic heterocycles containing 1, 2 or 3 nitrogen atoms; or a 7-13 membered aromatic heterocycle containing heteroatoms independently selected from N, O or S of 5 to 10 atoms, wherein at least one ring is aromatic.

Examples of particular heteroaryl of this invention are benzimidazolyl, benzofurandionyl, benzofuranyl, benzofurazanyl, benzopyrazolyl, benzotriazolyl, benzothienyl, benzoxazolyl, benzoxazolonyl, benzothiazolyl, benzothiadiazolyl, benzodioxolyl, benzoxadiazolyl, benzoisoxazolyl, benzoisothiazolyl, chromenyl, chromanyl, isochromanyl, carbazolyl, carbolinyl, cinnolinyl, epoxidyl, furanyl, furazanyl, imidazolyl, imidazothiazolyl, indolinyl, indolyl, indolizinyl, iso indo linyl, indazolyl, isobenzo furanyl, isoindolyl, isoquinolyl, isothiazolyl, isoxazolyl, naphthpyridinyl, oxadiazolyl, oxazolyl, oxazolinyl, oxoquinazolinyl isoxazolinyl, oxetanyl, purinyl, pyranyl, pyrazinyl, pyrazolyl, pyridazinyl, pyridopyridinyl, pyridazinyl, pyridinyl, pyrimidinyl, triazinyl, tetrazinyl, pyrrolyl, quinazolinyl, quinolyl, quinoxalinyl, quinolizinyl, tetrazolyl, tetrazolopyridyl, thiadiazolyl, thiazolidinyl, thiazolyl, thienyl, triazolyl, imidazopyridinyl, phthalazinyl, naphthyridinyl, quinazolinyl, pteridinyl and N-oxides thereof. Attachment of a heterocycles substituent can occur via a carbon atom or via a heteroatom.

The term "$C_{3-15}$heterocyclyl" refers to a non-aromatic 3- to 15-member ring radical, which consists of carbon atoms and at least one heteroatom of nitrogen, phosphorus, oxygen or sulfur. The heteroicyclic ring may be a mono-, bi-, tri-, or tetracyclic ring system, which may include fused, bridged or spiro ring systems, and the nitrogen, phosphorus, carbon, oxygen, or sulfur atoms in the heterocyclic ring radical may be optionally oxidized to various oxidation states. In addition, the nitrogen atom may be optionally quaternized.

The term $C_{3-15}$heterocyclyl-$C_{1-3}$alkyl refers to a radical of the formula $-R_aR_c$ wherein $R_a$ is an alkylene chain having from 1 to 3 carbon atoms and $R_c$ is an heterocyclyl group as defined above.

The term heteroaryl-$C_{1-3}$alkyl refers to a radical of the formula $-R_aR_c$ wherein $R_a$ is an alkylene chain having from 1 to 3 carbon atoms and $R_c$ is an heteroaryl group as defined above. Examples of particular heterocyclyl of the invention are tetrahydrofuranyl, tetrahydropyranyl, tetrahydrothiopyranyl, tetrahydroisoquinolinyl, azetidinyl, 1,4-dioxanyl, hexahydroazepinyl, piperazinyl, piperidyl, pyridin-2-onyl, pyrrolidinyl, imidazolinyl, pyrazolinyl, pyrrolinyl, morpholinyl, thiomorpholinyl, dihydrobenzoimidazolyl, dihydrobenzofuranyl, dihydrobenzothiophenyl, dihydrobenzoxazolyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, dihydroisochromenyl, dihydroimidazolonyl, dihydrotriazolonyl, dihydrobenzodioxinyl, dihydrothiazolopyrimidinyl, methylenedioxybenzoyl, tetrahydrofuranyl, tetrahydrothienyl, tetrahydroquinolinyl, thiazolidinonyl, imidazolonyl, isoindolinonyl, octahydroquinolizinyl, octahydroisoindolyl, azabicycloheptanyl, chromenonyl, triazolopyrimidinyl, dihydrobenzoxazinyl, thiazolotriazolyl, azoniabicycloheptanyl, azoniabicyclooctanyl. Attachment of a heterocyclyl substituent can occur via a carbon atom or via a heteroatom.

A preferred 4 membered saturated heterocycle is azetidine.

A preferred 5 membered saturated heterocycle is tetrahydrofurane.

Preferred 6 membered saturated or partially saturated heterocycles are pyrrolidinyl, piperidyl, piperazinyl, morpholinyl, thiomorpholinyl and thiazolidinyl.

Preferred 5 membered heteroaryls are thienyl, thiazolyl, pyrazolyl, isoxazolyl, imidazolyl, thiadiazolyl, oxazolyl, triazolyl, tetrazolyl, furyl and oxadiazolyl.

Preferred 6 membered heteroaryls are pyridinyl and pyrymidinyl.

Preferred 8-10 membered heteroaryls are benzothienyl, indolyl, benzothiadiazolyl, benzoxadiazolyl, thiazolotriazolyl, dihydrobenzodioxinyl, dihydrothiazolopyrimidinyl, dihydrobenzoxazinyl, dihydrobenzofuranyl, benzothiazolyl, quinolinyl, isoquinolinyl, benzimidazolyl, benzofuranyl, dihydrobenzoxazolyl, dihydroindolyl, dihydroquinazolinyl, dihydrophthalazinyl, indazolyl, benzisoxazolyl, benzotriazolyl, dihydroisoindolyl, tetrahydronaphthyridinyl, triazolopyrimidinyl and tetrahydroquinolinyl.

As used herein, the term 'halogen' refers to fluorine, chlorine, bromine and iodine, of which fluorine, chlorine

and bromine are preferred.

Included in the instant invention is the free base of compounds of formula (I), as well as the pharmaceutically acceptable salts and stereoisomers thereof. Some of the specific compounds exemplified herein are the protonated salts of amine compounds. Compounds of formula (I) containing one or more N atoms may be protonated on any one, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of formula (I). The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contain a basic or acidic moiety by conventional chemical methods. Generally, the salts of the basic compounds are prepared either by ion exchange chromatography or by reaction of the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base.

Thus, pharmaceutically acceptable salts of the compounds of this invention include the conventional non-toxic salts of the compounds of this invention as formed by reaction of a basic instant compound with an inorganic or organic acid. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Preferably, a pharmaceutically acceptable salt of this invention contains one equivalent of a compound of formula (I) and 1, 2 or 3 equivalent of an inorganic or organic acid. More particularly, pharmaceutically acceptable salts of this invention are the tartrate, trifluoroacetate or the chloride salts.

When the compound of the present invention is acidic, suitable "pharmaceutically acceptable salts" refers to salts prepared form pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,$N^1$-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine tripropylamine, tromethamine and the like.

The preparation of the pharmaceutically acceptable salts described above and other typical pharmaceutically acceptable salts is more fully described by Berg et al., "Pharmaceutical Salts," J. Pharm. Sci., 1977:66:1-19.

It will also be noted that the compounds of the present invention are potentially internal salts or zwitterions, since under physiological conditions a deprotonated acidic moiety in the compound, such as a carboxyl group, may be anionic, and this electronic charge might then be balanced off internally against the cationic charge of a protonated or alkylated basic moiety, such as a quaternary nitrogen atom.

The compounds of the present inventions are useful in the inhibition of HIV nucleocapsid, the prevention or treatment of infection by human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by e.g., blood transfusion, exchange of body fluids, bites, accidental needle stick, or exposure to patient blood during surgery.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

The invention also provides pharmaceutical compositions comprising one or more compounds of this invention and a pharmaceutically acceptable carrier. The pharmaceutical compositions containing the active ingredient may be

in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. The tablets may be uncoated or they may be coated by known techniques to mask the unpleasant taste of the drug or delay disintegration and absorption in the gastrointestinal tract and thereby provide a sustained action over a longer period. For example, a water soluble taste masking material such as hydroxypropyl-methylcellulose or hydroxypropylcellulose, or a time delay material such as ethyl cellulose, cellulose acetate butyrate maybe employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil.

Aqueous suspensions contain the active material in admixture with excipients suitable for the manufacture of aqueous suspensions. Such excipients are suspending agents, for example sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example arachis oil, olive oil, sesame oil or coconut oil, or in mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an anti-oxidant such as butylated hydroxyanisol or alpha-tocopherol.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present. These compositions may be preserved by the addition of an anti-oxidant such as ascorbic acid.

The pharmaceutical compositions of the invention may also be in the form of an oil-in-water emulsion. The oily phase may be a vegetable oil, for example olive oil or arachis oil, or a mineral oil, for example liquid paraffin or mixtures of these. Suitable emulsifying agents may be naturally occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening, flavoring agents, preservatives and antioxidants.

Syrups and elixirs may be formulated with sweetening agents, for example glycerol, propylene glycol, sorbitol or sucrose. Such formulations may also contain a demulcent, a preservative, flavoring and coloring agents and antioxidant.

The pharmaceutical compositions may be in the form of a sterile injectable aqueous solution. Among the acceptable vehicles and solvents that may be employed are water, Ringer's solution and isotonic sodium chloride solution.

The sterile injectable preparation may also be a sterile injectable oil-in-water microemulsion where the active ingredient is dissolved in the oily phase. For example, the active ingredient may be first dissolved in a mixture of soybean oil and lecithin. The oil solution may be then introduced into a water and glycerol mixture and processed to form a microemulstion. The injectable solutions or microemulsions may be introduced into a patient's blood stream by local bolus injection.

The pharmaceutical compositions may be in the form of a sterile injectable aqueous or oleagenous suspension for intramuscular and subcutaneous administration. This suspension may be formulated according to the known art using those suitable dispersing or wetting agents and suspending agents which have been mentioned above. The sterile injectable preparation may also be a sterile injectable solution or suspension in a non-toxic parenterally acceptable

diluent or solvent, for example as a solution in 1,3-butanediol. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

For topical use, creams, ointments, jellies, solutions or suspensions, etc., containing the compound of the invention are employed. The compounds of the present invention can be administered in intranasal form via topical use of suitable intranasal vehicles and delivery devices, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

When a compound according to this invention is administered into a human subject, the daily dosage regimen will normally be determined by the prescribing physician with the dosage generally varying according to the age, weight, sex and response of the individual patient, as well as the severity of the patient's symptoms.

The term "administration" and variants thereof (e.g., "administering" a compound) in reference to a compound of the invention means introducing the compound or a prodrug of the compound into the system of the subject in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician.

When a compound of the present invention is administered into a human subject, the daily dosage regimen will normally be determined by the prescribing physician, with the dosage generally varying according to the age, weight, and response of the individual patient, as well as the severity of the patient's symptoms. In one exemplary application, oral dosages of the present invention will range between about 0.01 mg per kg of body weight per day (mg/kg/day) to about 100 mg/kg/day, preferably 0.01 to 10 mg/kg/day, and most preferably 0.1 to 5.0 mg/kg/day.

As used herein, the term "nucleocapsid protein" or "NC protein" refers to the retroviral nucleocapsid protein, which is an integral part of the virion nucleocapsid where it coats the dimeric RNA genome, as described by Huang (1997) J. Virol. 71: 4378-4384; lapadat-Tapolsky (1997) J.Mol. Biol. 268: 250-260. HIV-1s nucleocapsid protein is termed "NCp7", see also Demene (1994) Biochemistry 33: 11707-11716.

HIV-1 nucleocapsid protein inhibition may be measured by any known methods, in particular by measuring the NC-induced destabilization of the secondary structure of cTAR DNA.

Certain compounds within the scope of the invention are capable of ejecting zinc from a zinc finger at some measurable rate. The rate of this effect is not necessarily indicative of potency for antiviral activity, however. Compounds which eject zinc slowly, i.e., with slow kinetics, are preferable for some uses, especially for certain in vivo applications. A "weak cation ejector" compound of the present invention would have a zinc ejection rate of about 0.86 RFU/min, or lower, as measured by the Trp37 zinc finger fluorescence assay. A "high" ejection rate would be in the range of approximately 8 RFU/min, or higher.

In certain embodiments, the zinc finger-containing protein is a viral protein. In certain embodiments, the viral protein is selected from the group consisting of a nucleocapsid protein, a Gag protein, and a Gag-Pol protein. The contacting of the protein with the compound or salt is performed in vitro or in vivo. In a preferred embodiment, the protein is that of an HIV-1 retrovirus.

The invention further provides a method for inactivating an HIV-1 retrovirus, the method comprising contacting the virus with a compound or salt of the present invention, whereby contacting the virus with said compound or salt inactivates the virus. In the above-described embodiments, preferably the contacting of the virus with the compound or salt is performed in vivo.

In some embodiments, the compound or salt of the invention is administered to inhibit the transmission of the virus. In this regard, a purpose for inhibiting the transmission of the virus refers to transmission of the virus from an infected individual to another individual. In some embodiments, inhibition of the transmission of the virus can be achieved

by administration of the compound or salt intra-vaginally or intra-rectally. In certain other embodiments, inhibition of the transmission of the virus can be achieved by administration of the compound or salt parenterally, intrathecally, subcutaneously, or orally. In these embodiments, the compound or salt of the invention can be administered to a human as a pharmaceutical formulation. In other embodiments, the compound or salt of the invention can be administered to an animal as a veterinary pharmaceutical formulation.

The present invention is also directed to combinations of the HIV nucleocapsid inhibitor compounds with one or more agents useful in the treatment of AIDS. For example, the compounds of this invention may be effectively administered, whether at periods of pre-exposure and/or post-exposure, in combination with effective amounts of the AIDS antivirals, immunomodulators, antiinfectives, or vaccines, such as those cited herein.

In other embodiments of the above-described method, the method further comprises contacting the virus with another anti-HIV agent. The anti-HIV agent can be any suitable anti-HIV active substance. Examples of the other anti-HIV agents and other anti-HIV active substances to be used for a multiple drug combination therapy include an anti-HIV antibody or other antibody, an HIV vaccine or other vaccine, immunostimulants such as interferon, interferon agonist and the like, a ribozyme against HIV, an HIV antisense drug, a nucleoside/nucleotide HIV reverse transcriptase inhibitor (NRTI), a non-nucleoside reverse transcriptase inhibitor (NNRTI), an HIV protease inhibitor, an HIV integrase inhibitor, an inhibitor of attachment between a receptor (CD4, CXCR4, CCR5 and the like) of a host cell recognized by virus and the virus (CCR5 antagonist and the like), a DNA polymerase inhibitor or DNA synthesis inhibitor, a medicament acting on HIVp24, an HIV fusion inhibitor, an IL-2 agonist or antagonist, a TNF-$\alpha$ antagonist, an $\alpha$-glucosidase inhibitor, a purine nucleoside phosphorylase inhibitor, an apoptosis agonist or inhibitor, a cholinesterase inhibitor, an immunomodulator and the like.

Specific examples of nucleoside or non-nucleoside HIV reverse transcriptase inhibitors include Retrovir[R] (zidovudine), Epivir[R] (lamivudine), Zerit[R] (sanilvudine), Videx[R] (didanosine), Hivid[R] (zalcitabine), Ziagen[R] (abacavir sulfate), Viramune[R] (nevirapine), Stocrin[R] or Sustiva[R] (efavirenz), Rescriptor [R] (delavirdine mesylate), Combivir[R] (zidovudine+lamivudine), Trizivir[R] (abacavir sulfate+lamivudine+zidovudine), Coactinon[R] (emivirine), Phosphonovir[R], Coviracil[R], alovudine (3'-fluoro-3'-deoxythymidine), Thiovir[R] (thiophosphonoformic acid), capravirin (5-[(3,5-dichlorophenyl)thio]-4-isopropyl-1-(4-pyridylmethyl)imidazole-2-methanol carbamic acid), tenofovir disoproxil fumarate ((R)-[[2-(6-amino-9H-purin-9-yl)-1-methylethoxy]methyl]phosphonic acid bis(isopropoxycarbonyloxymethyl)ester fumarate), tenofovir alafenamide fumarate, tenofovir alafenamide hemifumarate, DPC-083 ((4S)-6-chloro-4-[(1E)-cyclopropylethenyl]-3,4-dihydro-4-trifluoromethyl-2(1H)-quinazolinone), DPC-961 ((4S)-6-chloro-4-(cyclopropylethynyl)-3,4-dihydro-4-(trifluoromethyl)-2(1H)-quinazolinone), DAPD ((-)-$\beta$-D-2,6-diaminopurine dioxolane), MSK-055, MSA-254, MSH-143, NV-01, TMC-120, DPC-817, GS-7340, TMC-125, SPD-754, D-A4FC, capravirine, UC-781, emtricitabine, alovudine, Phosphazid, BCH-10618, DPC-083, Etravirine, BCH-13520, MIV-210, Abacavir sulfate/lamivudine, GS-7340, GW-5634, GW-695634, TMC-278 and the like, wherein [R] means a registered trademark and the names of medicaments without [R] are generic names (ex. INN) or code number named by company (hereinafter the same).

Specific examples of the HIV protease inhibitor include Crixivan[R] (indinavir sulfate ethanolate), saquinavir, Invirase[R] (saquinavir mesylate), Norvir[R] (ritonavir), Viracept[R] (nelfinavir mesylate), lopinavir, Prozei[R] (amprenavir), Kaletra[R] (ritonavir+lopinavir), mozenavir dimesylate ([4R-((4$\alpha$,5$\alpha$,6$\beta$)]-1,3-bis[(3-aminophenyl)methyl]-hexahydro-5,6-dihydroxy-4,7-bis(phenylmethyl)-2H-1,3-diazepin-2-one dimethanesulfonate), tipranavir (3'-[(1R)-1-[(6R)-5,6-dihydro-4-hydroxy-2-oxo-6-phenylethyl-6-propyl-2H-pyran-3-yl]propyl]-5-(trifluoromethyl)-2-pyridinesulfonamide), lasinavir (N-[5(S)-(tert-butoxycarbonylamino)-4(S)-hydroxy-6-phenyl-2(R)-(2,3,4-trimethoxybenzyl)hexanoyl]-L-valine 2-methoxyethylenamide), KNI-272 ((R)-N-tert-butyl-3-[(2S,3S)-2-hydroxy-3-N-[(R)-2-N-(isoquinolin-5-yloxyacetyl)amino-3-methylthiopropanoyl]amino-4-phenylbutanoyl]-5,5-dimethyl-1,3-thiazolidine-4-carboxamide), GW-433908, TMC-126, DPC-681, buckminsterfullerene, MK-944A (MK944 (N-(2(R)-hydroxy-1(S)-indanyl)-2(R)-phenylmethyl-4(S)-hydroxy-5-[4-(2-benzo[b]furanylmethyl)-2(S)-(tert-butylcarbamoyl)piperazin-1-yl]pentanamide)+indinavir sulfate), JE-2147 ([2(S)-oxo-4-phenylmethyl-3(S)-[(2-methyl-3-oxy)phenylcarbonylamino]-1-oxabutyl]-4-[(2-methylphenyl)methylamino]carbonyl-4(R)-5,5-dimethyl-1,3-thiazole), BMS-232632 (dimethyl (3S,8S,9S,12S)-3,12-bis(1,1-dimethylethyl)-8-hydroxy-4,11-dioxo-9-(phenylmethyl)-6-[[4-(2-pyridinyl)phenyl]methyl]-2,5,6,10,13-pentaazatetradecanedicarboxylate), DMP-850 ((4R,5S,6S,7R)-1-(3-amino-1H-indazol-5-ylmethyl)-4,7-dibenzyl-3-butyl-5,6-dihydroxyperhydro-1,3-diazepin-2-one), DMP-851, RO-0334649, Nar-DG-35, R-944, VX-385, TMC-114, Tipranavir, Fosamprenavir sodium, Fosamprenavir calcium, Darunavir, GW-0385, R-944, RO-033-4649, AG-1859 and the like.

The HIV integrase inhibitor is exemplified by S-1360, L-870810, ISENTRESS[R] (Raltegravir), JTK-303 (Elvitegravir), S/GSK1 349572 or Tivicay[R] (Dolutegravir) and the like, the DNA polymerase inhibitor or DNA synthesis inhibitor is exemplified by Foscavir[R], ACH-126443 (L-2',3'-didehydro-dideoxy-5-fluorocytidine), entecavir ((1S,3S,4S)-9-[4-hydroxy-3-(hydroxymethyl)-2-methylenecyclopentyl]guanine), calanolide A ([10R-(10$\alpha$,11$\beta$,12$\alpha$)]-11,12-dihydro-12-hydroxy-6,6,10,11-tetramethyl-4-propyl-2H,6H,10H-benzo[1,2-b:3,4-b':5,6-b"]tripyran-2-one), calanolide B, NSC-674447 (1,1'-azobisformamide), Iscador (viscum alubm extract), Rubitecan and the like, the HIV antisense drug is exemplified by HGTV-43, GEM-92 and the like, the anti-HIV antibody or other antibody is exemplified by NM-01, PRO-367, KD-247, Cytolin[R], TNX-355 (CD4 antibody), AGT-1, PRO-140 (CCR5 antibody), Anti-CTLA-4MAb and the like, the HIV vaccine

or other vaccine is exemplified by ALVAC[R], AIDSVAX[R], Remune[R], HIV gp41 vaccine, HIV gp120 vaccine, HIV gp140 vaccine, HIV gp160 vaccine, HIV p17 vaccine, HIV p24 vaccine, HIV p55 vaccine, AlphaVax Vector System, canarypox gp160 vaccine, AntiTat, MVA-F6 Nef vaccine, HIV rev vaccine, C4-V3 peptide, p2249f, VIR-201, HGP-30W, TBC-3B, PARTICLE-3B, Antiferon (interferon-α vaccine) and the like, the interferon or interferon agonist is exemplified by Sumiferon[R], MultiFeron[R], interferon-τ, Reticulose, human leukocyte interferon α and the like, the CCR5 antagonist is exemplified by SCH-351125 and the like, the medicament acting on HIV p24 is exemplified by GPG-NH2 (glycyl-prolyl-glycinamide) and the like, the HIV fusion inhibitor is exemplified by FP-21399 (1,4-bis[3-[(2,4-dichlorophenyl)carbonylamino]-2-oxo-5,8-disodium sulfonyl]naphthyl-2,5-dimethoxyphenyl-1,4-dihydrazone), T-1249, Synthetic Polymeric Construction No3, pentafuside, FP-21399, PRO-542, Enfuvirtide and the like, the IL-2 agonist or antagonist is exemplified by interleukin-2, Imunace[R], Proleukin[R], Multikine[R], Ontak[R] and the like, the TNF-α antagonist is exemplified by Thalomid[R] (thalidomide), Remicade[R] (infliximab), curdlan sulfate and the like, the α-glucosidase inhibitor is exemplified by Bucast[R] and the like, the purine nucleoside phosphorylase inhibitor is exemplified by peldesine (2-amino-4-oxo-3H,5H-7-[(3-pyridyl)methyl]pyrrolo[3,2-d]pyrimidine) and the like, the apoptosis agonist or inhibitor is exemplified by Arkin Z[R], Panavir[R], Coenzyme Q10 (2-deca(3-methyl-2-butenylene)-5,6-dimethoxy-3-methyl-p-benzoquinone) and the like, the cholinesterase inhibitor is exemplified by Cognex[R] and the like, and the immunomodulator is exemplified by Imunox[R], Prokine[R], Met-enkephalin (6-de-L-arginine-7-de-L-arginine-8-de-L-valinamide-adrenorphin), WF-10 (10-fold dilute tetrachlorodecaoxide solution), Perthon, PRO-542, SCH-D, UK-427857, AMD-070, AK-602 and the like.

In addition, Neurotropin[R], Lidakol[R], Ancer 20[R], Ampligen[R], Anticort[R], Inactivin[R], PRO-2000, Rev M10 gene, HIV specific cytotoxic T cell (CTL immunotherapy, ACTG protocol 080 therapy, CD4-ζ gene therapy), SCA binding protein, RBC-CD4 complex, Motexafin gadolinium, GEM-92, CNI-1493, (±)-FTC, Ushercell, D2S, BufferGel[R], VivaGel[R], Glyminox vaginal gel, sodium lauryl sulfate, 2F5, 2F5/2G12, VRX-496, Ad5gag2, BG-777, IGIV-C, BILR-255 and the like are exemplified.

The compound of the present invention can be combined with one or more (e.g., 1 or 2) kinds of other anti-HIV active substances (to be also referred to as other anti-HIV agents), and used as an anti-HIV agent and the like for the prophylaxis or treatment of HIV infection. The "other anti-HIV agents" and "other anti-HIV active substances" to be used for a multiple drug combination therapy with the compound of the present invention, includes HIV reverse transcriptase inhibitors (tenofovir disoproxil fumarate, tenofovir alafenamide, emtricitabine, abacavir, lamivudine, zidovudine, efavirenz, nevirapine, etravirine, rilpivirine), HIV protease inhibitors (darunavir, atazanavir, lopinavir), integrase inhibitors (raltegravir, elvitegravir, dolutegravir), entry inhibitors (enfuvirtide, maraviroc).

Two or three, or even a greater number of medicaments can be used in combination or processed into a combination drug, wherein a combination of medicaments having different action mechanisms is one of the preferable embodiments. In addition, selection of medicaments free of side effect duplication is preferable.

When used in combination the different compounds may be administered simultaneous, or sequentially in any order. They may be part of a single dosage unit or multiple dosage unit.

It will be understood that the scope of combinations of the compounds of this invention with AIDS antivirals, immunomodulators, anti-infectives or vaccines is not limited to the list of compounds herein cited, but includes in principle any combination with any pharmaceutical composition useful for the treatment of AIDS.

In other embodiments, the contacting of the virus with the compound or salt is performed on a blood product, blood plasma, nutrient media, protein, a pharmaceutical, a cosmetic, a sperm or oocyte preparation, cells, cell cultures, bacteria, viruses, food, drink, implant or prosthesis. In these embodiments, the contacting of the virus with the compound or salt is performed in vitro

These and other aspects of the invention will be apparent from the teachings contained herein.

## DETAILED DESCRIPTION OF THE INVENTION

## MATERIALS AND METHODS

### Chemistry

a) General procedures.

The general synthetic strategy for the synthesis of the pyrimidine nucleus starts from the appropriate carbonitrile (A) which is converted to the corresponding amidoxime (B) that is condensed with dimethyl acetylene dicarboxylate. The adduct thus formed undergoes a thermally induced cyclisation to afford the desired 2-substituted dihydroxypyrimidine methyl ester analog (C). This key intermediate can be converted into the final compounds by methods well known in the art. Suitable methods for the synthesis of carboxamides or esters are described for example in Jerry March, Advanced Organic Chemistrym 3rd edition, John Wiley & Sons, 19856, pp. 370-376.

The procedure outlined above and synthesized in the following Scheme 1 has been applied on multigram scale

for the synthesis of the compounds of the present invention.

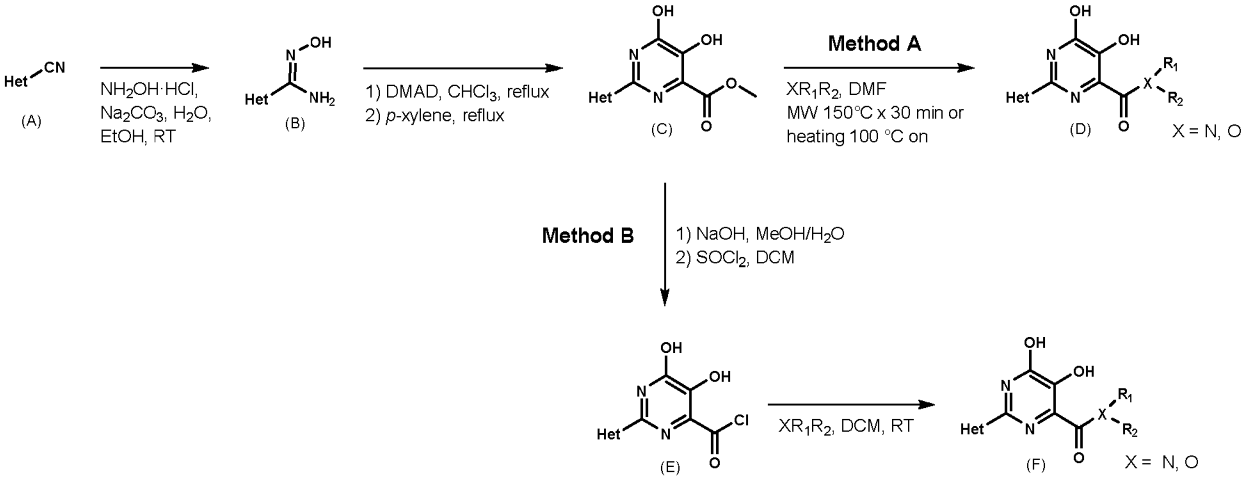


**Scheme 1**

The compounds prepared within the instant invention are listed in Table 1.

Table 1. Compounds of the invention

| Compound No | Structure | IUPAC Name |
|---|---|---|
| 1 | 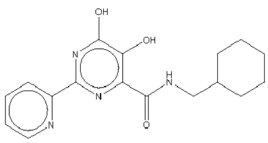 | *N*-(cyclohexylmethyl)-5,6-dihydroxy-2-(pyridin-2-yl) pyrimidine-4-carboxamide |
| 2 | 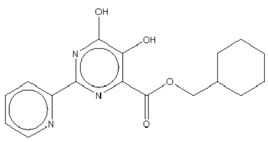 | cyclohexylmethyl 5,6-dihydroxy-2-(pyridin-2-yl) pyrimidine-4-carboxylate |
| 3 | 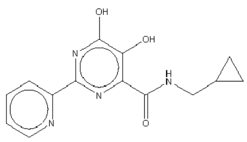 | *N*-(cyclopropylmethyl)-5,6-dihydroxy-2-(pyridin-2-yl) pyrimidine-4-carboxamide |
| 4 | 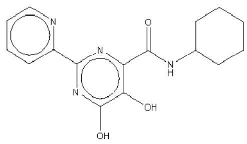 | *N*-cyclohexyl-5,6-dihydroxy-2-(pyridin-2-yl) pyrimidine-4-carboxamide |
| 5 | 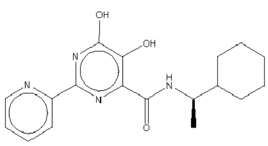 | (*R*)-*N*-(1-cyclohexylethyl)-5,6-dihydroxy-2-(pyridin-2-yl)pyrimidine-4-carboxamide |

(continued)

| Compound No | Structure | IUPAC Name |
|---|---|---|
| 6 | 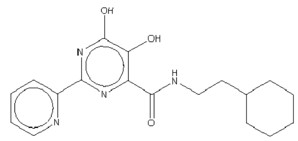 | *N*-(2-cyclohexylethyl)-5,6-dihydroxy-2-(pyridin-2-yl) pyrimidine-4-carboxamide |
| 7 | 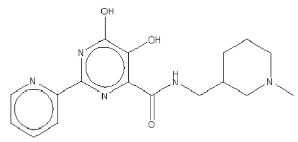 | 5,6-dihydroxy-*N*-((1-methylpiperidin-3 - yl)methyl)-2-(pyridin-2-yl)pyrimidine-4-carboxamide |
| 8 | 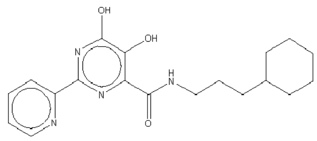 | *N*-(3-cyclohexylpropyl)-5,6-dihydroxy-2-(pyridin-2-yl) pyrimidine-4-carboxamide |
| 9 | 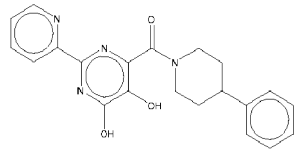 | (5,6-dihydroxy-2-(pyridin-2-yl)pyrimidin-4-yl)(4-phenylpiperidin-1-yl)methanone |
| 10 | 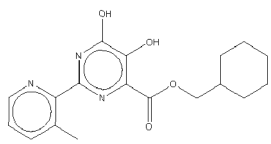 | cyclohexylmethyl 5,6-dihydroxy-2-(3-methylpyridin-2-yl)pyrimidine-4-carboxylate |
| 11 | 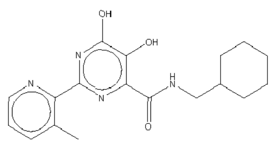 | *N*-(cyclohexylmethyl)-5,6-dihydroxy-2-(3-methylpyridin-2-yl)pyrimidine-4-carboxamide |
| 12 | 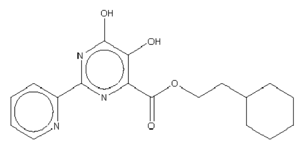 | 2-cyclohexylethyl 5,6-dihydroxy-2-(pyridin-2-yl) pyrimidine-4-carboxylate |
| 13 | 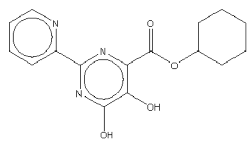 | cyclohexyl 5,6-dihydroxy-2-(pyridin-2-yl)pyrimidine-4-carboxylate |
| 14 | 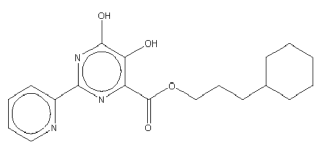 | 3-cyclohexylpropyl 5,6-dihydroxy-2-(pyridin-2-yl) pyrimidine-4-carboxylate |
| 15 | 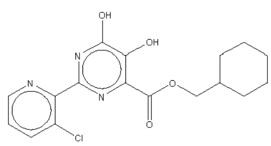 | cyclohexylmethyl 2-(3-chloropyridin-2-yl)-5,6-dihydroxypyrimidine-4-carboxylate |

(continued)

| Compound No | Structure | IUPAC Name |
|---|---|---|
| 16 | 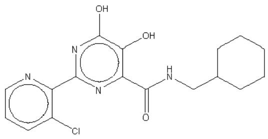 | 2-(3-chloropyridin-2-yl)-*N*-(cyclohexylmethyl)-5,6-dihydroxypyrimidine-4-carboxamide |
| 17 | 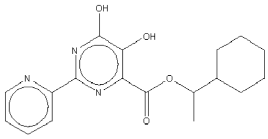 | 1-cyclohexylethyl 5,6-dihydroxy-2-(pyridin-2-yl) pyrimidine-4-carboxylate |
| 18 | 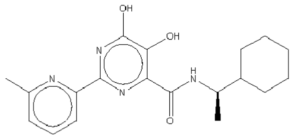 | (*R*)-*N*-(1-cyclohexylethyl)-5,6-dihydroxy-2-(6-methylpyridin-2-yl)pyrimidine-4-carboxamide |
| 19 | 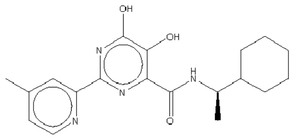 | (*R*)-*N*-(1-cyclohexylethyl)-5,6-dihydroxy-2-(4-methylpyridin-2-yl)pyrimidine-4-carboxamide |
| 20 | 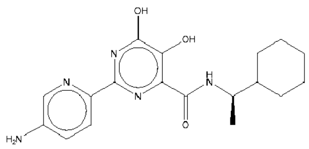 | (*R*)-2-(5-aminopyridin-2-yl)-*N*-(1-cyclohexylethyl)-5,6-dihydroxypyrimidine-4-carboxamide |
| 21 | 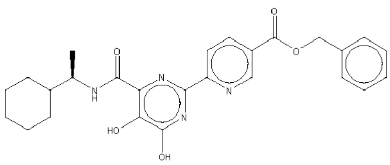 | benzyl (*R*)-6-(4-((1-cyclohexylethyl)carbamoyl)-5,6-dihydroxypyrimidin-2-yl)nicotinate |
| 22 | 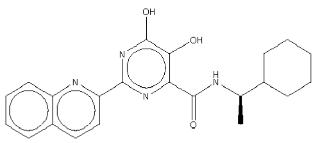 | (*R*)-*N*-(1-cyclohexylethyl)-5,6-dihydroxy-2-(quinolin-2-yl)pyrimidine-4-carboxamide |
| 23 | 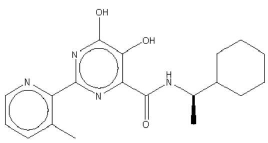 | (*R*)-*N*-(1-cyclohexylethyl)-5,6-dihydroxy-2-(3-methylpyridin-2-yl)pyrimidine-4-carboxamide |
| 24 | 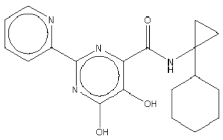 | *N*-(1-cyclohexylcyclopropyl)-5,6-dihydroxy-2-(pyridin-2-yl)pyrimidine-4-carboxamide |

(continued)

| Compound No | Structure | IUPAC Name |
|---|---|---|
| 25 | 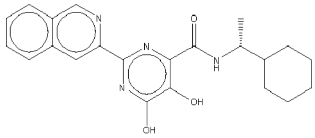 | (*R*)-*N*-(1-cyclohexylethyl)-5,6-dihydroxy-2-(isoquinolin-3-yl)pyrimidine-4-carboxamide |
| 26 | 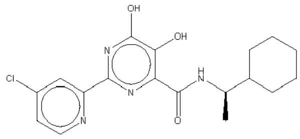 | (*R*)-2-(4-chloropyridin-2-yl)-*N*-(1-cyclohexylethyl)-5,6-dihydroxypyrimidine-4-carboxamide |
| 27 | 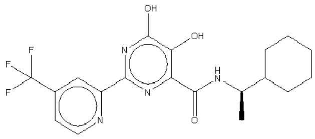 | (*R*)-*N*-(1-cyclohexylethyl)-5,6-dihydroxy-2-(4-(trifluoromethyl)pyridin-2-yl)pyrimidine-4-carboxamide |
| 28 | 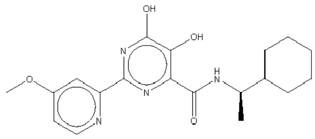 | (*R*)-*N*-(1-cyclohexylethyl)-5,6-dihydroxy-2-(4-methoxypyridin-2-yl)pyrimidine-4-carboxamide |
| 29 | 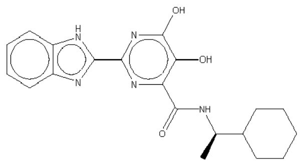 | (*R*)-2-(1*H*-benzo[d]imidazol-2-yl)-*N*-(1-cyclohexylethyl)-5,6-dihydroxypyrimidine-4-carboxamide |
| 30 | 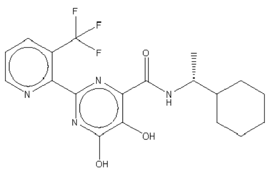 | (*R*)-*N*-(1-cyclohexylethyl)-5,6-dihydroxy-2-(3-(trifluoromethyl)pyridin-2-yl)pyrimidine-4-carboxamide |
| 31 | 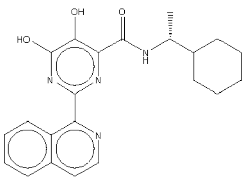 | (*R*)-N-(1-cyclohexylethyl)-5,6-dihydroxy-2-(isoquinolin-1-yl)pyrimidine-4-carboxamide |
| 32 | 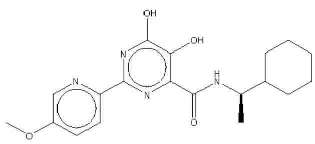 | (*R*)-*N*-(1-cyclohexylethyl)-5,6-dihydroxy-2-(5-methoxypyridin-2-yl)pyrimidine-4-carboxamide |
| 33 | 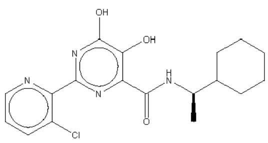 | (*R*)-2-(3-chloropyridin-2-yl)-*N*-(1-cyclohexylethyl)-5,6-dihydroxypyrimidine-4-carboxamide |

(continued)

| Compound No | Structure | IUPAC Name |
|---|---|---|
| 34 | 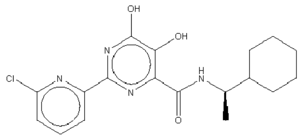 | (*R*)-2-(6-chloropyridin-2-yl)-*N*-(1-cyclohexylethyl)-5,6-dihydroxypyrimidine-4-carboxamide |
| 35 | 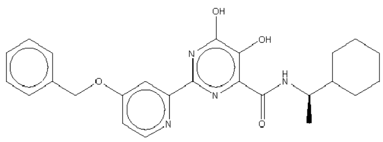 | (*R*)-2-(4-(benzyloxy)pyridin-2-yl)-*N*-(1-cyclohexylethyl)-5,6-dihydroxypyrimidine-4-carboxamide |
| 36 | 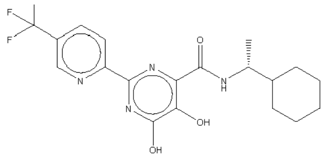 | (R)-*N*-(1-cyclohexylethyl)-5,6-dihydroxy-2-(5-(trifluoromethyl)pyridin-2-yl)pyrimidine-4-carboxamide |
| 37 | 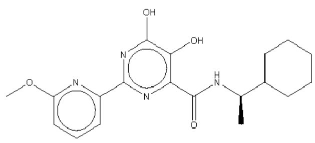 | (*R*)-*N*-(1-cyclohexylethyl)-5,6-dihydroxy-2-(6-methoxypyridin-2-yl)pyrimidine-4-carboxamide |
| 38 | 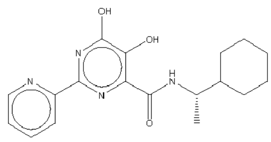 | (*S*)-*N*-(1-cyclohexylethyl)-5,6-dihydroxy- 2-(pyridin-2-yl)pyrimidine-4-carboxamide |
| 39 | 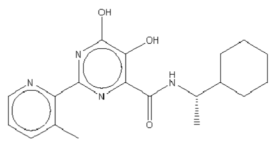 | (*S*)-*N*-(1-cyclohexylethyl)-5,6-dihydroxy-2-(3-methylpyridin-2-yl)pyrimidine-4-carboxamide |
| 40 | 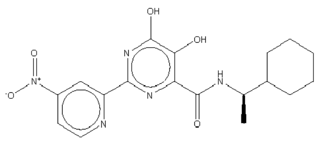 | (*R*)-N-(1-cyclohexylethyl)-5,6-dihydroxy-2-(4-nitropyridin-2-yl)pyrimidine-4-carboxamide |
| 41 | 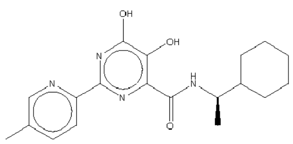 | (*R*)-*N*-(1-cyclohexylethyl)-5,6-dihydroxy-2-(5-methylpyridin-2-yl)pyrimidine-4-carboxamide |
| 42 | 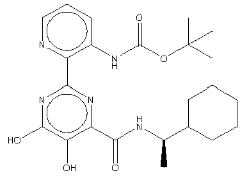 | *tert*-butyl (*R*)-(2-(4-((1-cyclohexylethyl)carbamoyl)-5,6-dihydroxypyrimidin-2-yl)pyridin-3-yl)carbamate |

(continued)

| Compound No | Structure | IUPAC Name |
|---|---|---|
| 43 | 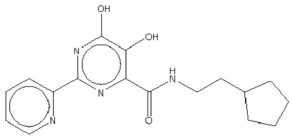 | *N*-(2-cyclopentylethyl)-5,6-dihydroxy-2-(pyridin-2-yl) pyrimidine-4-carboxamide |
| 44 | 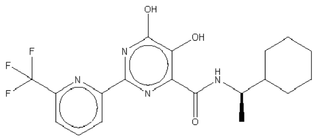 | (*R*)-*N*-(1-cyclohexylethyl)- 5,6-dihydroxy-2-(6-(trifluoromethyl)pyridin-2-yl)pyrimidine-4-carboxamide |
| 45 | 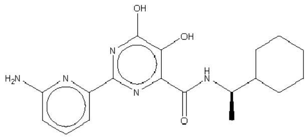 | (*R*)-2-(6-aminopyridin-2-yl)-*N*-(1-cyclohexylethyl)-5,6-dihydroxypyrimidine-4-carboxamide |
| 46 | 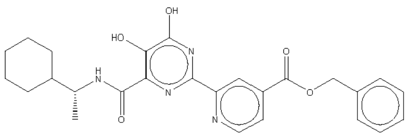 | benzyl (*R*)-2-(4-((1-cyclohexylethyl)carbamoyl)-5,6-dihydroxypyrimidin-2-yl)isonicotinate |
| 47 | 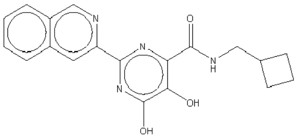 | *N*-(cyclobutylmethyl)-5,6-dihydroxy-2-(isoquinolin-3-yl)pyrimidine-4-carboxamide |
| 48 | 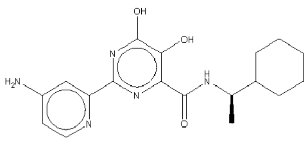 | (*R*)-2-(4-aminopyridin-2-yl)-*N*-(1-cyclohexylethyl)-5,6-dihydroxypyrimidine-4-carboxamide |
| 49 | 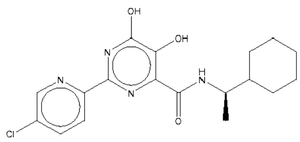 | (*R*)-2-(5-chloropyridin-2-yl)-*N*-(1-cyclohexylethyl)-5,6-dihydroxypyrimidine-4-carboxamide |
| 50 | 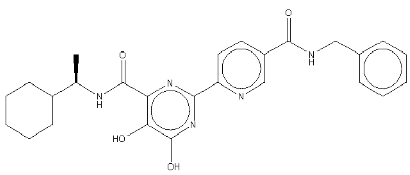 | (*R*)-2-(5-(benzylcarbamoyl)pyridin-2-yl)-*N*(1-cyclohexylethyl)-5,6-dihydroxypyrimidine-4-carboxamide |
| 51 | 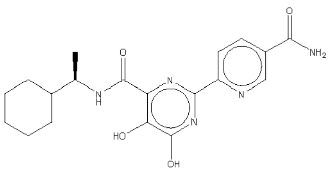 | (*R*)-2-(5-carbamoylpyridin-2-yl)-*N*-(1-cyclohexylethyl)-5,6-dihydroxypyrimidine-4-carboxamide |

(continued)

| Compound No | Structure | IUPAC Name |
|---|---|---|
| 52 | 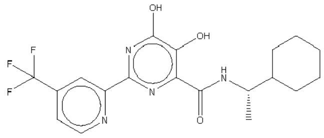 | (*S*)-*N*-(1-cyclohexylethyl)-5,6-dihydroxy-2-(4-(trifluoromethyl)pyridin-2-yl)pyrimidine-4-carboxamide |
| 53 | 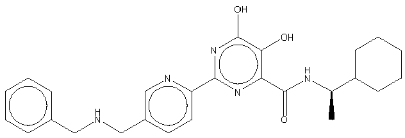 | (R)-2-(5-((benzylamino)methyl)pyridin-2-yl)-N-(1-cyclohexylethyl)-5,6-dihydroxypyrimidine-4-carboxamide |
| 54 | 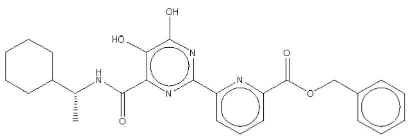 | benzyl (R)-6-(4-((1-cyclohexylethyl)carbamoyl)-5,6-dihydroxypyrimidin-2-yl)picolinate |
| 55 | 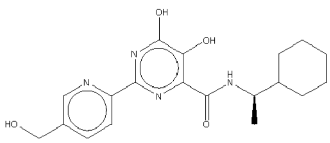 | (*R*)-*N*-(1-cyclohexylethyl)-5,6-dihydroxy-2-(5-(hydroxymethyl)pyridin-2-yl)pyrimidine-4-carboxamide |
| 56 | 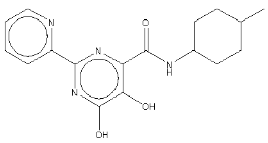 | 5,6-dihydroxy-*N*-(4-methylcyclohexyl)-2-(pyridin-2-yl) pyrimidine-4-carboxamide |
| 57 | 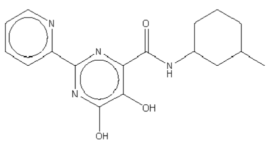 | 5,6-dihydroxy-*N*-(3-methylcyclohexyl)-2-(pyridin-2-yl) pyrimidine-4-carboxamide |
| 58 | 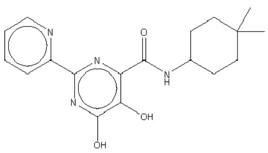 | *N*-(4,4-dimethylcyclohexyl)-5,6-dihydroxy-2-(pyridin-2-yl)pyrimidine-4-carboxamide |
| 59 | 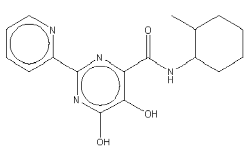 | 5,6-dihydroxy-*N*-(2-methylcyclohexyl)-2-(pyridin-2-yl) pyrimidine-4-carboxamide |
| 60 | 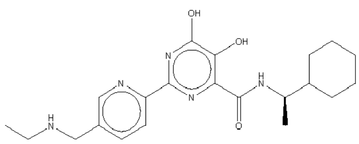 | (*R*)-*N*-(1-cyclohexylethyl)-2-(5-((ethylamino)methyl) pyridin-2-yl)-5,6-dihydroxypyrimidine-4-carboxamide |

(continued)

| Compound No | Structure | IUPAC Name |
|---|---|---|
| 61 | 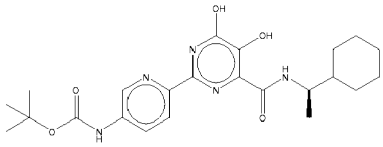 | *tert*-butyl (*R*)-(6-(4-((1-cyclohexylethyl)carbamoyl)-5,6-dihydroxypyrimidin-2-yl)pyridin-3-yl)carbamate |
| 62 | 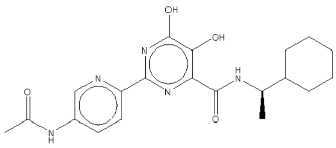 | (*R*)-2-(5-acetamidopyridin-2-yl)-*N*-(1-cyclohexylethyl)-5,6-dihydroxypyrimidine-4-carboxamide |
| 63 | 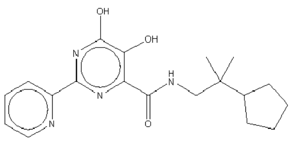 | *N*-(2-cyclopentyl-2-methylpropyl)-5,6-dihydroxy-2-(pyridin-2-yl)pyrimidine-4-carboxamide |
| 64 | 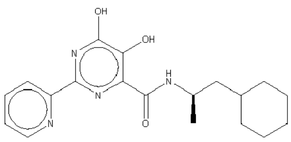 | (*R*)-*N*-(1-cyclohexylpropan-2-yl)-5,6-dihydroxy-2-(pyridin-2-yl)pyrimidine-4-carboxamide |
| 65 | 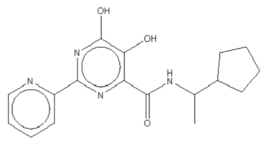 | *N*-(1-cyclopentylethyl)-5,6-dihydroxy-2-(pyridin-2-yl) pyrimidine-4-carboxamide |
| 66 | 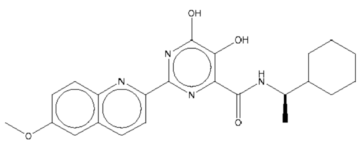 | (*R*)-*N*-(1-cyclohexylethyl)-5,6-dihydroxy-2-(6-methoxyquinolin-2-yl)pyrimidine-4-carboxamide |
| 67 | 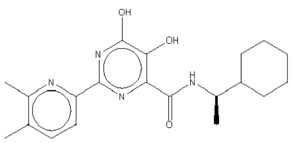 | (*R*)-*N*-(1-cyclohexylethyl)-2-(,5,6-dimethylpyridin-2-yl)-5,6-dihydroxypyrimidine-4-carboxamide |
| 68 | 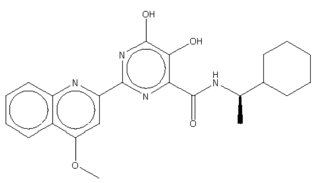 | *N*-[(1*R*)-1-cyclohexylethyl]-2-(4-methoxyquinolin-2-yl)-5,6-bis(oxidanyl)pyrimidine-4-carboxamide |
| 69 | 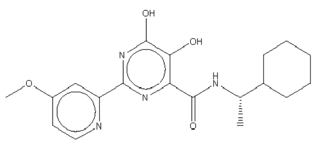 | *N*-[(1*S*)-1-cyclohexylethyl]-2-(4-methoxypyridin-2-yl)-5,6-bis(oxidanyl)pyrimidine-4-carboxamide |

(continued)

| Compound No | Structure | IUPAC Name |
|---|---|---|
| 70 | 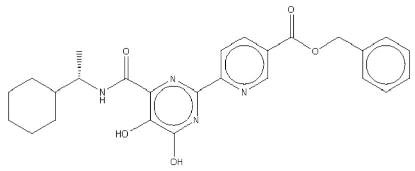 | benzyl (*S*)-6-(4-((1-cyclohexylethyl)carbamoyl)-5,6-dihydroxypyrimidin-2-yl)nicotinate |
| 71 | 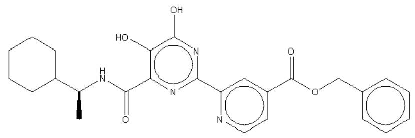 | benzyl (*S*)-2-(4-((1-cyclohexylethyl)carbamoyl)-5,6-dihydroxypyrimidin-2-yl)isonicotinate |
| 72 | 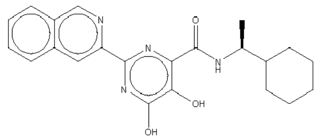 | (*S*)-*N*-(1-cyclohexylethyl)-5,6-dihydroxy-2-(isoquinolin-3-yl)pyrimidine-4-carboxamide |
| 73 | 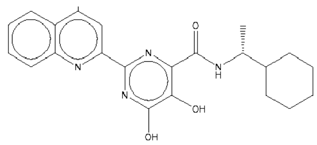 | (*R*)-*N*-(1-cyclohexylethyl)-5,6-dihydroxy-2-(4-methylquinolin-2-yl)pyrimidine-4-carboxamide |
| 74 | 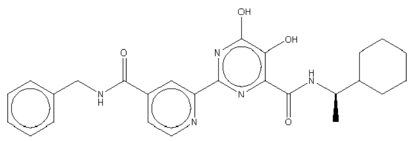 | (*R*)-2-(4-(benzylcarbamoyl)pyridin-2-yl)-*N*-(1-cyclohexylethyl)-5,6-dihydroxypyrimidine-4-carboxamide |
| 75 | 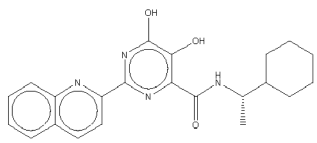 | (*S*)-*N*-(1-cyclohexylethyl)-5,6-dihydroxy-2-(quinolin-2-yl)pyrimidine-4-carboxamide |
| 76 | 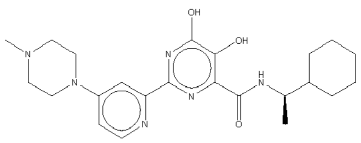 | (*R*)-*N*-(1-cyclohexylethyl)-5,6-dihydroxy-2-(4-(4-methylpiperazin-1-yl)pyridin-2-yl)pyrimidine-4-carboxamide |
| 77 | 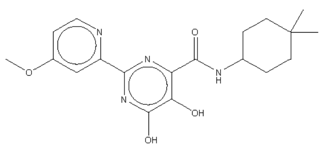 | *N*-(4,4-dimethylcyclohexyl)-5,6-dihydroxy-2-(4-methoxypyridin-2-yl)pyrimidine-4-carboxamide |
| 78 | 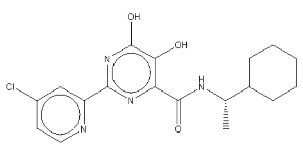 | (*S*)-2-(4-chloropyridin-2-yl)-*N*-(1-cyclohexylethyl)-5,6-dihydroxypyrimidine-4-carboxamide |

(continued)

| Compound No | Structure | IUPAC Name |
|---|---|---|
| 79 | 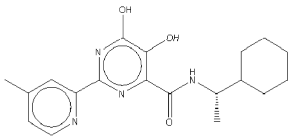 | (*S*)-*N*-(1-cyclohexylethyl)-5,6-dihydroxy-2-(4-methylpyridin-2-yl)pyrimidine-4-carboxamide |
| 80 | 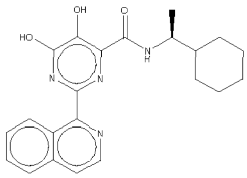 | (*S*)-*N*-(1-cyclohexylethyl)-5,6-dihydroxy-2-(isoquinolin-1-yl)pyrimidine-4-carboxamide |
| 81 | 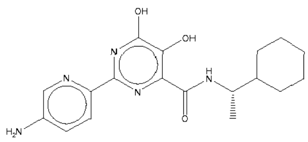 | (*S*)-2-(5-aminopyridin-2-yl)-*N*-(1-cyclohexylethyl)-5,6-dihydroxypyrimidine-4-carboxamide |
| 82 | 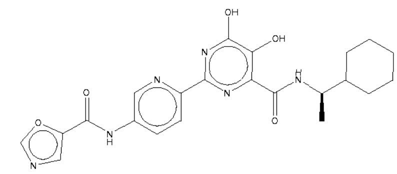 | (*R*)-*N*-(6-(4-((1-cyclohexylethyl)carbamoyl)-5,6-dihydroxypyrimidin-2-yl)pyridin-3-yl)oxazole-5-carboxamide |
| 83 | 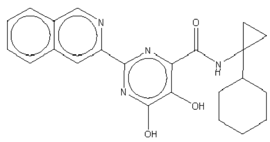 | *N*-(1-cyclohexylcyclopropyl)-5,6-dihydroxy-2-(isoquinolin-3-yl)pyrimidine-4-carboxamide |
| 84 | 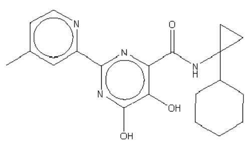 | *N*-(1-cyclohexylcyclopropyl)-5,6-dihydroxy-2-(4-methylpyridin-2-yl)pyrimidine-4-carboxamide |
| 85 | 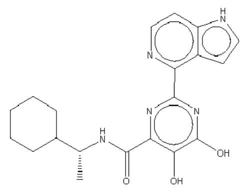 | (*R*)-*N*-(1-cyclohexylethyl)-5,6-dihydroxy-2-(1*H* pyrrolo[3,2-c]pyridin-4-yl)pyrimidine-4-carboxamide |
| 86 | 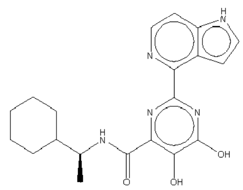 | (*S*)-*N*-(1-cyclohexylethyl)-5,6-dihydroxy-2-(1*H*-pyrrolo[3,2-c]pyridin-4-yl)pyrimidine-4-carboxamide |
| 87 | 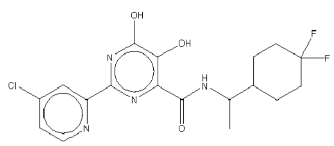 | 2-(4-chloropyridin-2-yl)-*N*-(1-(4,4-difluorocyclohexyl) ethyl)-5,6-dihydroxypyrimidine-4-carboxamide |

(continued)

| Compound No | Structure | IUPAC Name |
|---|---|---|
| 88 | 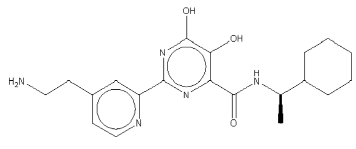 | (*R*)-2-(4-(2-aminoethyl)pyridin-2-yl)-*N*-(1-cyclohexylethyl)-5,6-dihydroxypyrimidine-4-carboxamide |
| 89 | 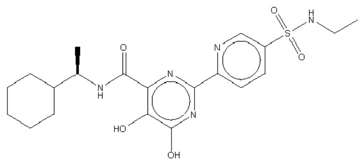 | (*R*)-N-(1-cyclohexylethyl)-2-(5-(*N*-ethylsulfamoyl) pyridin-2-yl)-5,6-dihydroxypyrimidine-4-carboxamide |
| 90 | 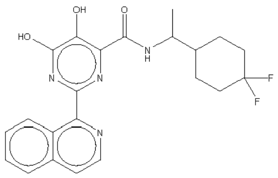 | *N*-(1-(4,4-difluorocyclohexyl)ethyl)-5,6-dihydroxy-2-(isoquinolin-1-yl)pyrimidine-4-carboxamide |
| 91 | 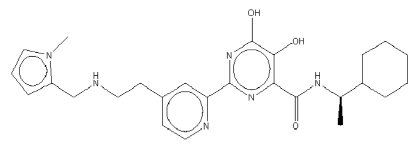 | (*R*)-*N*-(1-cyclohexylethyl)-5,6-dihydroxy-2-(4-(2-(((1-methyl-1*H*-pyrrol-2-yl)methyl)amino)ethyl)pyridin-2-yl)pyrimidine-*4-carboxamide* |
| 92 | 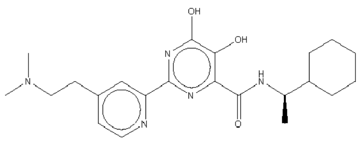 | (*R*)-*N*-(1-cyclohexylethyl)-2-(4-(2-(dimethylamino) ethyl)pyridin-2-yl)-5,6-dihydroxypyrimidine-4-carboxamide |
| 93 | 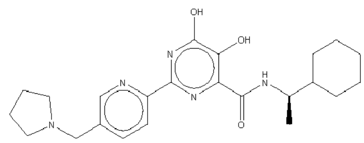 | (*R*)-*N*-(1-cyclohexylethyl)-5,6-dihydroxy-2-(5-(pyrrolidin-1-ylmethyl)pyridin-2-yl)pyrimidine-*4-carboxamide* |
| 94 | 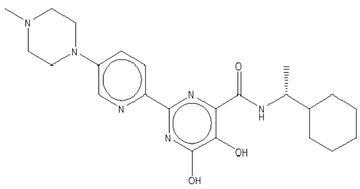 | (*R*)-*N*-(1-cyclohexylethyl)-5,6-dihydroxy-2-(5-(4-methylpiperazin-1-yl)pyridin-2-yl)pyrimidine-*4-carboxamide* |
| 95 | 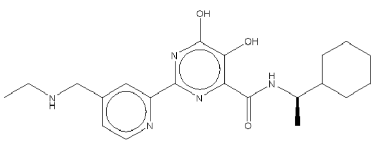 | (*R*)-*N*-(1-cyclohexylethyl)-2-(4-((ethylamino)methyl) pyridin-2-yl)-5,6-dihydroxypyrimidine-4-carboxamide |
| 96 | 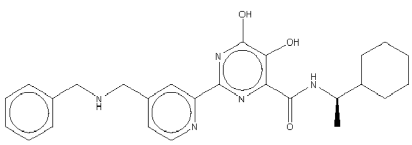 | (*R*)-2-(4-((benzylamino)methyl)pyridin-2-yl)-*N* (1-cyclohexylethyl)-5,6-dihydroxypyrimidine-4-carboxamide |

b) EXAMPLES

General methods

Unless otherwise stated, all reagents were obtained from commercial sources and were used as received without further purification. $^1$H NMR spectra were recorded on Bruker spectrometers operating at (reported) frequencies between 400 and 600 MHz. Chemical shifts (δ) for signals corresponding to non-exchangeable protons (and exchangeable protons where visible) were recorded in parts per million (ppm) relative to tetramethylsilane and were measured using the residual solvent peak as reference. Signals are tabulated in the order: multiplicity (s, singlet; d, doublet; t, triplet; q, quartet; m, multiplet; b, broad, and combinations thereof); number of protons; costant(s) in hertz. UPLC-MS analysis was conducted on a Waters UPLC system with both Diode Array detection and Electrospray (+'ve and -'ve ion) MS detection. The stationary phase was a Waters Acquity UPLC BEH C18 1.7um (2.1x50mm) column. The mobile phase comprised $H_2O$ containing 0.1% formic acid (A) and MeCN containing 0.1% formic acid (B) in the following linear gradients:

**Method A:** 90% A (0.1 min), 90%-0% A (2.5 min), 0% A (0.3 min), 0-90% A (0.1 min) with a flow rate 0.5 mL/min.
**Method B:** 95% A (0.5 min), 95%-50% A (1.9 min), 50-0% A (0.3 min), 0% (0.2 min), 0-100% A (0.1 min) with a flow rate 0.5 mL/min.

Reverse phase (C18) column chromatography was carried out using as mobile phase $H_2O$ containing 0.1% of trifluoroacetic acid and MeCN containing 0.1 % trifluoroacetic acid.

The following abbreviations are used in the Schemes and Examples:

AcOH: acetic acid; bs: broad singlet; Cy: cyclohexyl; DCM: dichloromethane; DIPEA: diisopropylethylamine; DMAD: dimethyl but-2-ynedioate; DMF: dimethylformamide; DMSO: dimethylsulfoxide; eq: equivalent(s); $Et_2O$: diethyl ether; EtOAc: ethyl actetate; EtOH: ethanol; h: hour(s); HATU: 1-[Bis(dimcthylamino)methylene]-1*H*-1,2,3-triazolo[4,5-b]pyridinium 3-oxid hexafluorophosphate; M: molar; *m*-CPBA: *meta*-chloroperoxybenzoic acid; MeCN: acetonitrile; MeOH: methanol; min: minutes; RP-HPLC: reversed phase high-performance liquid chromatography; RT: room temperature; TEA: triethylamine; THF: tetrahydrofuran; UPLC: ultra performance liquid chromatography.

The following Examples illustrate the present invention.

Example 1

**(*R*)-*N*-(1-cyclohexylethyl)-5,6-dihydroxy-2-(pyridin-2-yl)pyrimidine-4-carboxamide (Compound 5)**

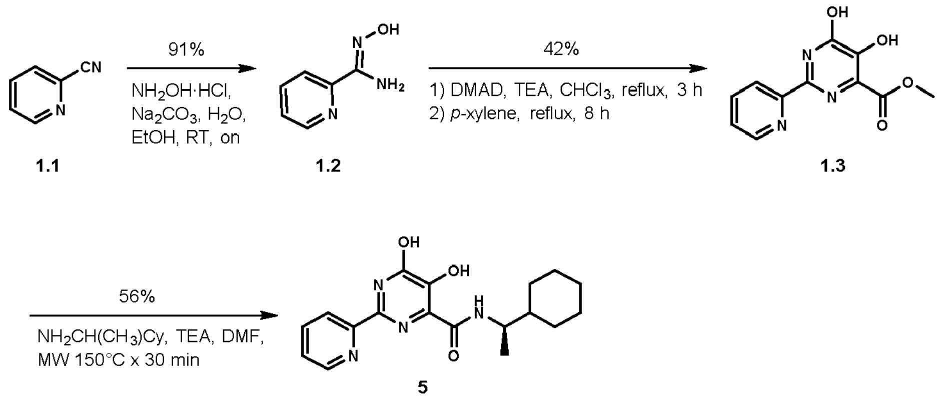


1.1 1.2 1.3 5

*Step 1: N-hydroxypicolinimidamide (compound 1.2)*

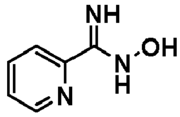

Picolinonitrile (1.0 eq) was suspended in $H_2O$ (0.24 M) and EtOH (1.8 M) then disodium carbonate (1.7 eq) followed by hydroxylamine hydrochloride (3.3 eq) were added. The mixture was stirred at room temperature overnight. The resulting precipitate was filtered, washed with $H_2O$ and dried to afford the title compound as a white powder which was used as such without further purifications (91%). $^1H$-NMR (400 MHz, DMSO-$d_6$, 300 K) δ 9.90 (s, 1H); 8.57-8.55 (m, 1H); 7.87-7.78 (m, 2H); 7.40 (ddd, 1H, J = 7.2 Hz, J = 4.9 Hz, J = 1.4 Hz); 5.83 (s, 2H); UPLC method B *t*R 0.39 min; MS ($ES^+$) *m*/*z* 138 $[M+H]^+$.

*Step 2: methyl 5,6-dihydroxy-(pyridin-2-yl)pyrimidine-4-carboxylate* (*compound 1.3)*

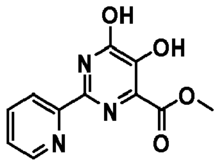

N-hydroxypicolinimidamide (1.0 eq) was suspended in $CH_3Cl$ (0.2 M), TEA (0.05 eq) and DMAD (1.05 eq) were added at room temperature. The mixture was refluxed for 3 h. After cooling, volatiles were removed *in vacuo* to afford dimethyl 2-((picolinimidamido)oxy)but-2-enedioate intermediate as a red oil which was used without further purification. UPLC method A *t*R 1.55 and 1.65 min; MS ($ES^+$) *m*/*z* 280 $[M+H]^+$. The solution of dimethyl 2-((picolinimidamido)oxy)but-2-enedioate in *p*-xylene (0.29 M) was refluxed for 8 h. The mixture was cooled to 4 °C, and the resulting precipitate was isolated by filtration and washed with petroleum ether to afford the title compound as a beige powder (42%). $^1H$-NMR (400 MHz, DMSO-$d_6$, 300 K) δ 12.51 (bs, 1H); 10.82 (bs, 1H), 8.68 (ddd, 1H, J = 4.8 Hz, J = 1.5 Hz, J = 0.9 Hz); 8.21-8.19 (m, 1H); 8.00 (ddd, 1H, J = 7.8 Hz, J = 7.8 Hz, J = 1.7 Hz); 7.57 (ddd, 1H, J = 7.5 Hz, J = 4.8 Hz, J = 1.2 Hz); 3.86 (s, 3H); UPLC method A *t*R 0.93 min; MS ($ES^+$) m/z 248 $[M+H]^+$.

*Step 3: (R)-N-(1-cyclohexylethyl)-5,6-dihydroxy-2-(pyridin-2-yl)pyrimidine-4-carboxamide (Compound 5)*

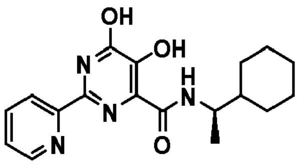

Methyl 5,6-dihydroxy-2-(pyridin-2-yl)pyrimidine-4-carboxylate (1.0 eq) was dissolved in DMF (0.5 M) then (1*R*)-1-cyclohexylethanamine (1.0 eq) followed by TEA (2.0 eq) were added and the mixture was stirred under microwave irradiation at 150 °C for 30 min. The volatiles were evaporated in vacuo and the crude was purified by flash chromatography on C18 column eluting with $H_2O$ / MeCN containing 0.1 % trifluoroacetic acid to afford the title compound as beige powder, as its trifluoroacetate salt (56%). $^1H$-NMR (600 MHz, DMSO-$d_6$, 300 K) δ 13.10 (bs, 1H); 12.28 (bs, 1H); 8.73-8.66 (m, 3H); 8.06 (ddd, 1H, J = 7.7 Hz, J = 7.7 Hz, J = 1.7 Hz); 7.60 (ddd, 1H, J = 7.5 Hz, J = 4.7 Hz, J = 1.1 Hz); 3.90-3.81 (m, 1H); 1.81-1.67 (m, 4H); 1.62-1.52 (m, 2 H); 1.27-1.09 (m, 6H); 1.02-0.90 (m, 2H); UPLC method A *t*R 2.05 min; MS ($ES^+$) m/z 343 $[M+H]^+$.

Example 2

***N*-(3-cyclohexylpropyl)-5,6-dihydroxy-2-(pyridin-2-yl)pyrimidine-4-carboxamide (Compound 8)**

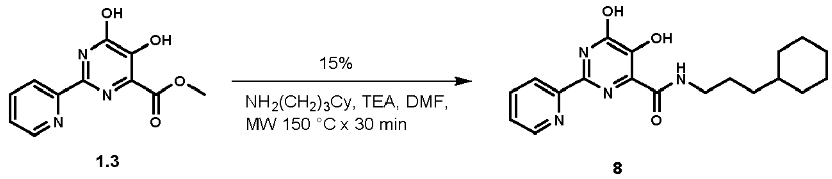


1.3

8

Methyl 5,6-dihydroxy-2-(pyridin-2-yl)pyrimidine-4-carboxylate (1.0 eq) was dissolved in DMF (0.2 M), 3-cyclohexylpropan-1-amine (1.0 eq) and TEA (2.0 eq) were added and the mixture was stirred under microwave irradiation at 150 °C for 30 min. Volatiles were evaporated *in vacuo* and the residue was purified by RP-HPLC C18 eluting with $H_2O$ / MeCN containing 0.1 % trifluoroacetic acid to afford the title compound, off-white solid, as its trifluoroacetate salt (15%). $^1$H-NMR (400 MHz, DMSO-$d_6$, 300 K) δ 13.01 (bs, 1H); 12.28 (bs, 1H); 9.19 (t, 1H, J = 6.3 Hz); 8.71-8.68 (m, 2H); 8.06-8.02 (m, 1H); 7.61-7.57 (m, 1H); 3.33-3.28 (m, 2H); 1.71-1.55 (m, 7H); 1.27-1.06 (m, 6H); 0.91-0.82 (m, 2H); UPLC method A: *t*R 2.24 min; MS (ES$^+$) *m/z* 357 [M+H]$^+$.

Example 3

**Benzyl (*R*)-2-(4-((1-cyclohexylethyl)carbamoyl)-5,6-dihydroxypyrimidin-2-yl)isonicotinate (Compound 46)**

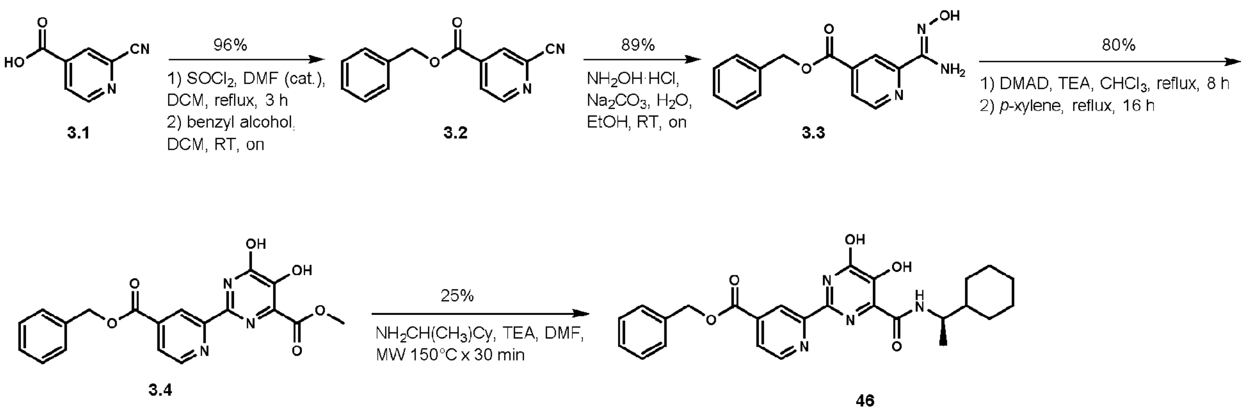


3.1 3.2 3.3 3.4 46

*Step 1: benzyl 2-cyanoisonicotinate (compound 3.2)*

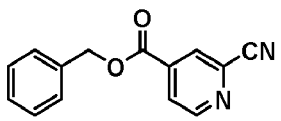

To a suspension of 2-cyanoisonicotinic acid (1.0 eq) in DCM (1.5 M), thionyl chloride (6.0 eq) and DMF (0.05 eq) were added. The reaction mixture was refluxed for 3 h, then allowed to cool at room temperature and concentrated to afford 2-cyanoisonicotinoyl chloride as brown oil used in the next step without further purification, UPLC method A (quencing with MeOH) *t*R 1.11 min; MS (ES$^+$) m/z 163 [M+H]$^+$. To the solution of 2-cyanoisonicotinoyl chloride in DCM (1.5 M), benzyl alcohol (1.0 eq.) and TEA (2.0 eq) were added. The reaction mixture was stirred at room temperature overnight. Volatiles were removed in vacuo and residue was taken up in EtOAc, washed with $H_2O$, dried over $Na_2SO_4$, filtered and evaporated to afford the title compound as a brown solid used for the next step without further purification (96%). UPLC method A *t*R 1.82 min; MS (ES$^+$) m/z 239 [M+H]$^+$.

*Step 2: benzyl 2-(N-hydroxycarbamimidoyl)isonicotinate (compound 3.3)*

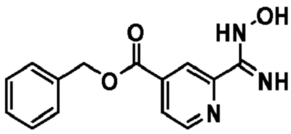

Benzyl 2-cyanoisonicotinate (1.0 eq) was suspended in $H_2O$ (0.23 M) and EtOH (1.7 M) then disodium carbonate (1.7 eq) followed by hydroxylamine hydrochloride (3.3 eq) were added. The mixture was stirred at room temperature overnight. The resulting precipitate was filtered, washed with $H_2O$ and dried to afford the title compound as ochre solid which was used as such without further purifications (89%). UPLC method A *t*R 1.45 min; MS ($ES^+$) m/z 272 $[M+H]^+$.

*Step 3: methyl 2-(4-((benzyloxy)carbonyl)pyridin-2-yl)-5,6-dihydroxypyrimidine-4-carboxylate (compound 3.4)*

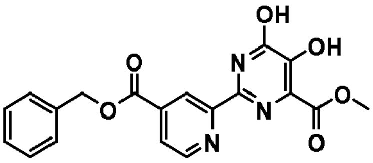

Benzyl 2-(*N*-hydroxycarbamimidoyl)isonicotinate (1.0 eq) was suspended in $CHCl_3$ (0.24 M) and TEA (0.1 eq) was added at room temperature followed by DMAD (1.0 eq). The mixture was refluxed for 8 h. After cooling, volatiles were removed *in vacuo* to afford dimethyl 2-((picolinimidamido)oxy)but-2-enedioate intermediate as a brown oil which was used without further purification. UPLC method A *t*R 1.96 and 2.01 min; MS ($ES^+$) *m/z* 414 $[M+H]^+$. The solution of dimethyl 2-((picolinimidamido)oxy)but-2-enedioate in *p*-xylene (0.23 M) was refluxed for 16 h. The mixture was cooled to 4 °C to allow the formation of a precipitate. The solid was collected by filtration and washed with petroleum ether to afford the title compound as a yellow powder (80%). $^1$H-NMR (400 MHz, DMSO-$d_6$, 300 K) $\delta$ 12.85 (bs, 1H), 10.86 (bs, 1H), 8.89 (d, 1H, J = 5.0 Hz), 8.60-8.60 (m, 1H), 8.01 (dd, 1H, J = 5.0 Hz, J = 1.5 Hz), 7.53-7.49 (m, 2H), 7.45-7.36 (m, 3H), 5.44 (s, 2H), 3.86 (s, 3H); UPLC method A *t*R 1.67 min; MS ($ES^+$) m/z 382 $[M+H]^+$.

*Step 4: benzyl (R)-2-(4-((1-cyclohexylethyl)carbamoyl)-5,6-dihydroxypyrimidin-2-yl)isonicotinate (Compound 46)*

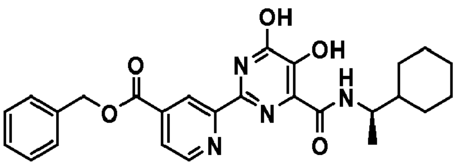

Methyl 2-(4-((benzyloxy)carbonyl)pyridin-2-yl)-5,6-dihydroxypyrimidine-4-carboxylate (1.0 eq) was dissolved in DMF (0.2 M), (1*R*)-1-cyclohexylethanamine (1.0 eq) and TEA (2.0 eq) were added and the mixture was stirred under microwave irradiation at 150 °C for 30 min. Volatiles were evaporated *in vacuo* and the crude was purified by flash chromatography column C18 eluting with $H_2O$ / MeCN containing 0.1 % trifluoroacetic acid to afford the title compound, brown powder, as its trifluoroacetate salt (25%). $^1$H-NMR (400 MHz, DMSO-$d_6$, 300 K) $\delta$ 13.04 (bs, 1H), 12.54 (bs, 1H), 8.90-8.87 (m, 2H), 8.60 (d, 1H, J = 9.2 Hz), 8.03 (dd, 1H, J = 5.0 Hz, J = 1.6 Hz), 7.55-7.52 (m, 2H), 7.44-7.36 (m, 3H), 5.45 (s, 2H), 3.90-3.80 (m, 1H), 1.79-1.49 (m, 6H), 1.23-0.90 (m, 8H). UPLC method A *t*R 2.43 min; MS ($ES^+$) m/z 477 $[M+H]^+$.

Example 4

**(*R*)-2-(4-(benzylcarbamoyl)pyridin-2-yl)-*N*-(1-cyclohexylethyl)-5,6-dihydroxypyrimidine-4-carboxamide (Compound 74)**

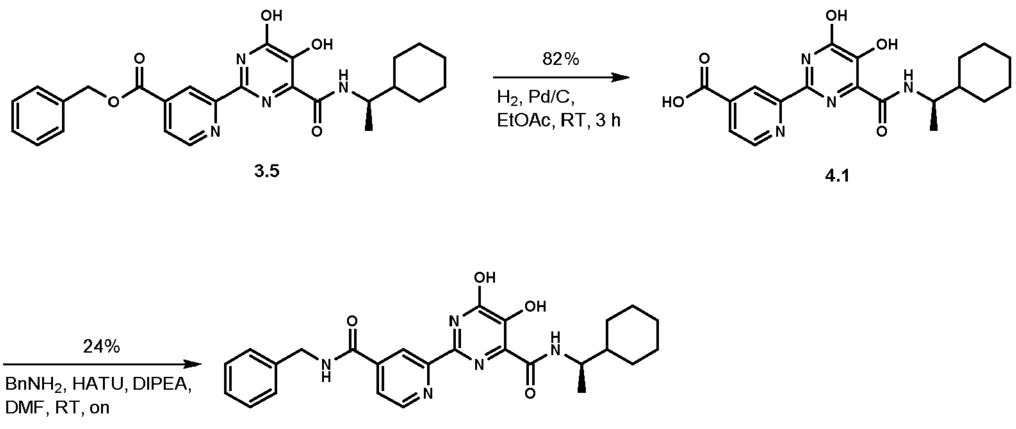


3.5 4.1

74

*Step 1: (R)-2-(4-((1-cyclohexylethyl)carbamoyl)-5,6-dihydroxypyrimidin-2-yl)isonicotinic acid (compound 4.1)*

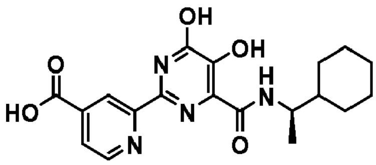

A suspension of Benzyl (R)-2-(4-((1-cyclohexylethyl)carbamoyl)-5,6-dihydroxypyrimidin-2-yl)isonicotinate (1.0 eq) in EtOAc (0.1 M) was treated with 10% Pd/C, and the mixture was stirred for 3 h under $H_2$ atmosphere. Catalyst was filtered off, and filtrate was evaporated to afford the title compound as a solid, which was used as such without further purifications (82%). UPLC method A *t*R 1.68 min; MS (ES$^+$) m/z 387 [M+H]$^+$.

*Step 2: (R)-2-(4-(benzylcarbamoyl)pyridin-2-yl)-N-(1-cyclohexylethyl)-5,6-dihydroxypyrimidine-4-carboxamide (Compound 74)*

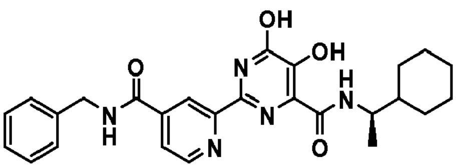

(R)-2-(4-((1-cyclohexylethyl)carbamoyl)-5,6-dihydroxypyrimidin-2-yl)isonicotinic acid (1.0 eq) was solved in DMF (0.26 M) then benzylamine (1.0 eq), HATU (1.1 eq) and DIPEA (3.0 eq) were added. The reaction mixture was stirred at room temperature overnight. Volatiles were removed *in vacuo* and residue was taken up in EtOAc, washed with 1N HCl, saturated solution $NaHCO_3$, brine, dried over $Na_2SO_4$, filtered and evaporated. The crude was purified by RP-HPLC C18 eluting with $H_2O$ / MeCN containing 0.1 % trifluoroacetic acid to afford the title compound, yellow powder, as its trifluoroacetate salt (24%). $^1$H NMR (400 MHz, DMSO-d$_6$, 300 K) $\delta$ 13.06 (s, 1H), 12.47 (s, 1H), 9.47 (t, 1H, J = 5.9 Hz), 8.84-8.82 (m, 2H), 8.62 (d, 1H, J = 9.1 Hz), 7.91 (dd, 1H, J = 4.8 Hz, J = 1.8 Hz), 7.39-7.32 (m, 4H), 7.29-7.24 (m, 1H), 4.54 (d, 2H, J = 5.8 Hz), 3.90-3.81 (m, 1H), 1.81-1.51 (m, 6H), 1.23-1.10 (m, 6H), 1.03-0.91 (m, 2H),. UPLC method A *t*R 2.05 min; MS (ES$^+$) m/z 476 [M+H]$^+$.

Example 5

**(*R*)-2-(4-(2-aminoethyl)pyridin-2-yl)-*N*-(1-cyclohexylethyl)-5,6-dihydroxypyrimidine-4-carboxamide (Compound 88)**

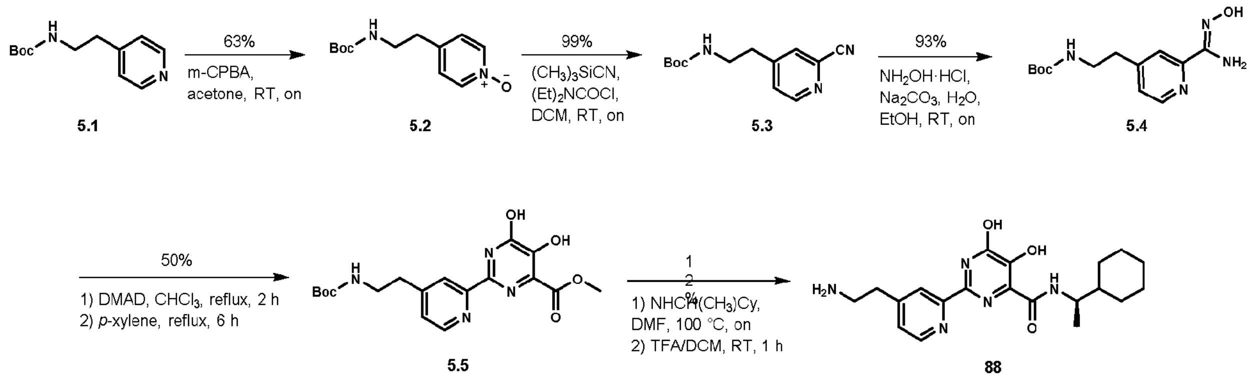


*Step 1: 4-(2-((tert-butoxycarbonyl)amino)ethyl)pyridine 1-oxide (compound 5.2)*

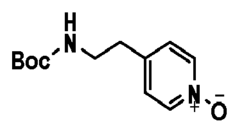

To a solution of *tert*-butyl(2-(pyridin-4-yl)ethyl)carbamate (1.0 eq) in acetone (0.22 M) was added *m*-chloroperbenzoic acid (3.0 eq) in acetone (0.7 M) via an addition funnel over 30 min. The reaction mixture was stirred at room temperature overnight. Volatiles were removed *in vacuo* and residue was taken up in $Et_2O$ and extracted with $H_2O$. The combined aqueous phase was saturated with solid NaCl and extracted with DCM. The organic phase was washed with brine, dried over $Na_2SO_4$, filtered and evaporated to afford the title compound as a yellow oil (63%). $^1$H-NMR (400 MHz, DMSO-$d_6$, 300 K) δ 8.11 (d, 2H, J = 6.7 Hz); 7.21 (d, 2H, J = 6.9 Hz); 6.88 (t, 1H, J = 5.8 Hz); 3.16 (q, 2H, J = 6.6 Hz); 2.67 (t, 2H, J = 6.9 Hz); 1.34 (s, 9H); UPLC method A *t*R 0.88 min; MS (ES$^+$) *m*/*z* 239 [M+H]$^+$.

*Step 2: tert-butyl (2-(2-cyanopyridin-4-yl)ethyl)carbamate (compound 5.3)*

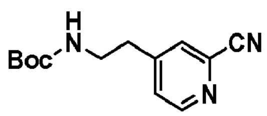

To a solution of 4-(2-((*tert*-butoxycarbonyl)amino)ethyl)pyridine 1-oxide (1.0 eq) in DCM (0.9 M), trimethylsilyl cyanide (2.0 eq) and *N,N*-diethylcarbamoyl chloride (2.0 eq) were added. After stirring overnight, the reaction mixture was evaporated under vacuum, diluted with saturated solution $NaHCO_3$ and extracted with EtOAc. The organic phase was dried over $Na_2SO_4$, filtered and concentrated to afford the title compound as an amber oil (99%). UPLC method A *t*R 1.41 min; MS (ES$^+$) *m*/*z* 248 [M+H]$^+$.

*Step 3: tert-butyl (2-(2-(N'-hydroxycarbamidoyl)pyridin-4-yl)ethyl)carbamate (compound 5.4)*

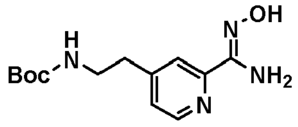

*Tert*-butyl (2-(2-cyanopyridin-4-yl)ethyl)carbamate (1.0 eq) was suspended in $H_2O$ (0.36 M) and EtOH (3.1 M) then disodium carbonate (1.0 eq) and hydroxylamine hydrochloride (1.0 eq) were added. The mixture was stirred at room temperature overnight. The crude was evaporated under vacuum to remove EtOH and extracted with EtOAc. The organic phase was dried over $Na_2SO_4$, filtered and concentrated *in vacuo* to afford the title compound as a yellow oil which was used as such without further purifications (93%). $^1$H NMR (400 MHz, DMSO-$d_6$, 300 K) δ 9.82 (s, 1H), 8.43 (d, 1H, J = 5.1 Hz), 7.68 (s, 1H), 7.23 (d, 1H, J = 5.0 Hz), 6.89 (t, 1H, J = 5.7 Hz), 5.79 (s, 2H), 3.18 (q, 2H, J = 6.4 Hz), 2.73 (t, 2H, J = 6.9 Hz), 1.34 (s, 9H). UPLC method A *t*R 0.96 min; MS (ES$^+$) *m/z* 281 [M+H]$^+$.

*Step 4: methyl 2-(4-(2-((tert-butoxycarbonyl)amino)ethyl)pyridin-2-yl)-5,6-dihydroxypyrimidine-4-carboxylate (compound 5.5)*

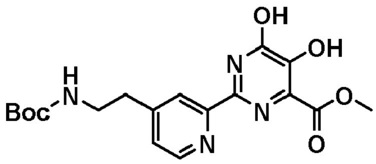

*Tert-butyl* (2-(2-(*N'*-hydroxycarbamimidoyl)pyridin-4-yl)ethyl)carbamate (1.0 eq) was suspended in $CHCl_3$ (0.26 M) and TEA (0.1 eq) was added at room temperature followed by DMAD (1.0 eq). The mixture was refluxed for 2 h. After cooling, solvent was removed *in vacuo* to afford dimethyl 2-((4-(2-((*tert*-butoxycarbonyl)amino)ethyl)picolinimidamido)oxy)but-2-enedioate intermediate as a brown oil which was used without further purification. UPLC method A *t*R 1.67 and 1.70 min; MS (ES$^+$) *m/z* 423 [M+H]$^+$. The solution of *2-((4-(2-((tert*-butoxycarbonyl)amino)ethyl)picolinimidamido)oxy)but-2-enedioate in *p*-xylene (0.26 M) was refluxed for 6 h until. After cooling to room temperature, volatiles were removed *in vacuo* and residue was purified by flash chromatography column C18 eluting with $H_2O$ / MeCN to afford the title compound as a brown solid (50%). $^1$H NMR (400 MHz, DMSO-$d_6$, 300 K) δ 12.39 (bs, 1H), 10.80 (bs, 1H), 8.56 (d, 1H, J = 5.0 Hz), 8.02 (s, 1H), 7.39 (dd, 1H, J = 5.0 Hz, J = 1.7 Hz), 6.93 (t, 1H, J = 5.4 Hz), 3.85 (s, 3H), 3.24-3.20 (m, 2H), 2.88-2.77 (m, 2H), 1.33 (s, 9H). UPLC method A *t*R 1.36 min; MS (ES$^+$) m/z 391 [M+H]$^+$.

*Step 5: (R)-2-(4-(2-aminoethyl)pyridin-2-yl)-N-(1-cyclohexylethyl)-5,6-dihydroxypyrimidine-4-carboxamide (Compound 88)*

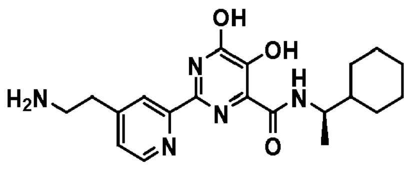

Methyl 2-(4-(2-((*tert*-butoxycarbonyl)amino)ethyl)pyridin-2-yl)-5,6-dihydroxypyrimidine-4-carboxylate (1.0 eq) was dissolved in DMF (0.15 M), (1*R*)-1-cyclohexylethanamine (1.0 eq) and TEA (2.0 eq) were added and the mixture was heated at 100 °C overnight. Volatiles were evaporated *in vacuo* and purified by flash chromatography column C18 eluting with $H_2O$ / MeCN to afford tert-butyl (R)-(2-(2-(4-((1-cyclohexylethyl)carbamoyl)-5,6-dihydroxypyrimidin-2-yl)pyridin-4-yl)ethyl)carbamate as a yellow solid (46%). UPLC method A *t*R 2.15 min; MS (ES$^+$) m/z 486 [M+H]$^+$. *Tert*-butyl (*R*)-(2-(2-(4-((1-cyclohexylethyl)carbamoyl)-5,6-dihydroxypyrimidin-2-yl)pyridin-4-yl)ethyl)carbamate (1.0 eq) was dissolved in DCM (0.08 M) and treated with TFA (10 eq). The mixture was stirred at room temperature for 1 h. The crude was evaporated and purified by RP-HPLC C18 eluting with $H_2O$ / MeCN containing 0.1 % trifluoroacetic acid to afford the title compound, beige solid, as its trifluoroacetate salt (27%). $^1$H NMR (400 MHz, DMSO-$d_6$, 300 K) δ 13.02 (s, 1H),

12.35 (s, 1H), 8.65 (d, 1H, J = 5.0 Hz), 8.51 (d, 1H, J = 9.3 Hz), 8.43 (s, 1H), 7.87 (bs, 2H), 7.52 (dd, 1H, J = 5.1 Hz, J = 1.7 Hz), 3.91-3.82 (m, 1H), 3.26-3.18 (m, 2H), 3.04 (t, 2H, J = 7.8 Hz), 1.82-1.52 (m, 6H), 1.25-0.92 (m, 8H). UPLC method A *t*R 1.22 min; MS ($ES^+$) m/z 386 $[M+H]^+$.

Example 6

**(R)-N-(1-cyclohexylethyl)-2-(4-(2-(dimethylamino)ethyl)pyridin-2-yl)-5,6-dihydroxypyrimidine-4-carboxamide (Compound 92)**

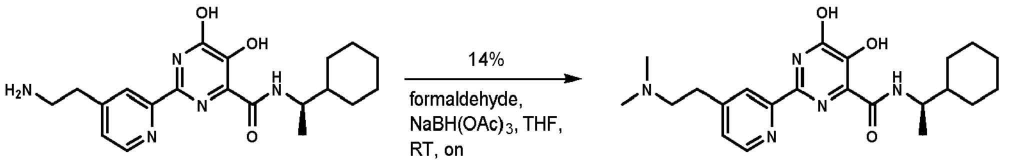


5.1 92

(*R*)-2-(4-(2-aminoethyl)pyridin-2-yl)-*N*-(1-cyclohexylethyl)-5,6-dihydroxypyrimidine-4-carboxamide (1.0 eq) was dissolved in THF (0.26 M) then $NaBH(OAc)_3$ (1.5 eq) and formaldeyde (20 eq) were added and the mixture was stirred at room temperature overnight. The crude was evaporated and purified by RP-HPLC C18 eluting with $H_2O$ / MeCN containing 0.1 % trifluoroacetic acid to afford the title compound, brown solid, as its trifluoroacetate salt (14%). $^1$H NMR (400 MHz, DMSO-$d_6$, 300 K) δ 12.97 (bs, 1H), 12.38 (bs, 1H), 9.57 (bs, 1H), 8.67 (d, 1H, J = 4.8 Hz), 8.48-8.43 (m, 2H), 7.54 (d, 1H, J = 5.0 Hz), 3.91-3.82 (m, 1H), 3.48-3.43 (m, 2H), 3.15 (t, 2H, J = 8.5 Hz), 2.87 (s, 3H), 2.86 (s, 3H), 1.81-1.52 (m, 6H), 1.25-1.11 (m, 6H), 1.04-0.92 (m, 2H). UPLC method A *t*R 1.25 min; MS ($ES^+$) m/z 414 $[M+H]^+$.

Example 7

**Cyclohexylmethyl 5,6-dihydroxy-2-(pyridin-2-yl)pyrimidine-4-carboxylate (Compound 2)**

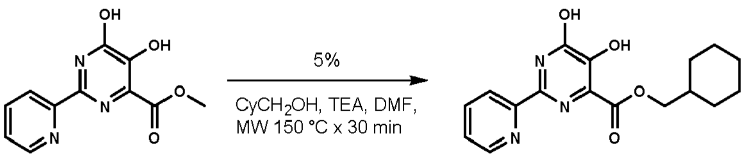


1.3 2

Methyl 5,6-dihydroxy-2-(pyridin-2-yl)pyrimidine-4-carboxylate (1.0 eq) was dissolved in DMF (0.8 M), cyclohexylmethanol (5.0 eq) and TEA (5.0 eq) were added and the mixture was stirred at 150 °C for 30 min under microwave irradiation. Volatiles were evaporated *in vacuo* and purified by RP-HPLC C18 eluting with $H_2O$ / MeCN containing 0.1 % trifluoroacetic acid to afford the title compound, beige solid, as its trifluoroacetate salt (5%). $^1$H-NMR (400 MHz, DMSO-$d_6$, 300 K) δ 12.58 (bs, 1H), 10.68 (bs, 1H), 8.69 (d, 1H, J = 4.8 Hz); 8.19 (d, 1H, J = 7.9 Hz); 8.02 (ddd, 1H, J = 7.6 Hz, J = 7.6 Hz, Hz, J = 1.8 Hz); 7.59-7.56 (m, 1H); 4.12 (d, 2H, J = 6.3 Hz); 1.80-1.63 (m, 6H); 1.29-1.00 (m, 5H); UPLC method A *t*R 1.97 min; MS ($ES^+$) m/z 330 $[M+H]^+$.

Example 8

**1-cyclohexylethyl 5,6-dihydroxy-2-(pyridin-2-yl)pyrimidine-4-carboxylate (Compound 17)**

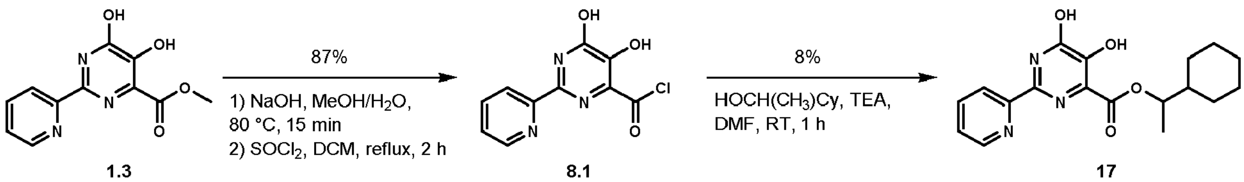

**1.3** **8.1** **17**

*Step 1: 5,6-dihydroxy-2-(pyridin-2-yl)pyrimidine-4-carbonyl chloride (compound 8.1)*

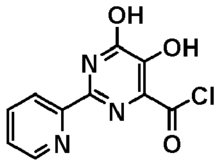

To a suspension of methyl 5,6-dihydroxy-2-(pyridin-2-yl)pyrimidine-4-carboxylate (1.0 eq) in MeOH (0.4 M) and $H_2O$ (0.4 M) was added 0.5 N NaOH (2.0 eq). The mixture was heated to 80 °C, after 15 min hydrolysis was completed. The solution was cooled to room temperature and 1 N HCl was added dropwise until pH 2. The solid was filtered, washed with $H_2O$ and dried under vacuum to give 5,6-dihydroxy-2-(pyridin-2-yl)pyrimidine-4-carboxylic acid as a yellow powder. $^1$H-NMR (400 MHz, DMSO-*d*6, 300 K) $\delta$ 8.69 (d, 1H, J = 4.3 Hz); 8.32 (d, 1H, J = 8.0 Hz); 8.02 (ddd, 1H, J = 7.7 Hz, J = 7.7, J = 1.8 Hz); 7.58 (ddd, 1H, J = 7.6 Hz, J = 4.8 Hz, J = 1.2 Hz); UPLC method B *t*R 1.04 min; MS (ES$^+$) *m/z* 234 $[M+H]^+$. To the solution of 5,6-dihydroxy-2-(pyridin-2-yl)pyrimidine-4-carboxylic acid (1.0 eq) in DCM (0.18 M) was added thionyl chloride (2.0 eq) and the mixture was refluxed for 2 h. After cooling to room temperature, the crude was concentrated under reduced pressure to afford the title compound as a yellow solid which was used as such without further purifications (87%). UPLC method A (quencing with MeOH) *t*R 0.97 min; MS (ES$^+$) *m/z* 248 $[M+H]^+$.

*Step 2: 1-cyclohexylethyl 5,6-dihydroxy-2-(pyridin-2-yl)pyrimidine-4- (Compounds 17)*

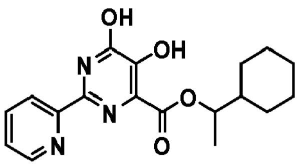

To a solution of 5,6-dihydroxy-2-(pyridin-2-yl)pyrimidine-4-carbonyl chloride (1.0 eq) in DCM (0.5 M), 1-cyclohexylethanol (1.0 eq) and TEA (0.55 mL, 4.0 mmol) were added and the mixture was stirred at room temperature overnight. The crude was evaporated *in vacuo* and purified by flash chromatography column C18 eluting with $H_2O$ / MeCN to afford the title compound as a beige solid (8%). $^1$H-NMR (400 MHz, DMSO-$d_6$, 300 K) $\delta$ 12.02 (bs, 2H), 8.68 (d, 1H, J = 3.04 Hz); 8.18 (d, 1H, J= 7.6 Hz); 8.03-7.99 (m, 1H); 7.57-7.54 (m, 1H); 4.93-4.87 (m, 1H); 1.87-1.55 (m, 6H); 1.26-1.04 (m, 8H); UPLC method A *t*R 2.11 min; MS (ES$^+$) *m/z* 344 $[M+H]^+$.

The following compounds (Table 2) were prepared according to the procedures described in Examples 1 to 8.

**Table 2. Characterization of the compounds of the invention**

| Compound No | Molecular Weight | MS (m/z) [M+H]+ | UPLC Method | tR |
|---|---|---|---|---|
| 1 | 328.37 | 329 | A | 1.89 |
| 2 | 329.35 | 330 | A | 1.97 |
| 3 | 286.29 | 287 | A | 1.44 |
| 4 | 314.34 | 315 | A | 1.94 |
| 5 | 342.39 | 343 | A | 2.05 |

(continued)

| Compound No | Molecular Weight | MS (m/z) [M+H]+ | UPLC Method | tR |
|---|---|---|---|---|
| 6 | 342.39 | 343 | A | 2.43 |
| 7 | 343.38 | 344 | A | 0.71 |
| 8 | 356.42 | 357 | A | 2.24 |
| 9 | 376.41 | 377 | B | 1.60 |
| 10 | 343.38 | 344 | A | 2.20 |
| 11 | 342.39 | 343 | A | 2.05 |
| 12 | 343.38 | 344 | A | 2.13 |
| 13 | 315.32 | 316 | A | 1.78 |
| 14 | 357.4 | 358 | A | 2.29 |
| 15 | 363.8 | 364 | A | 1.99 |
| 16 | 362.81 | 363 | A | 2.04 |
| 17 | 343.38 | 344 | B | 2.11 |
| 18 | 356.42 | 357 | A | 2.20 |
| 19 | 356.42 | 357 | A | 2.17 |
| 20 | 357.41 | 358 | A | 1.85 |
| 21 | 476.52 | 477 | A | 2.44 |
| 22 | 392.45 | 393 | A | 2.54 |
| 23 | 356.42 | 357 | A | 2.37 |
| 24 | 354.4 | 355 | B | 2.05 |
| 25 | 392.45 | 393 | A | 2.69 |
| 26 | 376.84 | 377 | A | 2.16 |
| 27 | 410.39 | 411 | A | 2.21 |
| 28 | 372.42 | 373 | A | 2.01 |
| 29 | 381.43 | 382 | A | 1.93 |
| 30 | 410.39 | 411 | A | 2.38 |
| 31 | 392.45 | 393 | A | 2.24 |
| 32 | 372.42 | 373 | A | 2.11 |
| 33 | 376.84 | 377 | A | 2.19 |
| 34 | 376.84 | 377 | A | 2.17 |
| 35 | 448.51 | 449 | A | 2.45 |
| 36 | 410.39 | 411 | A | 2.28 |
| 37 | 372.42 | 373 | A | 2.26 |
| 38 | 342.39 | 343 | A | 2.05 |
| 39 | 356.42 | 357 | A | 2.20 |
| 40 | 387.39 | 388 | A | 2.09 |
| 41 | 356.42 | 357 | A | 2.19 |
| 42 | 457.52 | 458 | A | 2.56 |
| 43 | 328.37 | 329 | A | 1.96 |

(continued)

| Compound No | Molecular Weight | MS (m/z) [M+H]+ | UPLC Method | tR |
|---|---|---|---|---|
| 44 | 410.39 | 411 | A | 2.21 |
| 45 | 357.41 | 358 | B | 1.89 |
| 46 | 476.52 | 477 | A | 2.43 |
| 47 | 350.37 | 351 | A | 1.95 |
| 48 | 357.41 | 358 | A | 1.38 |
| 49 | 376.84 | 377 | A | 2.13 |
| 50 | 475.54 | 476 | A | 2.02 |
| 51 | 385.42 | 386 | A | 1.60 |
| 52 | 410.39 | 411 | A | 2.21 |
| 53 | 461.56 | 462 | A | 1.47 |
| 54 | 476.52 | 477 | A | 2.26 |
| 55 | 372.42 | 373 | A | 1.69 |
| 56 | 328.37 | 329 | A | 1.85 |
| 57 | 328.37 | 329 | A | 1.85 |
| 58 | 342.39 | 343 | A | 1.97 |
| 59 | 328.37 | 329 | A | 1.81 |
| 60 | 399.49 | 400 | A | 1.32 |
| 61 | 457.52 | 458 | A | 2.26 |
| 62 | 399.44 | 400 | A | 1.75 |
| 63 | 356.42 | 357 | A | 2.12 |
| 64 | 356.42 | 357 | A | 2.11 |
| 65 | 328.37 | 329 | A | 1.89 |
| 66 | 422.48 | 423 | A | 2.34 |
| 67 | 370.45 | 371 | A | 2.29 |
| 68 | 422.48 | 423 | A | 2.38 |
| 69 | 372.42 | 373 | A | 2.1 |
| 70 | 476.52 | 477 | A | 2.36 |
| 71 | 476.52 | 477 | A | 2.35 |
| 72 | 392.45 | 393 | A | 2.20 |
| 73 | 406.48 | 407 | A | 2.39 |
| 74 | 475.54 | 476 | A | 2.05 |
| 75 | 392.45 | 393 | A | 2.27 |
| 76 | 440.54 | 441 | A | 1.29 |
| 77 | 372.42 | 373 | A | 2.00 |
| 78 | 376.84 | 377 | A | 2.16 |
| 79 | 356.42 | 357 | A | 2.08 |
| 80 | 392.45 | 393 | A | 2.22 |
| 81 | 357.41 | 358 | A | 1.76 |

(continued)

| Compound No | Molecular Weight | MS (m/z) [M+H]+ | UPLC Method | tR |
|---|---|---|---|---|
| 82 | 452.46 | 453 | A | 1.77 |
| 83 | 404.46 | 405 | B | 2.19 |
| 84 | 368.43 | 369 | B | 2.08 |
| 85 | 381.43 | 382 | A | 1.58 |
| 86 | 381.43 | 382 | A | 1.57 |
| 87 | 412.82 | 413 | A | 1.87 |
| 88 | 385.46 | 386 | A | 1.22 |
| 89 | 449.52 | 450 | A | 1.92 |
| 90 | 428.43 | 429 | A | 1.88 |
| 91 | 478.59 | 479 | A | 1.42 |
| 92 | 413.51 | 414 | A | 1.25 |
| 93 | 425.52 | 426 | A | 1.35 |
| 94 | 440.54 | 441 | A | 1.34 |
| 95 | 399.49 | 400 | A | 1.18 |
| 96 | 461.56 | 462 | A | 1.43 |

***Biology***

Compounds of the present invention could be tested for biological activity using the assay techniques below.

NC inhibition assay

The NC inhibition assay is based on the nucleic acid chaperone activity of the NC protein. NC destabilizes the secondary structure of cTAR DNA, the complementary sequence of the transactivation response element, which is involved in minus-strand DNA transfer during reverse transcription. In the assay, the cTAR sequence from the HIV-1 Mal strain (Bernacchi et al, J Mol Biol, 2002) is labeled with a dye (Alexa488 fluorophore) and a quencher 4-(4'-dimethylaminophenylazo)benzoic acid (Dabcyl) at its 5' and 3' ends, respectively. In the absence of destabilization, the close proximity of both cTAR ends results in a strong fluorescence quenching of the dye emission by the Dabcyl group. NC(11-55) added to cTAR at a ratio of 10 NC/cTAR melts the bottom of the cTAR stem, which increases the distance between the dye and the quencher and as a result increases the dye fluorescence signal [Shvadchak et al, (2009) Identification by high throughput screening of small compounds inhibiting the nucleic acid destabilization activity of the HIV-1 nucleocapsid protein. Biochimie, 916-923]. NCIs are expected to prevent the NC-induced destabilization of cTAR and thus reverse the NC-induced increase in the fluorescence of the labeled cTAR. The activity of the inhibitor is thus evaluated from the ratio of the fluorescence intensity observed in the presence of NC with the molecule to the one in the absence of the compound.

The doubly labelled cTAR DNA oligonucleotides (55nt) were synthesised and purified by IBA GmbH Nucleic Acids Product Supply (Göttingen, Germany). Extinction coefficient at 260 nm of $5.73 \times 10^5$ $M^{-1}$ $cm^{-1}$ was used to determine the concentration of cTAR.

The NC(11-55) peptide (Avilov et al, Antimicrob Agents Chemother, 2012) was synthesized by solid-phase peptide synthesis on a 433A synthesizer (ABI, Foster City, CA) as previously described [De Rocquigny H, (1991) First large scale chemical synthesis of the 72 amino acid HIV-1 nucleocapsid protein NCp7 in an active form. *Biochem Biophys Res Commun.,* 1010-1018]. The peptide was stored lyophilized and was resuspended in water before use. The concentration of peptide was determined by using an extinction coefficient of $5.7 \times 10^3$ $M^{-1}$ $cm^{-1}$ at 280 nm. Next, 2.5 molar equivalents of $ZnSO_4$ were added and pH was raised to its final value by adding buffer.

All experiments were performed at 20 °C in 25 mM Tris-HCl (pH 7.5), 30 mM NaCl, and 0.2 mM $MgCl_2$. To evaluate the inhibition activity of compounds on NC-induced cTAR destabilization, cTAR DNA (0.1 $\mu$M) was preincubated with 1 $\mu$M NC(11-55) (molar ratio NC:cTAR = 10:1) for 15 min at room temperature before addition of compound. For single point screening, all compounds were tested at three final concentrations: 1 $\mu$M, 10 $\mu$M and 100 $\mu$M. For the $IC_{50}$

determination, inhibition of NC-induced cTAR melting was monitored through addition of increasing concentrations of the selected molecules to the pre-formed NC(11-55)-cTAR complex. After the addition of compound, the mixture was incubated for 15 min at room temperature before the reading. The fluorescence signal was recorded with a plate reader Xenius (SAFAS Monaco) or Fluoromax 3 spectrofluorometer (Horiba Jobin-Yvon), and the excitation and emission wavelengths were 480 and 520 nm, respectively. The spectra were corrected for dilution, buffer fluorescence and screening effect (when it was required). In order to check the occurrence of aggregation, we performed additional reading of absorption with the plate reader Xenius (SAFAS Monaco) or Cary 4000 spectrophotometer (Agilent).

The percentage of inhibition (% inh) was calculated with the equation:

$$\% \, \boldsymbol{inh} = \frac{(I_{cTAR+NC}) - (I_{cTAR+NC+Inh})}{(I_{cTAR+NC}) - I_{cTAR}} * \mathbf{100},$$

where $I_{cTAR}$ is the fluorescence intensity of labeled cTAR in the absence of NC(11-55), $I_{cTAR+NC}$ is the fluorescence intensity of labeled cTAR in the presence of NC(11-55), and $I_{cTAR+NC+}$ Inh is fluorescence intensity of labeled cTAR in the presence of NC(11-55) and in the presence of compound.

To calculate the $IC_{50}$ values, the experimental data were fitted in a logarithm scale using Origin software (Dose response fitting). Each experiment was performed at least in duplicate. A compound showing >25% inhibition when tested at 100μM concentration is considered as "positive".

Electromobility Shift Assay (EMSA) assay

The assay used a recombinant full length (1-55) HIV-1 NCp7 proteins based on the NL4-3 sequence (GenBank accession number AF324493). The protein was expressed and purified as described in [Carteau, S., Gorelick, R. J. & Bushman, F. D. Coupled integration of human immunodeficiency virus type 1 cDNA ends by purified integrase in vitro: stimulation by the viral nucleocapsid protein. Journal of virology 73, 6670-6679 (1999)].

Competition for single-stranded (ss) DNA binding was realized by mixing 1 μL of serial dilutions of NC inhibitors (diluted in DMSO) with 44 μL of NCp7 (300 nM) in a reaction buffer (20 mM Tris-HCl pH 7.4, 2 mM $MgCl_2$, 100 mM NaCl, 1 mM DTT). 5 μL of a 10 ng/μL (4 nM) solution of M13mp18 ssDNA (BayouBiolabs) were then added and the tubes were incubated for 25 minutes at 37 °C. The reaction was terminated by chilling the tubes on ice followed by the addition of 5 μL of a loading buffer (30% glycerol, 0.01% xylene cyanol, 10 mM Tris-HCl pH 7.4). Samples were then resolved on 25 cm-long, 1% (w/v) agarose gels (SeaKem LE Agarose, Lonza) in 0.5x TBE. Gels were run in a Sub-Cell GT cuvette (BioRad) for 18 h at room temperature at 3 V/cm, stained with SybrGold (Molecular Probes) and scanned for fluorescence using a Typhoon 8600 (GE Healthcare). The experiments were performed in triplicate. Competition efficiency was realized by measuring the relative distance in pixels between the unbound ssDNA control, the NCp7-bound ssDNA control and the bound ssDNA for each points of the titration with the ImageQuant software. The $IC_{50}$ was estimated as the NC inhibitors concentrations for which the bound ssDNA migrated at 50% of the distance separating the bound from the unbound ssDNA.

A compound showing >25% inhibition when tested at 100μM concentration is considered as "positive".

MonoCycle/BiCycle assays

MonoCycle assay is a cell based assay allowing the evaluation of antiviral activity of HIV-1 inhibitors targeting the early steps of HIV-1 life cycle such as entry, uncoating of viral particles, reverse transcription and integration. The assay consists in the infection of TZM-bl cells (20,000/well) with the HIV-1 reference strain NL4-3 in presence of serial five-fold dilutions of the investigational inhibitors. After 48 hours, cells were lysed adding 40 μl of Glo Lysis Buffer (Promega) to each well for 5 minutes, then 40 μl of Brighy-Glo Luciferase Reagent (Promega) were added to each well for relative luminescence units (RLU) counting using Glo-Max Multi Detection System (Promega). RLU values from each well were elaborated using the GraphPad v5.0 software to calculate the $IC_{50}$ of each compound.

BiCycle Assay is cell based assay allowing the evaluation of the antiviral activity of HIV-1 inhibitors on the whole replication cycle of HIV-1. The assay consists in a first round of infection of the T-cell line MT-2 followed by infection of TZM-bl cells. MT-2 cells were seeded at a concentration of 50,000 cells/well in a 96-well plate and infected with the HIV-1 reference strain NL4-3 in presence of five-fold dilutions of the investigational compounds. After 72 hours, 50 μl of supernatant from each well, containing the virus produced in the first round of infection, were used to infect TZM-bl cells seeded in a 96-plate well at a concentration of 30,000 cells/well. Two days later, cells were lysed adding 40 μl of Glo Lysis Buffer (Promega) to each well for 5 minutes, then 40 μl of Brighy-Glo Luciferase Reagent (Promega) were added to each well for relative luminescence units (RLU) counting using Glo-Max Multi Detection System (Promega). RLU values from each well were elaborated using the GraphPad v5.0 software to calculate the $IC_{50}$ of each compound.

Proliferation assay PBMC

Peripheral blood mononuclear cells (PBMC) obtained from healthy donors by separation over Ficoll-Hypaque (GEHealthear-Cat.# GEH17-1440-02) were suspended at $2x10^6$ cells/mL in RPMI 1640 (Biowest-Cat.#L0501-500) supplemented with 10% of heat-inactivated sterile fetal bovine serum (Gibco-Cat.#10270-106), 2 mmol/l L-glutamine (Biowest-Cat.#X0550-100), 50 U/mL Penicillin-50 $\mu$g/mL Streptomycin (Biowest-Cat.#L0022-100), referred to as complete medium (CM). PBMC were supplemented with 5 $\mu$g/mL of phytohaemagglutinin (Sigma Aldr-Cat.#L1668) and cultured in 75 $cm^2$ flasks (Sarsted-Cat.#833.911.002) for 2 days at 37 °C in a humidified atmosphere with 5% $CO_2$.

PBMC were then washed in CM, counted by trypan blue exclusion (Gibco-Cat. #15250-061), centrifuged and suspended in fresh CM supplemented with 20 IU/mL recombinant human IL-2 (Miltenyi Biotec-Cat.#130-097-748) and seeded in a 96-well-tissue culture plate (Sarsted-Ca#831.837) at the density of $5x10^5$ cells/mL (or $1x10^5$ cells in 200 $\mu$L final CM volume per well). Cells were left untreated (negative control) or treated with different concentrations of the compounds.

After 7 days of incubation cytotoxicity was determined using Promega's CellTiter 96(R) AQueous One Solution Cell Proliferation Assay (MTS assay) (Promega-Cat.#G3581) according to the manufacturer's instuctions. This is a colorimetric method for determining the number of viable cells in proliferation or cytotoxicity assays. The MTS tetrazolium compound is bioreduced by cells into a colored formazan product that is soluble in tissue culture medium. This conversion is presumably accomplished by NADPH or NADH produced by dehydrogenase enzymes in metabolically active cells.

Briefly, MTS solution was thawed at room temperature, or in a water bath at 37 °C and 40 $\mu$L of MTS solution was pipeted into each well of the 96-well assay plate containing the samples in 200 $\mu$L of culture medium and incubated at 37 °C, 5% $CO_2$ for 4 hours. The optical density was read at 490 nm using a 96-well plate reader (Tecan Infinite F50) and $TD_{50}$ (toxic dose 50%, dose killing 50% of the cells compared to untreated control) value was calculated by interpolation of the curve obtained by plotting dose against percent mortality and by using "Magellan data analysis software". CM alone plus MTS solution was used as a blank, and cell culture well without compound was used as a cellular control.

HeLa Proliferation assay

HeLa cells (ATCC® CCL2™) in DMEM (Dulbecco Modified Eagle's Medium) without phenol red, Thermo, 11880, USA + 10% FBS + 1X PenStrep + 1X Gln or HUVEC cells (ATCC® CRL1730™) in EBM-2 (Endothelial Growth Basal Medium, Lonza, CC3156) + EGM-2 SingleQuotsTMSupplements and Growth Factors (Lonza, CC4176) + 1X Penicyllin-Streptomycin (PenStrep, ThermoFisher) + 1X Glutamine, are plated in a 384 well plate (Thermo, 4334-11, USA) to a density of 2000 cells per well and let recover for four hours at 37 °C, 5% $CO_2$ in a humidified atmosphere. After the recovery, compounds are transferred to assay plates as per compound preparation method. Assay plates are then incubated at 37 °C, 5% $CO_2$ in a humidified atmosphere for 72 hours. Cell viability is measured by the CellTiter Glo (Promega, G8080, USA) as per manufacturer instruction.

The compounds of the invention were tested in the assays described above and results are reported in the following Table 3.

**Table 3. Activity data for compounds of the invention.**

| Comp. No | NC inhibition screening results | NC inhibition $IC_{50}$ nM | EMSA screening results | Mono Cycle $IC_{50}$ nM | BiCycle $IC_{50}$ nM | Proliferation assay HELA $TD_{50}$ nM | Proliferation assay PBMC $TD_{50}$ nM |
|---|---|---|---|---|---|---|---|
| 1 | Positive | 166500 | Positive | >50000 | 1210 | 3585.7 | 2600 |
| 2 | Positive | 74000 | Negative | >50000 | 1775 | >50000 | 6000 |
| 3 | Positive | 390000 | Negative | 27393 | 1410.5 | >50000 | 6000 |
| 4 | Positive | 320000 | Negative | >50000 | 461 | 8135.3 | 4800 |
| 5 | Positive | 42500 | Positive | 2493 | 702.5 | 3184.4 | 3000 |
| 6 | Positive | 37500 | Positive | 4301 | 2258.5 | 4236.2 | 10480 |
| 7 | Positive | 335000 | Positive | >50000 | >50000 | >50000 | >50000 |
| 8 | Positive | 27500 | Positive | 4545 | 518 | 4847.2 | 2000 |
| 9 | Positive | 128500 | Negative | 32897 | 1998.5 | | |
| 10 | Positive | 55000 | Negative | >50000 | 1400 | >50000 | 8100 |

(continued)

| Comp. No | NC inhibition screening results | NC inhibition $IC_{50}$ nM | EMSA screening results | Mono Cycle $IC_{50}$ nM | BiCycle $IC_{50}$ nM | Proliferation assay HELA $TD_{50}$ nM | Proliferation assay PBMC $TD_{50}$ nM |
|---|---|---|---|---|---|---|---|
| 11 | Positive | 14313 | Negative | 49993 | 715.5 | 8283.9 | 8700 |
| 12 | Positive | 57500 | Negative | >50000 | 2816 | >50000 | 4000 |
| 13 | Positive | 237500 | Negative | | | >50000 | 16300 |
| 14 | Positive | 27500 | Negative | >50000 | 6830.5 | >50000 | 5600 |
| 15 | Positive | ND* | Positive | >50000 | 9663.5 | >50000 | 25000 |
| 16 | Positive | ND* | Positive | 51070 | 2048.5 | 16609 | 5700 |
| 17 | Positive | 90000 | Negative | >50000 | 2027 | >50000 | 5500 |
| 18 | Positive | 20000 | Positive | 37322 | 437 | 4630.2 | 2400 |
| 19 | Positive | 8500 | Positive | 15284 | 217.5 | 2735.7 | 3075 |
| 20 | Positive | 3000 | Positive | 2113 | 94.5 | 686.4 | 1650 |
| 21 | Positive | 10000 | Positive | >50000 | 118.5 | 3960.7 | <400 |
| 22 | Positive | 3000 | Positive | 100000 | 449.5 | 2844.6 | 8050 |
| 23 | Positive | 20500 | Positive | 88453 | 203 | 2333.9 | 8900 |
| 24 | Positive | 22500 | Positive | 3607 | 110 | 1513.1 | 2600 |
| 25 | Positive | 3000 | Positive | 63966 | 32 | 1393.4 | 1750 |
| 26 | Positive | 1500 | Positive | 17562 | 121 | 3197.7 | 2490 |
| 27 | Positive | 12500 | Positive | >50000 | 433 | 5975.3 | 1075 |
| 28 | Positive | 22000 | Positive | 34258 | 40.5 | 2860.9 | 2700 |
| 29 | Positive | 2500 | Positive | | 1777 | 3396.5 | 1700 |
| 30 | Positive | ND* | Positive | >50000 | 1731 | >50000 | 5400 |
| 31 | Positive | 3500 | Positive | >50000 | 100 | 2070.9 | 5000 |
| 32 | Positive | 45000 | Positive | 8263 | 340.67 | 935.6 | 3000 |
| 33 | Positive | 18000 | Positive | >50000 | 1216 | 6814.8 | 3400 |
| 34 | Positive | 47500 | Negative | 14522 | 665 | 8229.6 | 5880 |
| 35 | Positive | ND* | Positive | >50000 | 530 | 4589.6 | 670 |
| 36 | Positive | 22500 | Positive | 45352 | 131.5 | 4717.7 | 465 |
| 37 | Positive | 29000 | Positive | 6911 | 332.5 | 897.6 | 1500 |
| 38 | Positive | 46500 | Positive | 11220 | 153.67 | 578.2 | 2450 |
| 39 | Positive | 27000 | Negative | >50000 | 76.5 | 878.8 | 2300 |
| 40 | Positive | 6350 | Positive | | | | |
| 41 | Positive | ND* | Positive | | 297.5 | 2013 | 2800 |
| 42 | Positive | 14500 | Positive | | 1600 | | |
| 43 | Positive | ND* | Positive | | 1383 | 2190.1 | 8090 |
| 44 | Positive | ND* | Positive | >50000 | 936.5 | >50000 | 21550 |
| 45 | Positive | 30500 | Positive | >50000 | 1387.5 | 3227.2 | 3360 |
| 46 | Positive | 10000 | Positive | | 543 | 6478.4 | 1000 |

(continued)

| Comp. No | NC inhibition screening results | NC inhibition $IC_{50}$ nM | EMSA screening results | Mono Cycle $IC_{50}$ nM | BiCycle $IC_{50}$ nM | Proliferation assay HELA $TD_{50}$ nM | Proliferation assay PBMC $TD_{50}$ nM |
|---|---|---|---|---|---|---|---|
| 47 | Positive | 6500 | | 8931 | 869.5 | 3332.6 | 4900 |
| 48 | Positive | 4000 | | 9869 | 1340 | 1549.6 | 2600 |
| 49 | Positive | 9000 | | 10927 | 351.5 | 2099.9 | 3400 |
| 50 | Positive | 5000 | Positive | | 348 | 2291.2 | 2200 |
| 51 | Positive | 10000 | Negative | | 10584 | 26976 | 8000 |
| 52 | Positive | 26500 | Positive | | 433 | 10186 | 1200 |
| 53 | Positive | 7500 | Negative | 38054 | 530.5 | 2876.3 | 790 |
| 54 | Positive | ND* | Positive | | 422 | 24972 | 25000 |
| 55 | Positive | 40000 | Positive | 9047 | 504 | 1466.5 | |
| 56 | Positive | 35000 | Positive | | 341 | 3203.9 | 4600 |
| 57 | Positive | 35500 | Positive | | 432 | 1909.4 | 2700 |
| 58 | Positive | 23000 | Positive | | 268 | 2045.2 | 1600 |
| 59 | Positive | 50000 | Positive | >50000 | 408.5 | 694.6 | 2180 |
| 60 | Positive | 3500 | Negative | >50000 | 693.5 | 6428.6 | 1600 |
| 61 | Positive | 17000 | Positive | 7183 | 210 | 2035.8 | 2400 |
| 62 | Positive | 15500 | Positive | >50000 | 251.5 | 4551.9 | 1870 |
| 63 | Positive | 7000 | Positive | 11785 | 125 | 398.6 | 2100 |
| 64 | Positive | 14500 | Positive | 23721 | 210 | 471.7 | 2500 |
| 65 | Positive | 47000 | Positive | >50000 | 330 | 883.9 | 3100 |
| 66 | Positive | 8000 | Positive | | 1968 | 3660 | 6600 |
| 67 | Positive | ND* | Positive | | 1343 | 3757.7 | 6700 |
| 68 | Positive | ND* | Positive | 44337 | 239.5 | 5700.7 | 2660 |
| 69 | Positive | 49000 | Positive | >50000 | 357 | 3899.9 | 7110 |
| 70 | Positive | ND* | Positive | >50000 | 190.5 | 4100.6 | 500 |
| 71 | Positive | ND* | Positive | >50000 | 559 | 12825 | 1420 |
| 72 | Positive | ND* | Positive | 13429 | 382.5 | 3487.5 | 2270 |
| 73 | Positive | ND* | Positive | >50000 | 358 | 5371.9 | 5790 |
| 74 | Positive | ND* | Positive | >50000 | 299 | 3672.8 | 1680 |
| 75 | Positive | ND* | Positive | >50000 | 752 | 7535.5 | 6900 |
| 76 | Positive | 7000 | Positive | 18346 | 565 | 3683.1 | 2500 |
| 77 | Positive | 23500 | Positive | 17330 | 1169.5 | 2257.8 | 2200 |
| 78 | Positive | 18000 | Positive | 8744 | 285 | 2357.6 | 1900 |
| 79 | Positive | 41000 | Positive | 2350 | 178 | 1723.3 | 2300 |
| 80 | Positive | 11000 | Positive | 2851 | 144 | 1611.2 | 2000 |
| 81 | Positive | 22000 | Positive | 2293 | 221.5 | 542.5 | 1400 |
| 82 | Positive | 19500 | Positive | | | 6530.8 | |

(continued)

| Comp. No | NC inhibition screening results | NC inhibition $IC_{50}$ nM | EMSA screening results | Mono Cycle $IC_{50}$ nM | BiCycle $IC_{50}$ nM | Proliferation assay HELA $TD_{50}$ nM | Proliferation assay PBMC $TD_{50}$ nM |
|---|---|---|---|---|---|---|---|
| 83 | Positive | ND* | Positive | | | | |
| 84 | Positive | ND* | Positive | 21186 | 1879.5 | 1924.5 | 3470 |
| 85 | Positive | 11500 | Positive | >50000 | 2673 | | |
| 86 | Positive | 5500 | Positive | >50000 | 2280.5 | 1414.7 | |
| 87 | Positive | 41000 | Negative | >50000 | 1826 | 6158 | 3220 |
| 88 | Positive | 7500 | Positive | >50000 | 12703 | 7133.8 | 3250 |
| 89 | Positive | 28000 | Negative | >50000 | 10447 | 12629 | 3290 |
| 90 | Positive | ND* | Positive | >50000 | 1793.5 | 6452.2 | 4270 |
| 91 | Positive | 12000 | Positive | 2146 | 1103 | 1160.4 | 1090 |
| 92 | Positive | 10000 | Negative | 6116 | 1400 | 1168.4 | 2020 |
| 93 | Positive | 50000 | Negative | >50000 | 1359.5 | | 1870 |
| 94 | Positive | 18000 | Negative | | | | 740 |
| 95 | Positive | 28000 | Negative | | | | |
| 96 | Positive | ND* | Negative | | | | |
| (*)ND - not determined due to solubility problems. At the highest concentration without aggregation, inhibition <50% was obtained in these cases. | | | | | | | |

HIV-integrase assay

The integrase assay was performed using the HIV-1 integrase assay kit (Xpress Bio Co, catalog number EZ-1700, Thurmont, MD) according to the manufacturer's instructions. In this assay the streptavidin-coated 96-well plates were used and coated with a double stranded HIV-1 LTR U5 donor substrate (DS) DNA containing an end-labeled biotin. Full-length recombinant HIV-1 integrase protein was loaded onto the DS DNA substrate. After, the compounds were added in the concentration 5 - 1500 $\mu$M, and then a different double-stranded target substrate (TS) DNA containing a 3'-end modification was added to the reaction mixture. The HIV-1 integrase cleaved the terminal two bases from the exposed 3'-end of the HIV-1 LTR DS DNA and then catalyzed a strand-transfer recombination reaction to integrate the DS DNA into the TS DNA. The products of the reaction were detected colorimetrically using an HRP-labeled antibody directed against TS 3'-end modification. Isentress™ at 175 nM concentration was used as a positive control and showed ≥97% of inhibition in all experiments. The percentage of DMSO was fixed at 2.5% v/v in all samples. The absorbance at 450 nm was recorded with an iMark™ Microplate Absorbance Reader (Bio-Rad Laboratories). The absorbance values were corrected from the mean blank absorbance (reaction buffer negative control). To calculate the $IC_{50}$ values, the experimental data were fitted in a logarithm scale using Origin software (Dose response fitting).

Three of the most active compounds (Compounds 26, 28, 60) were tested in the HIV-1 integration assay and the results are presented in Table 4.

**Table 4. Activity data for compounds in the HIV-1 integrase assay.**

| Comp. No | IN $IC_{50}$ nM | NC inhibition $IC_{50}$ nM |
|---|---|---|
| 26 | 150000 | 1500 |
| 28 | 84000 | 22000 |
| 60 | 10000 | 3500 |

The $IC_{50}$ value of compound 26 is 100-fold higher in the IN assay as compared to the NC Inh assay, indicating a clear preference of this compound for the NC protein. In contrast, compounds 28 and 60 showed only a 3.8- and 2.9-

fold increase in IN $IC_{50}$ values in comparison to NC Inhibition $IC_{50}$ values, suggesting that these compounds may target both IN and NC. Such a dual mechanism of action related to the targeting of the different effector mechanisms (i.e. IN and NC) is an highly promising approach as potentially avoids the pharmacokinetic disadvantages of using two separate agents with different absorption and distribution properties.

The antiviral activity of some of the compounds listed in Table 3 was further evaluated determining the $IC_{50}$ with viruses carrying resistant mutations that reduce the susceptibility to drugs currently employed in clinical practice to treat HIV infection. The resistant viruses were obtained through the NIH AIDS Reagent Program (www.aidsreagent.org) and have been already characterized by the Phenosense Assay (Monogram Biosciences), considered as the reference assay for phenotypic investigation of HIV drug resistance due to its large application in clinical trials.

The features of HIV-1 resistant viral strains used considered in the analysis are reported below (Table 5).

**Table 5. HIV-1 resistant viral strains**

| | | |
|---|---|---|
| ***Protease inhibitor (PI) resistant viral strains*** | | |
| **NIH Catalogue Number:** | **11808** | |
| PI resistance mutations: 10F 11I 13V 20T 32I 33F 35G 36I 54V 63P 71I 73S 84V 89V 90M | | |
| Other mutations 12P 15V 19P 62V 70T 79A | | |
| Drug | Level of resistance | Fold change[a] (Biological[b] or clinical[c] cut-off) |
| Atazanavir | High resistance | 90 (2.2[c]) |
| Darunavir | High resistance | 127 (10[c]) |
| Lopinavir | High resistance | 70 (10[c]) |
| **NIH Catalogue Number:** | **12465** | |
| PI resistance mutations: 10I 13V 20I 35D 36I 46I 54V 58E 63P 71V 74P 82L 90M 93L | | |
| Other mutations : 33V 37T 62V | | |
| Drug | Level of resistance | Fold change[a] (Biological[b] or clinical[c] cut-off) |
| Atazanavir | High resistance | 110 (2.2[c]) |
| Darunavir | Susceptible | 5.2 (10[c]) |
| Lopinavir | Intermediate resistance | 36 (10[c]) |
| ***Nucleoside Reverse Transcriptase Inhibitors (NRTI) resistant viral strains*** | | |
| **NIH Catalogue Number:** | **7401** | |
| NRTI resistance mutations: 41L 67N 118I 184V 208Y 210W 215Y | | |
| Other mutations: 35M 39A 43E 123E 177E 196E 203K 207E 211K 223Q 272P 286A 297R | | |
| Drug | Level of resistance | Fold change[a] (Biological[b] or clinical[c] cut-off) |
| Lamivudine | High resistance | >200 (3.5[c]) |
| Abacavir | High resistance | 7.4 (4.5[c]) |
| Emtricitabine | High resistance | >200 (3.5[c]) |
| Zidovudine | High resistance | 24 (1.9[b]) |
| Tenofovir | High resistance | 1.5 (1.4[c]) |
| **NIH Catalogue Number: 7400** | | |
| NRTI resistance mutations: 41L 44D 67N 69D 118I 184V 210W 215Y | | |
| Other mutations : 39A 43A 122E 142V 207E 211K 287R 293V 297A | | |
| Drug | Level of resistance | Fold change[a] (Biological[b] or clinical[c] cut-off) |
| Lamivudine | High resistance | >200 (3.5[c]) |
| Abacavir | High resistance | 7.7 (4.5[c]) |
| Emtricitabine | High resistance | >200 (3.5[c]) |
| Zidovudine | High resistance | 61 (1.9[b]) |
| Tenofovir | High resistance | 2.3 (1.4[c]) |
| ***Non-Nucleoside Reverse Transcriptase Inhibitors (NNRTI) resistant viral strains*** | | |
| **NIH Catalogue Number:** | **12231** | |
| NNRTI resistance mutations: 103N 179F 181C | | |
| Other mutations: 35M 39A 43E 123E 177E 196E 203K 207E 211K 223Q 228R 272P 286A | | |
| Drug | Level of resistance | Fold change[a] (Biological[b] or clinical[c] cut-off) |

(continued)

| | | |
|---|---|---|
| ***Non-Nucleoside Reverse Transcriptase Inhibitors (NNRTI) resistant viral strains*** | | |
| **NIH Catalogue Number:** | **12231** | |
| Etravirine | High resistance | 8.8 (2.9[c]) |
| Rilpivirine | High resistance | 2.3 (2[b]) |
| Efavirenz | High resistance | 90 (3[b]) |
| Nevirapine | High resistance | >200 (4.5[b]) |
| *These resistance mutations confer a reduced susceptibility to NRTI abacavir, zidovudine and | | |
| **NIH Catalogue Number:** | **12229** | |
| NNRTI resistance mutations: 100I 103N 221Y | | |
| Other mutations: 83K 177E 200A 211Q 281R 284K 293V 297A | | |
| Drug | Level of resistance | Fold change[a] (Biological[b] or clinical[c] cut-off) |
| Etravirine | High resistance | 6.8 (2.9[c]) |
| Rilpivirine | High resistance | 6.3 (2[b]) |
| Efavirenz | High resistance | >200 (3[b]) |
| Nevirapine | High resistance | >200 (4.5[b]) |
| *These resistance | mutations confer a red | uced susceptibility to all NRTIs |
| ***Integrase Inhibitors (NRTI) resistant viral strains*** | | |
| **NIH Catalogue Number:** | **11845** | |
| INI resistance mutations: 140S 148H | | |
| Other mutations: 14R 63I 101I 112A 193E 201I 279G | | |
| Drug | Level of resistance | Fold change[a] (Biological[b] or clinical[c] cut-off) |
| Raltegravir™ | High resistance | >200 (1.5[b]) |
| Elvitegravir™ | Intermediate | Not available (2.5[b]) |
| Dolutegravir™ | Low resistance[d] | Not available (Not defined) |
| **NIH Catalogue Number:** | **11842** | |
| INI resistance mutations: 92Q 155H | | |
| Other mutations: 6E 17N 20K 25E 39C 45V 101I 124A 160T 201I 211R 288N | | |
| Drug | Level of resistance | Fold change[a] (Biological[b] or clinical[c] cut-off) |
| Raltegravir™ | High resistance | 96 (1.5[b]) |
| Elvitegravir™ | High resistance[d] | Not available (2.5[b]) |
| Dolutegravir™ | Susceptible[d] | Not available (Not defined) |

Legend
a) Resistant virus $IC_{50}$ value to wild-type reference virus $IC_{50}$ value ratio.
b) The biological cut-off was determined by in vitro analysis of multiple viral variants not exposed to the drug and defined as the mean fold-change plus two standard deviations with respect to the reference viral strain. It is used when a clinical cut-off has been not established.
c) The clinical cut-off was determined by statistical analysis of clinical data and is defined as the fold-change value corresponding to loss of drug activity in vivo.
d) Level of resistance predicted by AntiRetroScan prediction algorithm (www.dbarca.net).

$IC_{50}$ values of resistant viral strains were determined by using the BiCycle Assay as previously described. The results of the analysis are summarized in Table 6, where fold change values are calculated as the ratio between the $IC_{50}$ values of resistant HIV-1 strains and the $IC_{50}$ value of the wild type reference HIV-1 strain NL4-3.

**Table 6. Fold change values of resistant viral strains**

| Compound | ARP clone (drug class with decreased activity) | | | | | | | |
|---|---|---|---|---|---|---|---|---|
| | 11808 (PI) | 12465 (PI) | 7400 (NRTI) | 7401 (NRTI) | 12231 (NNRTI) | 12229 (NNRTI) | 11845 (INI) | 11842 (INI) |
| **1** | 0.8 | 2.9 | 0.4 | *NT* | 1.5 | 0.6 | 1.2 | *NT* |

(continued)

| Compound | ARP clone (drug class with decreased activity) | | | | | | | |
|---|---|---|---|---|---|---|---|---|
| | 11808 (PI) | 12465 (PI) | 7400 (NRTI) | 7401 (NRTI) | 12231 (NNRTI) | 12229 (NNRTI) | 11845 (INI) | 11842 (INI) |
| **28** | 1.4 | 3.7 | 0.8 | 0.8 | 2.8 | 0.1 | 2.3 | 2.9 |
| **31** | 1.6 | 2.1 | 0.7 | 1.3 | 2.0 | 1.3 | 0.9 | 9.8 |
| **23** | 5.1 | 2.3 | 0.7 | 1.4 | 2.8 | 1.0 | 1.2 | 8.6 |
| **26** | 0.2 | 0.5 | 0.3 | 0.1 | 0.8 | *NT* | 0.7 | *NT* |
| Legend. NT = not tested | | | | | | | | |

Quantification of 2-LTR products

During the replication cycle of HIV, several forms of episomic HIV-DNA can be found in the nucleous as a result of abortive integration events in the genome of the cells. Unintegrated HIV-DNA forms such 2-LTR circles are considered as a marker of ongoing replication and can be easily detected and quantified through real time PCR systems. Moreover, several in vitro studies showed that integrase inhibitors like Raltegravir™ are responsible for the accumulation of 2-LTR circles in infected cells as a consequence of the block of HIV-DNA integration. Therefore, the quantification of 2-LTR circles can be used as an indirect method to test the blockade of the integration step by a candidate or licensed integrase inhibitors (Reigadas S. et al, J. Antimicrob. Chemother. 2010; 65: 434-437).

Evaluation of the potential activity of NCp7 inhibitors during the integration of HIV-DNA in the host genome

Since NCp7 partecipates to several steps of HIV replication including integration, it was developed a cell based assay aimed at the quantification of 2-LTR circles in infected cells cultured in presence of inhibitory concentrations of candidate NCp7 inhibitors. In each culture 3x10^6 MT-2 cells were infected with HIV-1 reference strain NL4-3 at M.O.I. 0.05 in presence of investigational NCp7 inhibitors 23, 31, 28. A culture with the HIV-1 Integrase inhibitor Raltegravir™ was prepared as a reference for the inhibition of integration, while an infected culture without drugs (named "no-drug") was used to compare the amount of 2-LTR in the different cultures. Table 7 indicates the concentration of drugs used in each culture in comparison to the values of $EC_{50}$ (calculated through the BiCycle Assay) and the values of $TD_{50}$.

Table 7. Concentration of drugs used [c] in comparison to the values of $EC_{50}$ (calculated through the BiCycle Assay) and the values of $TD_{50}$.

| Compound | Mean $EC_{50}$ (nM) | [c] drug 2-LTR assay (nM) | $TD_{50}$ (nM) | [c] 2-LTR assay/EC50 ratio |
|---|---|---|---|---|
| 28 | 41 | 1000 | 2700 | 24,4 |
| 31 | 100 | 3000 | 5000 | 30,0 |
| 23 | 200 | 5000 | 8900 | 25,0 |
| Raltegravir™ | 1 | 1000 | > 50000 | 1000,0 |

Cultures were incubated at 37°C and 5% $CO_2$ for 24 hours, then cells were harvested and total DNA was extracted using the "MagCore® Genomic DNA Whole Blood Kit" automated system (Diatech).

Around 500 ng of extracted DNA were used in separated real time PCR reactions specific for the quantification of the total genomic and HIV-1 DNA, and the HIV-DNA 2-LTR circles, respectively. Both values were normalized as copies/10^6 cells according to β-globin gene quantification included in the first method. For each culture a ratio between 2-LTR/ total HIV-DNA circles was calculated and compared to the ratio obtained with the "no-drug" culture. Results are shown in Table 8.

**Table 8.** 2-LTR assay results

| Compound | 2-LTR/HIV-DNA ratio drug vs no-drug |
|---|---|
| **28** | 1.3 |

(continued)

| Compound | 2-LTR/HIV-DNA ratio drug vs no-drug |
|---|---|
| **31** | 1.1 |
| **23** | 0.5 |
| **Raltegravir™** | 2.9 |

The results of the analysis shown in Table 8 indicated that the amounts of 2-LTR circles in all NCp7 treated cultures were similar those of the "no-drug" culture, while a three times higher titer of 2-LTR circles was measured in presence of Raltegravir™, as expected. These data supported the hypothesis that NCp7 inhibitors included in this analysis have no effect on viral integration.

## Claims

**1.** A compound of general formula (I):

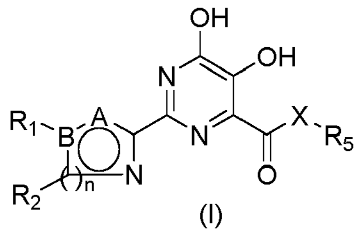

(I)

wherein:

A is selected from N, O, $CR_3$;
B is selected from N or C;
n is 1 or 2;
X is O, $NR_4$;
each of $R_1$ and $R_2$ is absent or independently selected from H, $C_{1-6}$alkyl, $C_{1-6}$alkoxy, halo$C_{1-6}$alkyl, halo$C_{1-6}$alkoxy, halogen, cyano, nitro, hydroxyl, C(O)-$C_{1-6}$alkyl, C(O)O-$C_{1-6}$alkyl, C(O)-aryl, C(O)heteroaryl, $N(R^a)_2$, $C(O)N(R^a)_2$, $SO_2N(R^a)_2$, aryl, heteroaryl, aryl-$C_{1-3}$alkyl, aryl-$C_{1-3}$alkoxy, each of said $C_{1-6}$alkyl, aryl and heteroaryl groups being optionally substituted by one or more groups independently chosen from halogen, hydroxy, $N(R^a)_2$, $C_{1-6}$alkyl, halo$C_{1-6}$alkyl, $C_{1-6}$alkoxy, halo$C_{1-6}$alkoxy, $C_{1-6}$alkyl-$N(R^a)_2$, nitro, aryl, heteroaryl;
or $R_1$ and $R_2$ together form a fused 5 or 6 membered aromatic ring optionally containing an heteroatom selected from N, O or S, said fused aromatic ring being optionally substituted by one or more groups independently chosen from halogen, hydroxy, $N(R^a)_2$, $C_{1-6}$alkyl, halo$C_{1-6}$alkyl, $C_{1-6}$alkoxy, halo$C_{1-6}$alkoxy, $C_{1-6}$alkyl-$N(R^a)_2$, nitro, aryl, heteroaryl;
when n = 2, $R_2$ is the substituent of both carbon atoms and $R_2$ and $R_2$ together may form a fused 5 or 6 membered aromatic ring optionally containing an heteroatom selected from N, O or S, said fused aromatic ring being optionally substituted by one or more groups independently chosen from halogen, hydroxy, $N(R^a)_2$, $C_{1-6}$alkyl, halo$C_{1-6}$alkyl, $C_{1-6}$alkoxy, halo$C_{1-6}$alkoxy, $C_{1-6}$alkyl-$N(R^a)_2$, nitro, aryl, heteroaryl;
$R_3$ is selected from H, $C_{1-6}$alkyl, halogen, halo$C_{1-6}$alkyl, nitro, cyano and $N(R^a)_2$;
$R_4$ is selected from H, $C_{1-6}$alkyl, halo$C_{1-6}$alkyl, aryl, heteroaryl, aryl-$C_{1-3}$alkyl;
$R_5$ is a group of general formula (II)

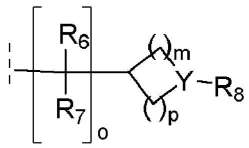

(II)

wherein

o is selected from 0 to 3;
m and p are selected from 0 to 3, with the proviso that at least one of m and n is not 0;
Y is selected from N and $CR_9$;
$R_6$ and $R_7$ are independently selected from H, $C_{1-6}$alkyl, halo$C_{1-6}$alkyl, $C_{3-6}$cycloalkyl, $C_{6-10}$aryl, $C_{6-10}$aryl-$C_{1-6}$alkyl, heteroaryl-$C_{1-3}$alkyl, $C_{3-15}$heterocyclyl, $C_{3-15}$heterocyclyl-$C_{1-3}$alkyl, $C_{3-6}$cycloalkyl-$C_{1-3}$alkyl, $C_{3-6}$cycloalkyl, aryl-$C_{1-3}$alkyl, heteroaryl-$C_{1-3}$alkyl, $C_{3-15}$heterocyclyl, $C_{3-15}$heterocyclyl-$C_{1-3}$alkyl, $C_{3-6}$cycloalkyl-$C_{1-3}$alkyl, each of them being optionally substituted by one or more groups independently chosen from $C_{1-6}$alkyl, $C_{1-6}$alkoxy, halogen, cyano, hydroxyl, C(O)-$C_{1-6}$alkyl, aryl, heteroaryl, aryl-$C_{1-3}$alkyl; or $R_6$ and $R_7$ are linked forming a cycloalkyl ring selected from cyclopropyl, cyclobutyl, cyclopentyl, cyclohexyl and cycloheptyl;
$R_8$ is selected from H, halogen, $C_{1-6}$alkyl, halo$C_{1-6}$alkyl, $C_{3-6}$cycloalkyl, $C_{6-10}$aryl, $C_{6-10}$aryl-$C_{1-6}$alkyl, heteroaryl-$C_{1-3}$alkyl, $C_{3-15}$heterocyclyl, $C_{3-15}$heterocyclyl-$C_{1-3}$alkyl, optionally substituted by one or more groups independently chosen from $C_{1-6}$alkyl, $C_{1-6}$alkoxy, halogen, hydroxyl, COOH, C(O)-$C_{1-6}$alkyl, $CONH_2$, CONH-$C_{1-6}$alkyl;
$R_9$ is selected from H, halogen, $C_{1-6}$alkyl;
or if X = N, $R_4$ and $R_5$ are alkyl chain linked together forming a ring selected from aziridine, azetidine, pyrrolidine, pyperidine, azepane, morpholine, piperazine, N-methylpiperazine wherein each of said ring is optionally substituted with $C_{1-6}$alkyl or $C_{6-10}$aryl;
each $R^a$ is independently selected from hydrogen, $C_{1-6}$alkyl, C(O)-$C_{1-6}$alkyl, C(O)-aryl, C(O)heteroaryl, C(O)O-$C_{1-6}$alkyl, $C_{6-10}$aryl-$C_{1-6}$alkyl, heteroaryl-$C_{1-3}$alkyl or $N(R^a)_2$ is a cyclic amine selected from pyrrolidine, pyperidine, and morpholine optionally substituted with one or
more groups independently selected from $C_{1-6}$alkyl, halogen, hydroxy;
and pharmaceutically acceptable salts, tautomers, stereoisomers thereof, for use as HIV-1 nucleocapsid inhibitor in the treatment of an HIV-1 infection.

**2.** The compound for use according to claim 1 wherein A is $CR_3$ or N and B is C.

**3.** The compound for use according to claim 1 of general formula (III):

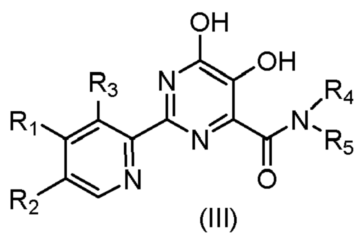

(III)

Wherein $R_1$, $R_2$, $R_3$, $R_4$ and $R_5$ are as defined in claim 1.

**4.** The compound for use according to claim 1, selected from:

- N-(cyclohexylmethyl)-5,6-dihydroxy-2-(pyridin-2-yl)pyrimidine-4-carboxamide;
- cyclohexylmethyl 5,6-dihydroxy-2-(pyridin-2-yl)pyrimidine-4-carboxylate;
- N-(cyclopropylmethyl)-5,6-dihydroxy-2-(pyridin-2-yl)pyrimidine-4-carboxamide;
- N-cyclohexyl-5,6-dihydroxy-2-(pyridin-2-yl)pyrimidine-4-carboxamide;
- (R)-N-(1-cyclohexylethyl)-5,6-dihydroxy-2-(pyridin-2-yl)pyrimidine-4-carboxamide;
- N-(2-cyclohexylethyl)-5,6-dihydroxy-2-(pyridin-2-yl)pyrimidine-4-carboxamide;
- 5,6-dihydroxy-N-((1-methylpiperidin-3-yl)methyl)-2-(pyridin-2-yl)pyrimidine-4-carboxamide;
- N-(3-cyclohexylpropyl)-5,6-dihydroxy-2-(pyridin-2-yl)pyrimidine-4-carboxamide;
- (5,6-dihydroxy-2-(pyridin-2-yl)pyrimidin-4-yl)(4-phenylpiperidin-1-yl)methanone;
- cyclohexylmethyl 5,6-dihydroxy-2-(3-methylpyridin-2-yl)pyrimidine-4-carboxylate;
- N-(cyclohexylmethyl)-5,6-dihydroxy-2-(3-methylpyridin-2-yl)pyrimidine-4-carboxamide;
- 2-cyclohexylethyl 5,6-dihydroxy-2-(pyridin-2-yl)pyrimidine-4-carboxylate;
- cyclohexyl 5,6-dihydroxy-2-(pyridin-2-yl)pyrimidine-4-carboxylate;
- 3-cyclohexylpropyl 5,6-dihydroxy-2-(pyridin-2-yl)pyrimidine-4-carboxylate;
- cyclohexylmethyl 2-(3-chloropyridin-2-yl)-5,6-dihydroxypyrimidine-4-carboxylate;
- 2-(3-chloropyridin-2-yl)-N-(cyclohexylmethyl)-5,6-dihydroxypyrimidine-4-carboxamide;
- 1-cyclohexylethyl 5,6-dihydroxy-2-(pyridin-2-yl)pyrimidine-4-carboxylate;

- (R)-N-(1-cyclohexylethyl)-5,6-dihydroxy-2-(6-methylpyridin-2-yl)pyrimidine-4-carboxamide;
- (R)-N-(1-cyclohexylethyl)-5,6-dihydroxy-2-(4-methylpyridin-2-yl)pyrimidine-4-carboxamide;
- (R)-2-(5-aminopyridin-2-yl)-N-(l-cyclohexylethyl)-5,6-dihydroxypyrimidine-4-carboxamide;
- benzyl (R)-6-(4-((1-cyclohexylethyl)carbamoyl)-5,6-dihydroxypyrimidin-2-yl)nicotinate;
- (R)-N-(1-cyclohexylethyl)-5,6-dihydroxy-2-(quinolin-2-yl)pyrimidine-4-carboxamide;
- (R)-N-(1-cyclohexylethyl)-5,6-dihydroxy-2-(3-methylpyridin-2-yl)pyrimidine-4-carboxamide;
- N-(1-cyclohexylcyclopropyl)-5,6-dihydroxy-2-(pyridin-2-yl)pyrimidine-4-carboxamide;
- (R)-N-(1-cyclohexylethyl)-5,6-dihydroxy-2-(isoquinolin-3-yl)pyrimidine-4-carboxamide;
- (R)-2-(4-chloropyridin-2-yl)-N-(1-cyclohexylethyl)-5,6-dihydroxypyrimidine-4-carboxamide;
- (R)-N-(1-cyclohexylethyl)-5,6-dihydroxy-2-(4-(trifluoromethyl)pyridin-2-yl)pyrimidine-4-carboxamide;
- (R)-N-(1-cyclohexylethyl)-5,6-dihydroxy-2-(4-methoxypyridin-2-yl)pyrimidine-4-carboxamide;
- (R)-2-(1H-benzo[d]imidazol-2-yl)-N-(1-cyclohexylethyl)-5,6-dihydroxypyrimidine-4-carboxamide;
- (R)-N-(1-cyclohexylethyl)-5,6-dihydroxy-2-(3-(trifluoromethyl)pyridin-2-yl)pyrimidine-4-carboxamide;
- (R)-N-(1-cyclohexylethyl)-5,6-dihydroxy-2-(isoquinolin-1-yl)pyrimidine-4-carboxamide;
- (R)-N-(1-cyclohexylethyl)-5,6-dihydroxy-2-(5-methoxypyridin-2-yl)pyrimidine-4-carboxamide;
- (R)-2-(3-chloropyridin-2-yl)-N-(1-cyclohexylethyl)-5,6-dihydroxypyrimidine-4-carboxamide;
- (R)-2-(6-chloropyridin-2-yl)-N-(1-cyclohexylethyl)-5,6-dihydroxypyrimidine-4-carboxamide;
- (R)-2-(4-(benzyloxy)pyridin-2-yl)-N-(1-cyclohexylethyl)-5,6-dihydroxypyrimidine-4-carboxamide;
- (R)-N-(1-cyclohexylethyl)-5,6-dihydroxy-2-(5-(trifluoromethyl)pyridin-2-yl)pyrimidine-4-carboxamide;
- (R)-N-(1-cyclohexylethyl)-5,6-dihydroxy-2-(6-methoxypyridin-2-yl)pyrimidine-4-carboxamide;
- (S)-N-(1-cyclohexylethyl)-5,6-dihydroxy-2-(pyridin-2-yl)pyrimidine-4-carboxamide;
- (S)-N-(1-cyclohexylethyl)-5,6-dihydroxy-2-(3-methylpyridin-2-yl)pyrimidine-4-carboxamide;
- (R)-N-(1-cyclohexylethyl)-5,6-dihydroxy-2-(4-nitropyridin-2-yl)pyrimidine-4-carboxamide;
- (R)-N-(1-cyclohexylethyl)-5,6-dihydroxy-2-(5-methylpyridin-2-yl)pyrimidine-4-carboxamide;
- tert-butyl (R)-(2-(4-((1-cyclohexylethyl)carbamoyl)-5,6-dihydroxypyrimidin-2-yl)pyridin-3-yl)carbamate;
- N-(2-cyclopentylethyl)-5,6-dihydroxy-2-(pyridin-2-yl)pyrimidine-4-carboxamide;
- (R)-N-(1-cyclohexylethyl)-5,6-dihydroxy-2-(6-(trifluoromethyl)pyridin-2-yl)pyrimidine-4-carboxamide;
- (R)-2-(6-aminopyridin-2-yl)-N-(1-cyclohexylethyl)-5,6-dihydroxypyrimidine-4-carboxamide;
- benzyl (R)-2-(4-((1-cyclohexylethyl)carbamoyl)-5,6-dihydroxypyrimidin-2-yl)isonicotinate;
- N-(cyclobutylmethyl)-5,6-dihydroxy-2-(isoquinolin-3-yl)pyrimidine-4-carboxamide;
- (R)-2-(4-aminopyridin-2-yl)-N-(1-cyclohexylethyl)-5,6-dihydroxypyrimidine-4-carboxamide;
- (R)-2-(5-chloropyridin-2-yl)-N-(1-cyclohexylethyl)-5,6-dihydroxypyrimidine-4-carboxamide;
- (R)-2-(5-(benzylcarbamoyl)pyridin-2-yl)-N-(1-cyclohexylethyl)-5,6-dihydroxypyrimidine-4-carboxamide;
- (R)-2-(5-carbamoylpyridin-2-yl)-N-(1-cyclohexylethyl)-5,6-dihydroxypyrimidine-4-carboxamide;
- (S)-N-(1-cyclohexylethyl)-5,6-dihydroxy-2-(4-(trifluoromethyl)pyridin-2-yl)pyrimidine-4-carboxamide;
- (R)-2-(5-((benzylamino)methyl)pyridin-2-yl)-N-(1-cyclohexylethyl)-5,6-dihydroxypyrimidine-4-carboxamide;
- benzyl (R)-6-(4-((1-cyclohexylethyl)carbamoyl)-5,6-dihydroxypyrimidin-2-yl)picolinate;
- (R)-N-(1-cyclohexylethyl)-5,6-dihydroxy-2-(5-(hydroxymethyl)pyridin-2-yl)pyrimidine-4-carboxamide;
- 5,6-dihydroxy-N-(4-methylcyclohexyl)-2-(pyridin-2-yl)pyrimidine-4-carboxamide;
- 5,6-dihydroxy-N-(3-methylcyclohexyl)-2-(pyridin-2-yl)pyrimidine-4-carboxamide;
- N-(4,4-dimethylcyclohexyl)-5,6-dihydroxy-2-(pyridin-2-yl)pyrimidine-4-carboxamide;
- 5,6-dihydroxy-N-(2-methylcyclohexyl)-2-(pyridin-2-yl)pyrimidine-4-carboxamide;
- (R)-N-(1-cyclohexylethyl)-2-(5-((ethylamino)methyl)pyridin-2-yl)-5,6-dihydroxypyrimidine-4-carboxamide;
- tert-butyl (R)-(6-(4-((1-cyclohexylethyl)carbamoyl)-5,6-dihydroxypyrimidin-2-yl)pyridin-3-yl)carbamate;
- (R)-2-(5-acetamidopyridin-2-yl)-N-(l-cyclohexylethyl)-5,6-dihydroxypyrimidine-4-carboxamide;
- N-(2-cyclopentyl-2-methylpropyl)-5,6-dihydroxy-2-(pyridin-2-yl)pyrimidine-4-carboxamide;
- (R)-N-(1-cyclohexylpropan-2-yl)-5,6-dihydroxy-2-(pyridin-2-yl)pyrimidine-4-carboxamide;
- N-(1-cyclopentylethyl)-5,6-dihydroxy-2-(pyridin-2-yl)pyrimidine-4-carboxamide;
- (R)-N-(1-cyclohexylethyl)-5,6-dihydroxy-2-(6-methoxyquinolin-2-yl)pyrimidine-4-carboxamide;
- (R)-N-(1-cyclohexylethyl)-2-(5,6-dimethylpyridin-2-yl)-5,6-dihydroxypyrimidine-4-carboxamide;
- N-[(1R)-1-cyclohexylethyl]-2-(4-methoxyquinolin-2-yl)-5,6-bis(oxidanyl)pyrimidine-4-carboxamide;
- N-[(1S)-1-cyclohexylethyl]-2-(4-methoxypyridin-2-yl)-5,6-bis(oxidanyl)pyrimidine-4-carboxamide;
- benzyl (S)-6-(4-((1-cyclohexylethyl)carbamoyl)-5,6-dihydroxypyrimidin-2-yl)nicotinate;
- benzyl (S)-2-(4-((1-cyclohexylethyl)carbamoyl)-5,6-dihydroxypyrimidin-2-yl)isonicotinate;
- (S)-N-(1-cyclohexylethyl)-5,6-dihydroxy-2-(isoquinolin-3-yl)pyrimidine-4-carboxamide;
- (R)-N-(1-cyclohexylethyl)-5,6-dihydroxy-2-(4-methylquinolin-2-yl)pyrimidine-4-carboxamide;
- (R)-2-(4-(benzylcarbamoyl)pyridin-2-yl)-N-(1-cyclohexylethyl)-5,6-dihydroxypyrimidine-4-carboxamide;
- (S)-N-(1-cyclohexylethyl)-5,6-dihydroxy-2-(quinolin-2-yl)pyrimidine-4-carboxamide;

- (R)-N-(1-cyclohexylethyl)-5,6-dihydroxy-2-(4-(4-methylpiperazin-1-yl)pyridin-2-yl)pyrimidine-4-carboxamide;
- N-(4,4-dimethylcyclohexyl)-5,6-dihydroxy-2-(4-methoxypyridin-2-yl)pyrimidine-4-carboxamide;
- (S)-2-(4-chloropyridin-2-yl)-N-(1-cyclohexylethyl)-5,6-dihydroxypyrimidine-4-carboxamide;
- (S)-N-(1-cyclohexylethyl)-5,6-dihydroxy-2-(4-methylpyridin-2-yl)pyrimidine-4-carboxamide;
- (S)-N-(1-cyclohexylethyl)-5,6-dihydroxy-2-(isoquinolin-4-yl)pyrimidine-4-carboxamide;
- (S)-2-(5-aminopyridin-2-yl)-N-(1-cyclohexylethyl)-5,6-dihydroxypyrimidine-4-carboxamide;
- (R)-N-(6-(4-((1-cyclohexylethyl)carbamoyl)-5,6-dihydroxypyrimidin-2-yl)pyridin-3-yl)oxazole-5-carboxamide;
- N-(1-cyclohexylcyclopropyl)-5,6-dihydroxy-2-(isoquinolin-3-yl)pyrimidine-4-carboxamide;
- N-(1-cyclohexylcyclopropyl)-5,6-dihydroxy-2-(4-methylpyridin-2-yl)pyrimidine-4-carboxamide;
- (R)-N-(1-cyclohexylethyl)-5,6-dihydroxy-2-(1H-pyrrolo[3,2-c]pyridin-4-yl)pyrimidine-4-carboxamide;
- (S)-N-(1-cyclohexylethyl)-5,6-dihydroxy-2-(1H-pyrrolo[3,2-c]pyridin-4-yl)pyrimidine-4-carboxamide;
- 2-(4-chloropyridin-2-yl)-N-(1-(4,4-difluorocyclohexyl)ethyl)-5,6-dihydroxypyrimidine-4-carboxamide;
- (R)-2-(4-(2-aminoethyl)pyridin-2-yl)-N-(1-cyclohexylethyl)-5,6-dihydroxypyrimidine-4-carboxamide;
- (R)-N-(1-cyclohexylethyl)-2-(5-(N-ethylsulfamoyl)pyridin-2-yl)-5,6-dihydroxypyrimidine-4-carboxamide;
- N-(1-(4,4-difluorocyclohexyl)ethyl)-5,6-dihydroxy-2-(isoquinolin-1 -yl)pyrimidine-4-carboxamide;
- (R)-N-(1-cyclohexylethyl)-5,6-dihydroxy-2-(4-(2-(((1-methyl-1H-pyrrol-2-yl)methyl)amino)ethyl)pyridin-2-yl)pyrimidine-4-carboxamide;
- (R)-N-(1-cyclohexylethyl)-2-(4-(2-(dimethylamino)ethyl)pyridin-2-yl)-5,6-dihydroxypyrimidine-4-carboxamide;
- (R)-N-(1-cyclohexylethyl)-5,6-dihydroxy-2-(5-(pyrrolidin-1-ylmethyl)pyridin-2-yl)pyrimidine-4-carboxamide;
- (R)-N-(1-cyclohexylethyl)-5,6-dihydroxy-2-(5-(4-methylpiperazin-1-yl)pyridin-2-yl)pyrimidine-4-carboxamide;
- (R)-N-(1-cyclohexylethyl)-2-(4-((ethylamino)methyl)pyridin-2-yl)-5,6-dihydroxypyrimidine-4-carboxamide;
- (R)-2-(4-((benzylamino)methyl)pyridin-2-yl)-N-(1-cyclohexylethyl)-5,6-dihydroxypyrimidine-4-carboxamide;

and pharmaceutically acceptable salts, tautomers, stereoisomers thereof.

**5.** A combination of a compound as defined in any one of claim 1 to 4 or pharmaceutically acceptable salts, tautomers, stereoisomers thereof with at least one further active compound for use in the treatment of an HIV-1 infection.

**6.** A pharmaceutical composition comprising an effective amount of one or more compound as defined in any one of claims 1 to 4, alone or in combination with at least one further active compound, and at least one pharmaceutically acceptable excipient for use in the treatment of HIV-1 infections.

**7.** The combination for use according to claim 5 or the pharmaceutical composition for use according to claim 6 wherein the at least one further active compound is active against HIV.

**8.** The combination for use or the pharmaceutical composition for use according to claim 7 wherein the at least one further active compound active against HIV is selected from the group consisting of a nucleoside reverse transcriptase inhibitor, a non-nucleoside reverse transcriptase inhibitor, a protease inhibitor, an integrase inhibitor, an entry inhibitor and a fusion inhibitor.

**9.** The compound for use according to any one of claim 1 to 4, the combination for use according to claim 5, 7 or 8 or the pharmaceutical composition for use according to claim 6-8 wherein the HIV-1 infection is a drug resistant HIV-1 infection.

**10.** The compound for use, the combination for use or the pharmaceutical composition for use according to claim 9 wherein the drug resistant HIV-1 is resistant to at least one member selected from the group consisting of a nucleoside reverse transcriptase inhibitor, a non-nucleoside reverse transcriptase inhibitor, a protease inhibitor, an integrase inhibitor, an entry inhibitor and a fusion inhibitor.

Europäisches Patentamt
European Patent Office
Office européen des brevets

# EUROPEAN SEARCH REPORT

Application Number
EP 16 18 6511

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 03/035076 A1 (ANGELETTI P IST RICHERCHE BIO [IT]; DI FRANCESCO MARIA EMILIA [IT]; GA) 1 May 2003 (2003-05-01)<br>* claims 1, 11, 23, 24, 26, 45, 46, 49 *<br>----- | 1-10 | INV.<br>C07D401/14<br>C07D401/04<br>C07D403/04<br>A61K31/506<br>A61P31/18 |
| A | PETROCCHI ET AL: "From dihydroxypyrimidine carboxylic acids to carboxamide HIV-1 integrase inhibitors: SAR around the amide moiety", BIOORGANIC & MEDICINAL CHEMISTRY LETTERS, PERGAMON, AMSTERDAM, NL, vol. 17, no. 2, 11 January 2007 (2007-01-11), pages 350-353, XP005827231, ISSN: 0960-894X, DOI: 10.1016/J.BMCL.2006.10.054<br>* page 352; table 1; compound 8 *<br>----- | 1-10 | |
| A | WO 2012/151567 A1 (ST JUDE CHILDRENS RES HOSPITAL; WEBB THOMAS R [US]; BOYD VINCENT A [US) 8 November 2012 (2012-11-08)<br>* claim 1 *<br>* page 76 - page 101; examples 32, 49, 51, 58, 62, 73, 99 *<br>----- | 1-10 | TECHNICAL FIELDS SEARCHED (IPC)<br>C07D |

The present search report has been drawn up for all claims

1

| Place of search | Date of completion of the search | Examiner |
|---|---|---|
| Munich | 4 October 2016 | Beligny, Samuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 18 6511

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2016

| Patent document cited in search report | Publication date | Patent family member(s) | Publication date |
|---|---|---|---|
| WO 03035076 A1 | 01-05-2003 | AT 355064 T | 15-03-2006 |
| | | CA 2463975 A1 | 01-05-2003 |
| | | DE 60218511 T2 | 25-10-2007 |
| | | DK 1441734 T3 | 11-06-2007 |
| | | EP 1441734 A1 | 04-08-2004 |
| | | ES 2281565 T3 | 01-10-2007 |
| | | JP 4351053 B2 | 28-10-2009 |
| | | JP 2005510500 A | 21-04-2005 |
| | | PT 1441734 E | 31-05-2007 |
| | | SI 1441734 T1 | 31-08-2007 |
| | | US 2005075356 A1 | 07-04-2005 |
| | | US 2007083045 A1 | 12-04-2007 |
| | | WO 03035076 A1 | 01-05-2003 |
| WO 2012151567 A1 | 08-11-2012 | US 2014079666 A1 | 20-03-2014 |
| | | WO 2012151567 A1 | 08-11-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012076822 A
- EP 1087941 A
- WO 03014062 A
- WO 2005121376 A
- WO 2004032869 A
- WO 2011156674 A
- WO 2003035076 A


### Non-patent literature cited in the description


- **MORI M. et al.** *ACS Chemical Biology,* 2014, vol. 9, 1950-1955
- **KRISHNAMOORTHY G. et al.** *Nucleic Acids Res.,* 2003, vol. 31, 5425-5432
- **POLJAK L et al.** *J. Mol. Biol.,* 2003, vol. 329, 411-421
- **MIRAMBEAU G. et al.** *PLoS One,* 2007, vol. 2, e669
- **MORI M et al.** *Curr. Pharm. Des.,* 2011, vol. 17, 3713-3728
- **PACE, P. et al.** *J. Med. Chem,* 2007, vol. 50 (9), 2225-2239
- Design of Prodrugs. Elsevier, 1985
- **E.L. ELIEL ; S.H. WILEN.** Stereochemistry of Carbon Compounds. John Wiley & Sons, 1994, 1119-1190
- **BERG et al.** Pharmaceutical Salts. *J. Pharm. Sci.,* 1977, vol. 66, 1-19
- **HUANG.** *J. Virol.,* 1997, vol. 71, 4378-4384
- **LAPADAT-TAPOLSKY.** *J.Mol. Biol.,* 1997, vol. 268, 250-260
- **DEMENE.** *Biochemistry,* 1994, vol. 33, 11707-11716
- **JERRY MARCH.** Advanced Organic Chemistrym. John Wiley & Sons, 19 August 1956, 370-376
- **CARTEAU, S. ; GORELICK, R. J. ; BUSHMAN, F. D.** Coupled integration of human immunodeficiency virus type 1 cDNA ends by purified integrase in vitro: stimulation by the viral nucleocapsid protein. *Journal of virology,* 1999, vol. 73, 6670-6679
- **REIGADAS S. et al.** *J. Antimicrob. Chemother,* 2010, vol. 65, 434-437